# EUROPEAN PATENT SPECIFICATION

(11) **EP 2 287 192 B1**
(45) Date of publication and mention of the grant of the patent: **26.08.2015**
(21) Application number: 10178225.8
(22) Date of filing: 17.11.2003
(51) Int. Cl.: C07K 16/24, C07K 16/28, C12N 5/12, A61K 39/395, A61P 35/04, A61K 31/663, A61K 38/17, A61K 38/19, G01N 33/50

(54) **Methods for preventing and treating cancer metastasis and bone loss associated with cancer metastasis**
Verfahren zur Prävention und Behandlung von Krebsmetastasen und Knochenschwund, der mit Krebsmetastasen assoziiert ist
Procédés de prévention et de traitement des métastases de cancer et perte osseuse associée aux métastases de cancer

(30) Priority: 15.11.2002 US 426781 P
(43) Date of publication of application: 23.02.2011
(62) Divisional of application: 10004581.4
(73) Proprietor: Novartis Vaccines and Diagnostics, Inc., Emeryville CA 94662-8097 (US)
(72) Inventor: Zimmerman, Deborah Lee, Emeryville, CA 94662-8097 (US); Harrowe, Gregory, Emeryville, CA 94662-8097 (US); Liu, Cheng, Emeryville, CA 94662-8097 (US); Koths, Kirston, Emeryville, CA 94662-8097 (US); Kavanaugh, W. Michael, Emeryville, CA 94662-8097 (US); Long, Li, Emeryville, CA 94662-8097 (US)
(74) Representative: Marshall, Cameron John

(56) References cited:
- EP-A1- 0 457 804
- WO-A-99/29345
- US-A- 5 491 065
- US-A- 6 025 146
- US-A- 6 117 422
- YONEDA T ET AL: "INHIBITION OF OSTEOLYTIC BONE METASTASIS OF BREAST CANCER BY COMBINED TREATMENT WITH THE BIPHOSPHONATE IBANDRONATE AND TISSUE INHIBITOR OF THE MATRIX METALLOPROTEINASE-2", JOURNAL OF CLINICAL INVESTIGATION, NEW YORK, NY, US, vol. 99, no. 10, 15 May 1997 (1997-05-15), pages 2509-2517, XP000946362, ISSN: 0021-9738
- LOKESHWAR B L ET AL: "Development and characterization of monoclonal antibodies to murine macrophage colony-stimulating factor.", JOURNAL OF IMMUNOLOGY (BALTIMORE, MD. : 1950) 15 JUL 1988, vol. 141, no. 2, 15 July 1988 (1988-07-15) , pages 483-488, XP002430404, ISSN: 0022-1767
- SHIOI A ET AL: "ENRICHMENT OF GENERATED MURINE OSTEOCLASTS", CALCIFIED TISSUE INTERNATIONAL, NEW YORK, NY, US, vol. 55, no. 5, 1994, pages 387-394, XP008075572, ISSN: 0171-967X
- MANCINO A T ET AL: "Breast cancer increases osteoclastogenesis by secreting M-CSF and upregulating RANKL in stromal cells.", THE JOURNAL OF SURGICAL RESEARCH SEP 2001, vol. 100, no. 1, September 2001 (2001-09), pages 18-24, XP002430405, ISSN: 0022-4804
- NEALE S D ET AL: "MACROPHAGE COLONY-STIMULATING FACTOR AND INTERLEUKIN-6 RELEASE BY PERIPROSTHETIC CELLS STIMULATES OSTEOCLAST FORMATION AND BONE RESORPTION", JOURNAL OF ORTHOPAEDIC RESEARCH, THE JOURNAL OF BONE AND JOINT SURGERY, INC.,, US, vol. 17, no. 5, September 1999 (1999-09), pages 686-694, XP001013119, ISSN: 0736-0266
- SHERR C J ET AL: "INHIBITION OF COLONY-STIMULATING FACTOR-1 ACTIVITY BY MONOCLONAL ANTIBODIES TO THE HUMAN CSF-1 RECEPTOR", BLOOD, W.B. SAUNDERS, PHILADELPHIA, VA, US, vol. 73, no. 7, 15 May 1989 (1989-05-15), pages 1786-1793, XP000568719, ISSN: 0006-4971
- TAKAHASHI M ET AL: "Amino-terminal region of human macrophage colony-stimulating factor (M-CSF) is sufficient for its in vitro biological activity: Molecular cloning and expression of carboxyl-terminal deletion mutants of human M-CSF", BIOCHEMICAL AND BIOPHYSICAL RESEARCH COMMUNICATIONS, ACADEMIC PRESS INC. ORLANDO, FL, US LNKD- DOI:10.1016/0006-291X(89)92683-1, vol. 161, no. 2, 15 June 1989 (1989-06-15) , pages 892-901, XP024837490, ISSN: 0006-291X [retrieved on 1989-06-15]

## Description

### TECHNICAL FIELD

This invention relates to methods for preventing and treating cancer metastasis and bone loss associated with cancer metastasis by administering M-CSF antagonist to a subject.

### BACKGROUND OF THE INVENTION

Cancer metastasis is the primary cause of post-operation or post-therapy recurrence in cancer patients. Despite intensive efforts to develop treatments, cancer metastasis remains substantially refractory to therapy. Bone is one of the most common sites of metastasis of various types of human cancers (e.g., breast, lung, prostate and thyroid cancers). The occurrence of osteolytic bone metastases causes serious morbidity due to intractable pain, high susceptibility to fracture, nerve compression and hypercalcemia. Despite the importance of these clinical problems, there are few available treatments for bone loss associated with cancer metastasis.

Osteoclasts mediate bone readsorption. Osteoclasts are multinucleated cells differentiating from haemopoietic cells. It is generally accepted that osteoclasts are formed by the fusion of mononuclear precursors derived from haemopoietic stem cells in the bone marrow, rather than incomplete cell divisions (Chambers, Bone and Mineral Research, 6: 1-25, 1989; Göthling et al., Clin Orthop Relat R. 120: 201-228, 1976; Kahn et al., Nature 258: 325-327, 1975, Suda et al., Endocr Rev 13: 66-80, 1992; Walker, Science 180: 875, 1973; Walker, Science 190: 785-787, 1975; Walker, Science 190: 784-785, 1975). They share a common stem cell with monocyte-macrophage lineage cells (Ash et al., Nature 283: 669-670, 1980, Kerby et al., J. Bone Miner Res 7: 353-62, 1992). The differentiation of osteoclast precursors into mature multinucleated osteoclasts requires different factors including hormonal and local stimuli (Athanasou et al., Bone Miner 3: 317-333, 1988; Feldman et al., Endocrinology 107: 1137-1143, 1980; Walker, Science 190: 784-785, 1975; Zheng et al., Histochem J 23: 180-188, 1991) and living bone and bone cells have been shown to play a critical role in osteoclast development (Hagenaars et al., Bone Miner 6: 179-189, 1989). Osteoblastic or bone marrow stromal cells are also required for osteoclast differentiation. One of the factors produced by these cells that supports osteoclast formation is macrophage-colony stimulating factor, M-CSF (Wiktor-Jedrzejczak et al., Proc Natl Acad Sci USA 87: 4828-4832, 1990; Yoshida et al., Nature 345: 442-444, 1990). Receptor activator for NF-κB ligand (RANKL, also known as TRANCE, ODF and OPGL) is another signal (Suda et al., Endocr Rev 13: 66-80, 1992) through which osteoblastic/stromal cells stimulate osteoclast formation and resorption via a receptor, RANK (TRANCER), located on osteoclasts and osteoclast precursors (Lacey et al., Cell 93: 165-176, 1998; Tsuda et al., Biochem Biophys Res Co 234: 137-142, 1997; Wong et al., J Exp Med 186: 2075-2080, 1997; Wong et al., J Biol. Chem 272: 25190-25194, 1997; Yasuda et al., Endocrinology 139: 1329-1337, 1998; Yasuda et al., Proc Natl Acad Sci US 95: 3597-3602, 1998). Osteoblasts also secrete a protein that strongly inhibits osteoclast formation called osteoprotegerin (OPG, also known as OCIF), which acts as a decoy receptor for the RANKL thus inhibiting the positive signal between osteoclasts and osteoblasts via RANK and RANKL.

Osteoclasts are responsible for dissolving both the mineral and organic bone matrix (Blair et al., J Cell Biol 102: 1164-1172, 1986). Osteoclasts represent terminally differentiated cells expressing a unique polarized morphology with specialized membrane areas and several membrane and cytoplasmic markers, such as tartrate resistant acid phosphatase (TRAP) (Anderson et al. 1979), carbonic anhydrase II (Väänänen et al., Histochemistry 78: 481-485, 1983), calcitonin receptor (Warshafsky et al., Bone 6: 179-185, 1985) and vitronectin receptor (Davies et al., J Cell Biol 109: 1817-1826, 1989). Multinucleated osteoclasts usually contain less than 10 nuclei, but they may contain up to 100 nuclei being between 10 and 100 µm in diameter (Göthling et al., Clin Orthop Relat R 120: 201-228, 1976). This makes them relatively easy to identify by light microscopy. They are highly vacuolated when in the active state, and also contain many mitochondria, indicative of a high metabolic rate (Mundy, in Primer on the metabolic bone diseases and disorders of mineral metabolism, pages 18-22, 1990). Since osteoclasts play a major role in osteolytic bone metastases, there is a need in the art for new agents and methods for preventing osteoclast stimulation.

Thus, there remains a need in the art to identify new agents and methods for preventing or treating cancer metastasis, including osteolytic bone metastases.

### SUMMARY OF THE INVENTION

The invention provides an M-CSF antagonist, for use in:
a) preventing bone metastases in a subject afflicted with metastatic cancer;
b) preventing, in a subject afflicted with metastatic cancer, bone loss associated with the cancer;
c) treating a subject afflicted with a metastatic cancer to bone,
d) reducing, in a subject afflicted with a metastatic cancer to bone, the severity of bone loss associated with the cancer,
e) treating a subject suffering from osteolytic bone metastases, or
f) treating a subject suffering from bone loss associated with cancer metastasis,
wherein said antagonist inhibits the interaction between M-CSF and its receptor (M-CSFR) and is selected from the group consisting of:
i) a polypeptide comprising an anti-M-CSF antibody; and
ii) a polypeptide comprising an anti-M-CSFR antibody.

### SUMMARY OF THE DISCLOSURE

The compositions and methods disclosed herein fulfill the aforementioned and other related needs in the art. In one embodiment, a method is provided for preventing bone metastases comprising administering to a subject afflicted with metastatic cancer a therapeutically effective amount of M-CSF antagonist, thereby preventing bone loss associated with the metastatic cancer. In another embodiment, a method of treating a subject afflicted with a metastatic cancer to bone is disclosed, comprising administering to said subject a therapeutically effective amount of M-CSF antagonist, thereby reducing the severity of bone loss associated with the metastatic cancer. In related embodiments, the aforementioned methods are provided wherein the subject is a mammal or wherein the mammal is a human.

It is contemplated that the methods disclosed herein achieve their therapeutic potential by inhibiting the interaction between M-CSF and its receptor (M-CSFR). It is further contemplated that the inhibition of M-CSF/M-CSFR interaction inhibits osteoclast proliferation and/or differentiation induced by tumor cells. In one embodiment, the M-CSF antagonist of the aforementioned methods is selected from the group consisting of: a polypeptide comprising an anti-M-CSF antibody; a polypeptide comprising an anti-M-CSFR antibody thereof; a soluble polypeptide comprising an M-CSF mutein or derivative thereof; or a soluble polypeptide comprising an M-CSFR mutein or derivative thereof.

In another embodiment, the aforementioned method is disclosed wherein the M-CSF antagonist is an anti-M-CSF antibody. In a related embodiment, the M-CSF antagonist is a polypeptide comprising an anti-M-CSFR antibody. In still another related embodiment, the M-CSF antagonist is a soluble polypeptide comprising an M-CSF mutein or derivative thereof. In yet another related embodiment, the M-CSF antagonist is a soluble polypeptide comprising an M-CSFR mutein or derivative thereof.

In another embodiment, the M-CSF antagonist is an antibody selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies. In still another embodiment, the antibody binds to the same epitope as monoclonal antibody 5H4 (ATCC Accession No. HB10027).

A number of metastatic cancers are contemplated to be amenable to the methods disclosed herein. In one embodiment, the metastatic cancer is breast, lung, renal, multiple myeloma, thyroid, prostate, adenocarcinoma, blood cell malignancies, including leukemia and lymphoma; head and neck cancers; gastrointestinal cancers, including stomach cancer, colon cancer, colorectal cancer, pancreatic cancer, liver cancer; malignancies of the female genital tract, including ovarian carcinoma, uterine endometrial cancers and cervical cancer; bladder cancer; brain cancer, including neuroblastoma; sarcoma, osteosarcoma; and skin cancer, including malignant melanoma or squamous cell cancer.

Administration of a therapeutically effective dose of a M-CSF antagonist is also contemplated. In one embodiment, the M-CSF antagonist is an antibody administered at a dose between about 0.01 mg/kg and about 100 mg/kg.

In another embodiment, a non-murine antibody is disclosed that binds to M-CSF for treating a subject afflicted with a metastatic cancer, wherein said antibody effectively reduces the severity of bone loss associated with the metastatic cancer.

As described herein, antibodies disclosed herein inhibit osteolysis. In one embodiment, a non-murine monoclonal antibody is disclosed that specifically binds to the same epitope of M-CSF as 5H4. In another embodiment, a non-murine monoclonal antibody that competes with 5H4 for binding to M-CSF is provided. Preferably, the non-murine monoclonal antibody competes with 5H4 for binding to M-CSF by more than 10%, more preferably by more than 25%, still more preferably by more than 50%, even more preferably by more than 75%, and most preferably more than 90%.

It is contemplated that any of the aforementioned antibodies can be used to administer to a subject in need thereof. Accordingly, one embodiment discloses a pharmaceutical composition comprising any one of the aforementioned antibodies and a pharmaceutically acceptable carrier, excipient or diluent.

A non-murine antibody for treating a subject afflicted with a metastatic cancer is disclosed in one embodiment, wherein the antibody effectively reduces the severity of bone loss associated with the metastatic cancer. In a related embodiment, the aforementioned antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies. In another embodiment, the antibody is specific to M-CSF. In yet another embodiment, the antibody is specific to M-CSFR. In other embodiments, the antibody is a fully human antibody or a humanized antibody. In still another embodiment a hybridoma is disclosed that secretes an aforementioned antibody.

A number of metastatic cancers are contemplated to be amenable to the antibodies disclosed herein. In one embodiment, the metastatic cancer is breast, lung, renal, multiple myeloma, thyroid, prostate, adenocarcinoma, blood cell malignancies, including leukemia and lymphoma; head and neck cancers; gastrointestinal cancers, including stomach cancer, colon cancer, colorectal cancer, pancreatic cancer, liver cancer; malignancies of the female genital tract, including ovarian carcinoma, uterine endometrial cancers and cervical cancer; bladder cancer; brain cancer, including neuroblastoma; sarcoma, osteosarcoma; and skin cancer, including malignant melanoma or squamous cell cancer. In a related embodiment, a pharmaceutical composition comprising an aforementioned antibody and a pharmaceutically suitable carrier, excipient or diluent is provided.

M-CSF antagonists potentially useful in preventing or treating bone loss associated with cancer metastasis may be screened using various assays. In one embodiment, a method of screening for an M-CSF antagonist is disclosed comprising the steps of contacting metastatic tumor cell medium, osteoclasts and a candidate antagonist; detecting osteoclast formation, proliferation and/or differentiation; and identifying said candidate antagonist as an M-CSF antagonist if a decrease in osteoclast formation, proliferation and/or differentiation is detected. In a related embodiment, a method of screening is contemplated wherein the metastatic tumor cell medium includes tumor cells.

In another embodiment, a method is disclosed wherein the contacting step occurs *in vivo,* the detecting step comprises detecting size and/or number of bone metastases, and the candidate antagonist is identified as an M-CSF antagonist if a decrease in size and/or number of bone metastases is detected. In a related embodiment, the method further comprises the step of determining if said candidate antagonist binds to M-CSF. In yet another embodiment, the method further comprises the step of determining if said candidate antagonist inhibits interaction between M-CSF and its receptor M-CSFR.

It is contemplated that a number of different antagonists can be identified using the screening methods described herein. In one embodiment, a method is provided wherein the candidate antagonist is selected from the group consisting of: a polypeptide comprising an anti-M-CSF antibody; a polypeptide comprising an anti-M-CSFR antibody thereof; a soluble polypeptide comprising an M-CSF mutein or derivative thereof; a soluble polypeptide comprising an M-CSFR mutein or derivative thereof; a peptide; or a small molecule. In a related embodiment, a method is disclosed wherein said candidate antagonist is an M-CSF mutein. In yet another related embodiment, a method is provided wherein said candidate antagonist is an anti-M-CSF antibody. In another embodiment, the candidate antagonist is an M-CSF mutein. In another related embodiment, the candidate antagonist is an anti-M-CSF antibody. In yet another embodiment, the candidate antagonist is an anti-M-CSFR antibody.

Another embodiment discloses a method of identifying an M-CSF antagonist that can prevent or treat metastatic cancer to bone, comprising the steps of: detecting binding of a candidate antagonist to M-CSF; and assaying the ability of said candidate antagonist to prevent or treat metastatic cancer to bone *in vitro* or *in vivo.* In a related embodiment, a method of identifying an M-CSF antagonist that can prevent or treat metastatic cancer to bone is disclosed, comprising the steps of: detecting binding of a candidate antagonist to M-CSFR; and assaying the ability of said candidate antagonist to prevent or treat metastatic cancer to bone *in vitro* or *in vivo.* In yet another embodiment, a method of identifying an M-CSF antagonist that can prevent or treat metastatic cancer to bone is disclosed, comprising the steps of: identifying a candidate antagonist that inhibits the interaction between M-CSF and M-CSFR; and assaying the ability of said candidate antagonist to prevent or treat metastatic cancer to bone *in vitro* or *in vivo.*

It may be further advantageous to mix two or more M-CSF antagonists together to provide improved efficacy against cancer metastasis and/or bone loss associated with cancer metastasis. Accordingly, one embodiment discloses a method of preventing bone metastases and tumor growth comprising administering to a subject afflicted with metastatic cancer therapeutically effective amounts of M-CSF antagonist and a therapeutic agent, thereby preventing bone loss associated with the metastatic cancer and preventing tumor growth. Similarly, another embodiment discloses a method of treating a subject afflicted with a metastatic cancer comprising administering to the subject therapeutically effective amounts of M-CSF antagonist and a therapeutic agent, thereby reducing the severity of bone loss associated with the metastatic cancer and inhibiting tumor growth. In a related aspect, the subject of the aforementioned method is a mammal. In yet another embodiment, the subject is a human.

In another embodiment, the aforementioned methods are disclosed wherein the antagonist inhibits the interaction between M-CSF and its receptor M-CSFR. In another embodiment, the antagonist inhibits osteoclast proliferation and/or differentiation induced by tumor cells. In yet another embodiment, the M-CSF antagonist is selected from the group consisting of: a polypeptide comprising an anti-M-CSF antibody; a polypeptide comprising an anti-M-CSFR antibody thereof; a soluble polypeptide comprising an M-CSF mutein or derivative thereof; and a soluble polypeptide comprising an M-CSFR mutein or derivative thereof.

A number of M-CSF antagonist antibodies are contemplated. In one embodiment, the aforementioned methods are disclosed wherein said antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies.

In another embodiment, the aforementioned methods are disclosed wherein the therapeutic agent is a bisphosphonate. In a further embodiment, the bisphophonate is zeledronate, pamidronate, clodronate, etidronate, tilundronate, alendronate, or ibandronate. In another embodiment, the aforementioned methods are disclosed wherein the therapeutic agent is a chemotherapeutic agent. It is contemplated that some subjects may be precluded from receiving bisphosphonate treatment. By way of example, a subject may be precluded from receiving bisphosphonate treatment if the subject does not adequately respond to bisphosphonate, if the subject does not tolerate bisphosphonate treatment, or if bisphosphonate is contraindicated for the subject's particular symptoms (e.g., renal failure). Cancer chemotherapeutic agents include, without limitation, alkylating agents, such as carboplatin and cisplatin; nitrogen mustard alkylating agents; nitrosourea alkylating agents, such as carmustine (BCNU); antimetabolites, such as methotrexate; purine analog antimetabolites, mercaptopurine; pyrimidine analog antimetabolites, such as fluorouracil (5-FU) and gemcitabine; hormonal antineoplastics, such as goserelin, leuprolide, and tamoxifen; natural antineoplastics, such as aldesleukin, interleukin-2, docetaxel, etoposide (VP-16), interferon alfa, paclitaxel, and tretinoin (ATRA); antibiotic natural antineoplastics, such as bleomycin, dactinomycin, daunorubicin, doxorubicin, and mitomycin; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide, Schizophyllan, cytarabine, dacarbazine, thioinosine, thiotepa, tegafur, , neocarzinostatin, OK-432, bleomycin, furtulon, broxuridine, busulfan, honvan, peplomycin, , Bestatin (ubenimex), interferon-β, mepitiostane, mitobronitol, merphalan, laminin peptides, lentinan, Coriolus versicolor extract, tegafur/uracil, estramustine (estrogen/mechlorethamine).

Further, additional agents used as adjunctive therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ.; hormones, such as luteinizing hormone releasing hormone (LHRH) and analogues and, gonadotropin releasing hormone (GnRH); growth factors, such as transforming growth factor- β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor- α. & β (TNF- α & β); invasion inhibiting factor-2 (IIF-2); bone morphogenetic proteins 1-7 (BMP 1-7); somatostatin; thymosin- α-1; γ-globulin; superoxide dismutase (SOD); complement factors; anti-angiogenesis factors; antigenic materials; pro-drugs; growth factor receptor kinase inhibitors; anti-Her2 anitbody; and VEGF neutralizing antibody.

In a related embodiment, the M-CSF antagonist is effective to reduce the dosage of therapeutic agent required to achieve a therapeutic effect. Thus, an M-CSF antagonist may improve efficacy of the second therapeutic agent, or reduce side effects associated with administration of the second therapeutic agent, or improve the safety of the second therapeutic agent. An M-CSF antagonist may also improve efficacy, reduce side effects of, or improve safety of a second therapeutic modality such as surgery or radiation therapy. In yet another related embodiment, the aforementioned methods further comprise the step of administering a non-M-CSF colony stimulating factor, for example G-CSF. In still another embodiment, a pharmaceutical composition is disclosed comprising a M-CSF antagonist and a cancer therapeutic agent.

In another embodiment, a package, vial or container is disclosed comprising a medicament comprising an M-CSF antagonist and instructions that the medicament should be used in combination with surgery or radiation therapy. In another embodiment, a method of preventing or treating metastatic cancer to bone is disclosed, comprising the steps of administering an M-CSF antagonist to a subject and treating said subject with surgery or radiation therapy.

It is contemplated that M-CSF antagonists may be useful as immunotherapeutic agents. Accordingly, one embodiment discloses a method of targeting a tumor cell expressing membrane-bound M-CSF on its surface comprising the step of administering an antibody that specifically binds to the extracellular portion of membrane-bound M-CSF [SEQ ID NO: 2]. In another embodiment, the antibody is conjugated to a radionuclide or other toxin. In yet another embodiment, the antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies.

In yet further aspects, the disclosure contemplates immunotherapy-based methods of treating cancer by using anti-M-CSF antibodies to target tumor cells expressing membrane-bound M-CSF on their surface. The examples herein show that a variety of cancer cells express the membrane-bound form of M-CSF, including but not limited to breast cancer, colon cancer, kidney cancer, liver cancer, lung cancer, lymphoma, melanoma, ovarian cancer, pancreatic cancer, prostate cancer, osteosarcoma and thyroid cancer. Thus, the disclosure contemplates methods of killing or inhibiting growth of tumor cells expressing membrane-bound M-CSF comprising the step of administering an effective amount of an antibody that binds to M-CSF, alone or conjugated to a cytotoxic moiety. While antibodies specific for membrane-bound M-CSF are preferred, any antibody that binds to the extracellular portion of membrane-bound M-CSF is useful according to these methods.

It is also contemplated that any one of the M-CSF antagonists of the disclosure may be useful for treating cancer, including non-metastatic cancer. In other aspects, the disclosure contemplates use of any one of the antagonists of the invention for the treatment of hypercalcemia, Paget's disease or osteoporosis.

In one embodiment, a method of treating a subject suffering from a cancer is disclosed, wherein the cells comprising said cancer do not secrete M-CSF, comprising the step of administering an M-CSF antagonist. In a related embodiment, a method is disclosed for preventing bone metastases comprising administering to a subject afflicted with metastatic cancer an amount of M-CSF antagonist effective to neutralize M-CSF produced by the subject's cells, the amount being larger than the amount effective to neutralize M-CSF produced by the cancer cells. In yet another embodiment, a method of treating a subject afflicted with a metastatic cancer to bone is disclosed, comprising administering to the subject an amount of M-CSF antagonist effective to neutralize M-CSF produced by the subject's cells, the amount being larger than the amount effective to neutralize M-CSF produced by the cancer cells.

Numerous uses are contemplated. By way of example, one embodiment discloses any of the aforementioned antibodies for use in medicine. Similarly, a use of a M-CSF antagonist in the manufacture of a medicament is disclosed for preventing bone metastases in a subject afflicted with metastatic cancer. In another embodiment, a use of a M-CSF antagonist in the manufacture of a medicament for preventing, in a subject afflicted with metastatic cancer, bone loss associated with the cancer is disclosed. In still another embodiment, a use of a M-CSF antagonist in the manufacture of a medicament is disclosed for treating a subject afflicted with a metastatic cancer to bone. In yet another embodiment, a use of a M-CSF antagonist in the manufacture of a medicament for reducing, in a subject afflicted with a metastatic cancer to bone, the severity of bone loss associated with the cancer is disclosed.

Numerous subjects are contemplated to benefit by the methods and uses of M-CSF antagonists described herein. In one embodiment, the subject is a mammal. In another embodiment, the mammal is human.

In another embodiment, the aforementioned uses are isclosed wherein the antagonist inhibits the interaction between M-CSF and its receptor (M-CSFR). In another embodiment, the antagonist inhibits osteoclast proliferation and/or differentiation induced by tumor cells. In still another embodiment, the M-CSF antagonist is selected from the group consisting of: a polypeptide comprising an anti-M-CSF antibody; a polypeptide comprising an anti-M-CSFR antibody thereof; a soluble polypeptide comprising an M-CSF mutein or derivative thereof; or a soluble polypeptide comprising an M-CSFR mutein or derivative thereof. In a related embodiment, the antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies or fragments.

In another embodiment, the aforementioned uses are disclosed wherein the antibody is specific to M-CSF. In a related embodiment, the antibody is antibody 5H4. In still another related embodiment, the antibody is specific to M-CSFR.

Numerous metastatic cancers are contemplated as targets in connection with the aforementioned uses. In one embodiment, the metastatic cancer is breast, lung, renal, multiple myeloma, thyroid, prostate, adenocarcinoma, blood cell malignancies, including leukemia and lymphoma; head and neck cancers; gastrointestinal cancers, including stomach cancer, colon cancer, colorectal cancer, pancreatic cancer, liver cancer; malignancies of the female genital tract, including ovarian carcinoma, uterine endometrial cancers and cervical cancer; bladder cancer; brain cancer, including neuroblastoma; sarcoma, osteosarcoma; and skin cancer, including malignant melanoma or squamous cell cancer. In another embodiment, the M-CSF antagonist of the aforementioned uses is an antibody administered at a dose between about 0.01 mg/kg and about 100 mg/kg.

Numerous medicaments are contemplated. By way of example, the aforementioned uses are disclosed in the manufacture of a medicament for treating a subject afflicted with metastatic cancer. In another embodiment, the use of the antibody in the manufacture of a medicament for reducing, in a subject afflicted with a metastatic cancer, the severity of bone loss associated with the cancer is provided. In yet another embodiment, a use of a M-CSF antagonist and a therapeutic agent in the manufacture of a medicament is disclosed for preventing, in a subject afflicted with metastatic cancer, bone metastases and tumor growth. In another embodiment, a use of a M-CSF antagonist and a therapeutic agent in the manufacture of a medicament is disclosed for preventing, in a subject afflicted with metastatic cancer, bone loss associated with the cancer.

In another embodiment, a use of a M-CSF antagonist and a therapeutic agent in the manufacture of a medicament for treating a metastatic cancer is disclosed. In another embodiment, a use of a M-CSF antagonist and a therapeutic agent in the manufacture of a medicament is disclosed for reducing the severity of bone loss associated with the cancer and inhibiting tumor growth in a subject afflicted with metastatic cancer. In yet another embodiment, a product comprising an M-CSF antagonist and a therapeutic agent as a combined preparation for simultaneous, separate or sequential use in treating cancer is disclosed.

In another embodiment, a use of an M-CSF antagonist in preparation of a medicament for preventing or treating metastatic cancer to bone, wherein the medicament is simultaneously separately or sequentially administered with a cancer therapeutic agent is disclosed. In another embodiment, a use of a cancer therapeutic agent in preparation of a medicament is disclosed for preventing or treating metastatic cancer to bone, wherein the medicament is simultaneously separately or sequentially administered with an M-CSF antagonist. In still another embodiment, a package, vial or container is provided comprising a medicament comprising an M-CSF antagonist and instructions that the medicament should be used in combination with surgery or radiation therapy. Further, the aforementioned uses are disclosed wherein the subject is a mammal. Even further, the aforementioned uses are disclosed wherein the mammal is human. In another embodiment, the antagonist of the aforementioned uses inhibits the interaction between M-CSF and its receptor M-CSFR. In still another embodiment, the antagonist inhibits osteoclast proliferation and/or differentiation induced by tumor cells.

In another embodiment, the aforementioned uses are disclosed wherein said M-CSF antagonist is selected from the group consisting of: a polypeptide comprising an anti-M-CSF antibody; a polypeptide comprising an anti-M-CSFR antibody thereof; a soluble polypeptide comprising an M-CSF mutein or derivative thereof; or a soluble polypeptide comprising an M-CSFR mutein or derivative thereof. Further, another embodiment provides that the antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned anybodies.

Numerous therapeutic agents are contemplated. In one embodiment, the aforementioned uses are disclosed wherein the therapeutic agent is a bisphosphonate. Further, in another embodiment the bisphosphonate is zeledronate, pamidronate, clodronate, etidronate, tilundronate, alendronate, or ibandronate. In another embodiment, the therapeutic agent is a chemotherapeutic agent. In a related embodiment, the subject is precluded from receiving bisphophonate treatment.

In another embodiment, a use of a M-CSF antagonist in the manufacture of a medicament for reducing the dose of a therapeutic agent administered to a subject to treat or prevent bone metastases and tumor growth is disclosed. In another embodiment, a use of a M-CSF antagonist, a therapeutic agent, and a non-M-CSF colony stimulating factor in the manufacture of a medicament for preventing, in a subject afflicted with metastatic cancer, bone metastases and tumor growth is disclosed. In yet another embodiment, a use of a M-CSF antagonist, a therapeutic agent, and a non-M-CSF colony stimulating factor in the manufacture of a medicament is disclosed for preventing, in a subject afflicted with metastatic cancer, bone loss associated with the cancer.

In another embodiment, a use of a M-CSF antagonist, a therapeutic agent, and a non-M-CSF colony stimulating factor in the manufacture of a medicament for treating a metastatic cancer is disclosed. In another embodiment, a use of a M-CSF antagonist, a therapeutic agent, and a non-M-CSF colony stimulating factor in the manufacture of a medicament is disclosed for reducing the severity of bone loss associated with the cancer and inhibiting tumor growth in a subject afflicted with metastatic cancer. In a related embodiment, the aforementioned uses are provided wherein the non-M-CSF colony stimulating factor is G-CSF.

Another embodiment discloses a use of an antibody that specifically binds to the extracellular portion of membrane-bound M-CSF in the manufacture of a medicament for targeting a tumor cell that expresses membrane-bound M-CSF on its surface. In another embodiment, a use of an antibody that (a) specifically binds to the extracellular portion of membrane-bound M-CSF, and (b) is conjugated to a radionuclide or other toxin in the manufacture of a medicament for treating cancer is disclosed. In a related embodiment, the aforementioned use is disclosed wherein said antibody is selected from the group consisting of: a polyclonal antibody; a monoclonal antibody; a humanized antibody; a human antibody; a chimeric antibody; Fab, F(ab')₂ or Fᵥ antibody fragment; and a mutein of any one of the aforementioned antibodies.

In another embodiment, a use of a non-murine anti-M-CSF antibody in the manufacture of a medicament for treating cancer is disclosed. In a related embodiment, the cells comprising the cancer do not secrete M-CSF. In another embodiment, a use of a M-CSF antagonist, in an amount that is larger that the amount effective to neutralize M-CSF produced by cancer cells, in the manufacture of a medicament for preventing bone metastases is disclosed. In another embodiment, a use of a M-CSF antagonist, in an amount that is larger that the amount effective to neutralize M-CSF produced by cancer cells, in the manufacture of a medicament for neutralizing M-CSF produced by a subject's cells is disclosed. In yet another embodiment, a use of a M-CSF antagonist, in an amount that is larger that the amount effective to neutralize M-CSF produced by cancer cells, in the manufacture of a medicament for treating a subject afflicted with a metastatic cancer to bone. In still another embodiment, a use of a M-CSF antagonist, in an amount that is larger than the amount effective to neutralize M-CSF produced by cancer cells, in the manufacture of a medicament for treating cancer is disclosed.

Kits are also contemplated. A typical kit can comprise a package, container or vial comprising an M-CSF antagonist of the invention and instructions, such as a product insert or label, directing the user to utilize the pharmaceutical formulation according to any of the methods of the present disclosure.

### BRIEF DESCRIPTION OF THE DRAWING

Figure 1 is a topology diagram showing the disulfide bonds in truncated dimeric M-CSF.
Figure 2 is a stereodiagram of the C-alpha backbone with every tenth residue labeled and with the non-crystallographic symmetry axis indicated by a dotted line.
Figure 3 is a comparison of osteoclast inducing activity between purified M-CSF and conditioned medium (CM) from MDA 231 cells and MCF7 cells.
Figure 4 is a comparison of the neutralizing activity of a monoclonal antibody 5H4 against purified M-CSF relative to CM from MDA231 cells.
Figure 5 shows neutralizing activity of 5H4 and other antibodies against human M-CSF.
Figure 6 shows that the number of animals with a mean osteolysis score of ≥ 2.5 was lowest in the group that received the combination of 5A1 + 5H4 antibodies.
Figure 7 shows that the number of animals with a mean osteolysis score of ≥ 2.5 was lowest in the group that received the combination of 5A1 + 5H4 antibodies.
Figure 8 shows that an M-CSF-specific antibody bound to breast cancer cell line MDA231 or to multiple myeloma cancer cell line ARH77.
Figure 9 shows that M-CSF is prevalent on a number of cancer cell surfaces.

### DETAILED DESCRIPTION

The ability to metastasize is a defining characteristic of a cancer. Metastasis refers to the spread of cancer cells to other parts of the body or to the condition produced by this spread. Metastasis is a complex multi-step process that includes changes in the genetic material of a cell, uncontrolled proliferation of the altered cell to form a primary tumor, development of a new blood supply for the primary tumor, invasion of the circulatory system by cells from the primary tumor, dispersal of small clumps of primary tumor cells to other parts of the body, and the growth of secondary tumors in those sites.

Bone is one of the most common sites of metastasis in human breast, lung, prostate and thyroid cancers, as well as other cancers, and in autopsies as many as 60% of cancer patients are found to have bone metastasis. Osteolytic bone metastasis shows a unique step of osteoclastic bone resorption that is not seen in metastasis to other organs. Bone loss associated with cancer metastasis is mediated by osteoclasts (multinucleated giant cells with the capacity to resorb mineralized tissues), which seem to be activated by tumor products.

Colony stimulating factor (CSF 1), also known as macrophage colony stimulating factor (M-CSF), has been found crucial for osteoclast formation. In addition, M-CSF has been shown to modulate the osteoclastic functions of mature osteoclasts, their migration and their survival in cooperation with other soluble factors and cell to cell interactions provided by osteoblasts and fibroblasts (Fixe and Praloran, Cytokine 10: 3-7, 1998; Martin et al., Critical Rev. in Eukaryotic Gene Expression 8: 107-23 (1998)).

The full-length human M-CSF mRNA encodes a precursor protein of 554 amino acids (SEQ ID NO: 4). Through alternative mRNA splicing and differential post-translational proteolytic processing, M-CSF can either be secreted into the circulation as a glycoprotein or chondroitin sulfate containing proteoglycan or be expressed as a membrane spanning glycoprotein on the surface of M-CSF producing cells. The three-dimensional structure of the bacterially expressed amino terminal 150 amino acids of human M-CSF, the minimal sequence required for full in vitro biological activity, indicates that this protein is a disulfide linked dimer with each monomer consisting of four alpha helical bundles and an anti-parallel beta sheet (Pandit et al., Science 258: 1358-62 (1992)). Three distinct M-CSF species are produced through alternative mRNA splicing. The three polypeptide precursors are M-CFSα of 256 amino acids (DNA and amino acid sequence set forth in SEQ ID NOS: 1 and 2), M-CSFβ of 554 amino acids (DNA and amino acid sequence set forth in SEQ ID NOS: 3 and 4), and M-CSFγ of 438 amino acids (DNA and amino acid sequence set forth in SEQ ID NOS: 5 and 6). M-CSFβ is a secreted protein that does not occur in a membrane-bound form. M-CSFα is expressed as an integral membrane protein that is slowly released by proteolytic cleavage. M-CSFα is cleaved at amino acids 191-197 of SEQ ID NO: 2. The membrane-bound form of M-CSF can interact with receptors on nearby cells and therefore mediates specific cell-to-cell contacts.

Various forms of M-CSF function by binding to its receptor M-CSFR on target cells. M-CSFR (DNA and amino acid sequence set forth in SEQ ID NOS: 7 and 8) is a membrane spanning molecule with five extracellular immunoglobulin-like domains, a transmembrane domain and an intracellular interrupted Src related tyrosine kinase domain. M-CSFR is encoded by the *c*-*fms* proto-oncogene. Binding of M-CSF to the extracellular domain of M-CSFR leads to dimerization of the receptor, which activates the cytoplasmic kinase domain, leading to autophosphorylation and phosphorylation of other cellular proteins (Hamilton J. A., J Leukoc Biol.,62(2):145-55 (1997); Hamilton J, A., Immuno Today., 18(7): 313-7(1997).

Phosphorylated cellular proteins induce a cascade of biochemical events leading to cellular responses: mitosis, secretion of cytokines, membrane ruffling, and regulation of transcription of its own receptor (Fixe and Praloran, Cytokine 10: 32-37 (1998)).

M-CSF is expressed in stromal cells, osteoblasts, and other cells. It is also expressed in breast, uterine, and ovarian tumor cells. The extent of expression in these tumors correlates with high grade and poor prognosis (Kacinski Ann. Med. 27: 79-85 (1995); Smith et al., Clin. Cancer Res. 1: 313-25 (1995)). In breast carcinomas, M-CSF expression is prevalent in invasive tumor cells as opposed to the intraductal (pre-invasive) cancer (Scholl et al., J. Natl. Cancer Inst. 86: 120-6 (1994)). In addition, M-CSF is shown to promote progression of mammary tumors to malignancy (Lin et al., J. Exp. Med. 93: 727-39 (2001)). For breast and ovarian cancer, the production of M-CSF seems to be responsible for the recruitment of macrophages to the tumor.

There exists no report of using a M-CSF antagonist in preventing or treating cancer metastasis or bone loss associated with cancer metastasis. It has been discovered, as part of the present invention, that M-CSF antagonists neutralize osteoclast induction by metastatic cancer cells. Thus, the present invention provides compositions and methods for treating or preventing cancer metastasis and bone loss associated with cancer metastasis.

Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma,; lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells, or render the tumor cells more susceptible to treatment by other therapeutic agents, e.g., radiation and/or chemotherapy. The "pathology" of cancer includes all phenomena that compromise the well being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

As used herein, the phrase "metastatic cancer" is defined as cancers that have potential to spread to other areas of the body, particularly bone. A variety of cancers can metastasize to the bone, but the most common metastasizing cancers are breast, lung, renal, multiple myeloma, thyroid and prostate. By way of example, other cancers that have the potential to metastasize to bone include but are not limited to adenocarcinoma, blood cell malignancies, including leukemia and lymphoma; head and neck cancers; gastrointestinal cancers, including stomach cancer, colon cancer, colorectal cancer, pancreatic cancer, liver cancer; malignancies of the female genital tract, including ovarian carcinoma, uterine endometrial cancers and cervical cancer; bladder cancer; brain cancer, including neuroblastoma; sarcoma, osteosarcoma; and skin cancer, including malignant melanoma and squamous cell cancer. The present invention especially contemplates prevention and treatment of tumor-induced osteolytic lesions in bone.

### I. Antagonists

As used herein, the term "antagonist" generally refers to the property of a molecule, compound or other agent to, for example, interfere with the binding of one molecule with another molecule or the stimulation of one cell by another cell either through steric hindrance, conformational alterations or other biochemical mechanisms. In one regard, the term antagonist relates to the property of an agent to prevent the binding of a receptor to its ligand, e.g., the binding of M-CSF with M-CSFR, thereby inhibiting the signal transduction pathway triggered by M-CSF. The term antagonist is not limited by any specific action mechanism, but, rather, refers generally to the functional property presently defined. Antagonists of the present invention include, but are not limited to: M-CSF antibodies and fragments and muteins and modifications thereof, soluble M-CSF and fragments and muteins and modifications thereof, M-CSFR antibodies and fragments and muteins and modifications thereof, soluble M-CSFR and fragments and muteins and modifications thereof, and peptides as well as other chemical compounds and molecules that bind to M-CSF or M-CSFR and antisense compounds that inhibit expression of M-CSF and M-CSFR. Antagonists of the present invention optionally exclude antisense molecules that target M-CSF. Any of the antagonists of the present invention can be administered in any manner known in the art. For example, M-CSF muteins, M-CSFR muteins or antibody fragments that bind to M-CSF or M-CSFR can be administered via gene therapy.

M-CSF antagonists of the present invention include, where applicable, functional equivalents. For example, molecules may differ in length, structure, components, etc., but may still retain one or more of the defined functions. More particularly, functional equivalents of the antibodies, antibody fragments or peptides of the present invention may include mimetic compounds, i.e., constructs designed to mimic the proper configuration and/or orientation for antigen binding.

Preferred M-CSF antagonists may optionally be modified by addition of side groups, etc., e.g., by amino terminal acylation, carboxy terminal amidation or by coupling of additional groups to amino acid side chains. Antagonists may also comprise one or more conservative amino acid substitutions. By "conservative amino acid substitutions" is meant those changes in amino acid sequence that preserve the general charge, hydrophobicity/hydrophilicity and/or steric bulk of the amino acid substituted. For example, substitutions between the following groups are conservative: Gly/Ala, Val/Ile/Leu, Asp/Glu, Lys/Arg, Asn/Gln, Ser/Cys/Thr, and Phe/Trp/Tyr. Such modifications will not substantially diminish the efficacy of the M-CSF antagonists and may impart such desired properties as, for example, increased *in vivo* half life or decreased toxicity.

The invention is also intended to include polypeptides bearing modifications other than the insertion, deletion, or substitution of amino acid residues. By way of example, the modifications may be covalent in nature, and include for example, chemical bonding with polymers, lipids, other organic, and inorganic moieties. Such derivatives may be prepared to increase circulating half-life of a polypeptide, or may be designed to improve targeting capacity for the polypeptide to desired cells, tissues, or organs. Similarly, the invention further embraces M-CSF or M-CSFR polypeptides that have been covalently modified to include one or more water soluble polymer attachments such as polyethylene glycol, polyoxyethylene glycol, or polypropylene glycol.

As used herein, the phrase "therapeutically effective amount" refers to an amount of therapeutic or prophylactic M-CSF antagonist that would be appropriate for an embodiment of the present invention, that will elicit the desired therapeutic or prophylactic effect or response when administered in accordance with the desired treatment regimen.

Human "M-CSF" as used herein refers to a human polypeptide having substantially the same amino acid sequence as the mature human M-CSFα, M-CSFβ, or M-CSFγ polypeptides described in Kawasaki et al., Science 230:291 (1985), Cerretti et al., Molecular Immunology, 25:761 (1988), or Ladner et al., EMBO Journal 6:2693 (198 7) Such terminology reflects the understanding that the three mature M-CSFs have different amino acid sequences, as described above, and that the active form of M-CSF is a disulfide bonded dimer; thus, when the term "M-CSF" refers to the biologically active form, the dimeric form is intended. "M-CSF dimer" refers to two M-CSF polypeptide monomers that have dimerized and includes both homodimers (consisting of two of the same type of M-CSF monomer) and heterodimers (consisting of two different monomers). M-CSF monomers may be converted to M-CSF dimers in vitro as described in U.S. Pat. No. 4,929,700.

### M-CSF Antibodies

The term "antibody" is used in the broadest sense and covers fully assembled antibodies, antibody fragments that can bind antigen (e.g., Fab', F'(ab)2, Fv, single chain antibodies, diabodies), and recombinant peptides comprising the forgoing.

The term "monoclonal antibody" as used herein refers to an antibody obtained from a population of substantially homogeneous antibodies, i.e., the individual antibodies comprising the population are identical except; for possible naturally occurring mutations that may be present in minor amounts. Monoclonal antibodies are highly specific, being directed against a single antigenic site. Furthermore, in contrast to conventional (polyclonal) antibody preparations that are typically include different antibodies directed against different determinants (epitopes), each monoclonal antibody is directed against a single determinant on the antigen. In addition to their specificity, the monoclonal antibodies are advantageous in that they are synthesized by the homogeneous culture, uncontaminated by other immunoglobulins with different specificities and characteristics.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 [19751, or may be made by recombinant DNA methods (see, e.g., U.S. Patent No. 4,816,567). The "monoclonal antibodies" may also be isolated from phage antibody libraries using the techniques described inClackson et al.,Nature,352:624628[1991] end Marks et al., J. Mol. Biol., 222:581-597 (1991), for example.

"Antibody fragments" comprise a portion of an intact antibody, preferably the antigen binding or variable region of the intact antibody. Examples of antibody fragments include Fab, Fab', F(ab')2, and Fv fragments; diabodies; linear antibodies (Zapata et al., Protein Eng.,8(10):1057-1062 (1995)); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two "Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains that enables the Fv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and 30 Hollinger et al., Proc. Natl. Acad. Sci. USA, 90:6444-6448 (1993).

An "isolated" antibody is one that has been identified and separated and for recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient too obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

"Fv" is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen binding site on the surface of the VH VI dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH 1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them.

By "neutralizing antibody" is meant an antibody molecule that is able to eliminate or significantly reduce an effecter function of a target antigen to which is binds. Accordingly, a "neutralizing" anti-target antibody is capable of eliminating or significantly reducing an effecter function, such as enzyme activity, ligand binding, or intracellular signaling.

As provided herein, the compositions for and methods of treating cancer metastasis and/or bone loss associated with cancer metastasis may utilize one or more antibody used singularly or in combination with other therapeutics to achieve the desired effects. Antibodies according to the present invention may be isolated from an animal producing the antibody as a result of either direct contact with an environmental antigen or immunization with the antigen. Alternatively, antibodies may be produced by recombinant DNA methodology using one of the antibody expression systems well known in the art (See, e.g., Harlow and Lane, Antibodies: A Laboratory Manual, Cold Spring Harbor Laboratory (1988)). Such antibodies may include recombinant IgGs, chimeric fusion proteins having immunoglobulin derived sequences or "humanized" antibodies that may all be used for the treatment of cancer metastasis and/or bone loss associated with cancer metastasis according to the present invention. In addition to intact, full-length molecules, the term "antibody" also refers to fragments thereof (such as, e.g., scFv, Fv, Fd, Fab, Fab' and F(ab)'2 fragments) or multimers or aggregates of intact molecules and/or fragments that bind to M-CSF (or M-CSFR). These antibody fragments bind antigen and may be derivatized to exhibit structural features that facilitate clearance and uptake, e.g., by incorporation of galactose residues.

In one embodiment of the present invention, M-CSF antagonists are monoclonal antibodies prepared essentially as described in Halenbeck et al. U.S. Pat. No. 5,491,065 (1997). Exemplary M-CSF antagonists include monoclonal antibodies that bind to an apparent conformational epitope associated with recombinant or native dimeric M-CSF with concomitant neutralization of biological activity. These antibodies are substantially unreactive with biologically inactive forms of M-CSF including monomeric and chemically derivatized dimeric M-CSF.

In other embodiments of the present invention, humanized anti-M-CSF monoclonal antibodies are provided. The phrase "humanized antibody" refers to an antibody derived from a non-human antibody, typically a mouse monoclonal antibody. Alternatively, a humanized antibody may be derived from a chimeric antibody that retains or substantially retains the antigen binding properties of the parental, non-human, antibody but which exhibits diminished immunogenicity as compared to the parental antibody when administered to humans. The phrase "chimeric antibody," as used herein, refers to an antibody containing sequence derived from two different antibodies (see, e.g., U.S. Patent No. 4,816,567) which typically originate from different species. Most typically, chimeric antibodies comprise human and murine antibody fragments, generally human constant and mouse variable regions.

The phrase "complementarity determining region" refers to amino acid sequences which together define the binding affinity and specificity of the natural Fv region of a native immunoglobulin binding site (See, e.g., Chothia et al., J. Mol. Biol. 196:901 917 (1987); Kabat et al., U.S. Dept. of Health and Human Services NIH Publication No. 91 3242 (1991)). The phrase "constant region" refers to the portion of the antibody molecule that confers effector functions. In the present invention, mouse constant regions are preferably substituted by human constant regions. The constant regions of the subject humanized antibodies are derived from human immunoglobulins. The heavy chain constant region can be selected from any of the five isotypes: alpha, delta, epsilon, gamma or mu.

The antibodies of the present invention are said to be immunospecific or specifically binding if they bind to antigen with a Kₐ of greater than or equal to about 104M⁻¹, preferably of greater than or equal to about 10⁵M⁻¹, more preferably of greater than or equal to about 10⁶M⁻¹ and still more preferably of greater than or equal to about 10⁷M⁻¹, and most preferably of greater than or equal to about 10⁸M⁻¹,10⁹M⁻¹, or 10¹⁰M⁻¹. For example, anti-M-CSF/M-CSFR specific antibodies suitable bind to their antigen with an affinity of at least 10⁴M⁻¹, preferably of greater than or equal to about 10⁵M⁻¹, more preferably of greater than or equal to about 10⁶M⁻¹ and still more preferably of greater than or equal to about 10⁷M⁻¹, and most preferably of greater than or equal to about 10⁸M⁻¹,10⁹M⁻¹, or 10¹⁰M⁻¹. The anti-M-CSF/M-CSFR antibodies bind to different naturally occurring forms of M-CSF/M-CSFR, including those expressed by the host's/subject's tissues as well as that expressed by the tumor. The monoclonal antibodies disclosed herein have affinity for M-CSF and M-CSFR and are characterized by a dissociation equilibrium constant (Kd) of at least 10⁻⁴ M, preferably at least about 10-7 M to about 10-8 M, more preferably at least about 10⁻⁸ M to about 10⁻¹² M. Monoclonal antibodies and antigen-binding fragments thereof that are suitable for use in the methods of the invention are capable of specifically binding to M-CSF/M-CSFR. Such affinities may be readily determined using conventional techniques, such as by equilibrium dialysis; by using the BIAcore 2000 instrument, using general procedures outlined by the manufacturer; by radioimmunoassay using ¹²⁵I labeled M-CSF; or by another method known to the skilled artisan. The affinity data may be analyzed, for example, by the method of Scatchard et al., Ann N.Y. Acad. Sci., 51:660 (1949). Thus, it will be apparent that preferred M-CSF antagonists will exhibit a high degree of specificity for M-CSF and will bind with substantially lower affinity to other molecules.

The antigen to be used for production of antibodies may be, e.g., intact M-CSF or a fragment of M-CSF that retains the desired epitope, optionally fused to another polypeptide that allows the epitope to be displayed in its native conformation.

### Polyclonal Antibodies

Polyclonal antibodies are preferably raised in animals by multiple subcutaneous (sc) or intraperitoneal (ip) injections of the relevant antigen and an adjuvant. An improved antibody response may be obtained by conjugating the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride or other agents known in the art.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to {fraction (1/10)} the original amount of peptide or conjugate in Freund's complete adjuvant by subcutaneous injection at multiple sites. At 7-14 days post-booster injection, the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### Monoclonal Antibodies

Monoclonal antibodies may be made using the hybridoma method first described by Kohler et al., Nature, 256:495 (1975), or may be made by recombinant DNA methods.

In the hybridoma method, a mouse or other appropriate host animal, such as a hamster or macaque monkey, is immunized as herein described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium. Human myeloma and mouse-human heteromyeloma cell lines also have been described for the production of human monoclonal antibodies (Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by Scatchard analysis (Munson et al., Anal. Biochem., 107:220 (1980)).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies may be isolated and sequenced from the hybridoma cells using conventional procedures (e.g., by using oligonucleotide probes that are capable of binding specifically to genes encoding the heavy and light chains of the monoclonal antibodies). Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies is well known in the art.

Amino acid sequence variants of the desired antibody may be prepared by introducing appropriate nucleotide changes into the encoding DNA, or by peptide synthesis. Such variants include, for example, deletions from, and/or insertions into and/or substitutions of, residues within the amino acid sequences of the antibodies. Any combination of deletion, insertion, and substitution is made to arrive at the final construct, provided that the final construct possesses the desired characteristics. The amino acid changes also may alter post-translational processes of the humanized or variant antibody, such as changing the number or position of glycosylation sites.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Because chimeric or humanized antibodies are less immunogenic in humans than the parental mouse monoclonal antibodies, they can be used for the treatment of humans with far less risk of anaphylaxis. Thus, these antibodies may be preferred in therapeutic applications that involve in vivo administration to a human.

Chimeric monoclonal antibodies, in which the variable Ig domains of a mouse monoclonal antibody are fused to human constant Ig domains, can be generated using standard procedures known in the art (See Morrison, S. L., et al. (1984) Chimeric Human Antibody Molecules; Mouse Antigen Binding Domains with Human Constant Region Domains, Proc. Natl. Acad. Sci. USA 81, 6841-6855; and, Boulianne, G. L., et al, Nature 312, 643-646 . (1984)). Although some chimeric monoclonal antibodies have proved less immunogenic in humans, the mouse variable Ig domains can still lead to a significant human anti-mouse response.

Humanized antibodies may be achieved by a variety of methods including, for example: (1) grafting the non-human complementarity determining regions (CDRs) onto a human framework and constant region (a process referred to in the art as humanizing through "CDR grafting"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. These methods are disclosed in, e.g., Jones et al., Nature 321:522 525 (1986); Morrison et al., Proc. Natl. Acad. Sci., U.S.A., 81:6851 6855 (1984); Morrison and Oi, Adv. Immunol., 44:65 92 (1988); Verhoeyer et al., Science 239:1534 1536 (1988); Padlan, Molec. Immun. 28:489 498 (1991); Padlan, Molec. Immunol. 31(3):169 217 (1994); and Kettleborough, C.A. et al., Protein Eng. 4(7):773 83 (1991).

CDR grafting involves introducing one or more of the six CDRs from the mouse heavy and light chain variable Ig domains into the appropriate four framework regions of human variable Ig domains is also called CDR grafting. This technique (Riechmann, L., et al., Nature 332, 323 (1988)), utilizes the conserved framework regions (FR1-FR4) as a scaffold to support the CDR loops which are the primary contacts with antigen. A disadvantage of CDR grafting, however, is that it can result in a humanized antibody that has a substantially lower binding affinity than the original mouse antibody, because amino acids of the framework regions can contribute to antigen binding, and because amino acids of the CDR loops can influence the association of the two variable Ig domains. To maintain the affinity of the humanized monoclonal antibody, the CDR grafting technique can be improved by choosing human framework regions that most closely resemble the framework regions of the original mouse antibody, and by site-directed mutagenesis of single amino acids within the framework or CDRs aided by computer modeling of the antigen binding site (e.g., Co, M. S., et al. (1994), J. Immunol. 152, 2968-2976).

One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region which disturb the structure of the CDRs until the predicted conformation of the humanized sequence model closely approximates the conformation of the non-human CDRs of the parent non-human antibody. Such humanized antibodies may be further derivatized to facilitate uptake and clearance, e.g., via Ashwell receptors (See, e.g., U.S. Patent Nos. 5,530,101 and 5,585,089).

A number of humanizations of mouse monoclonal antibodies by rational design have been reported (See, for example, 20020091240 published July 11, 2002, WO 92/11018 and U.S. Patent No., 5,693,762, U.S. Patent No. 5,776,886.

A useful method for identification of certain residues or regions of the antibody that are preferred locations for mutagenesis is called "alanine scanning mutagenesis," as described by Cunningham and Wells Science, 244:1081-1085 (1989). Here, a residue or group of target residues are identified (e.g., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intra-sequence insertions of single or multiple amino acid residues. Examples of terminal insertions include an antibody with an N-terminal methionyl residue or the antibody fused to an epitope tag. Other insertional variants of the antibody molecule include the fusion to a polypeptide which increases the serum half-life of the antibody.

Another type of variant is an amino acid substitution variant. These variants have at least one amino acid residue in the antibody molecule removed and a different residue inserted in its place. Substitutional mutagenesis within any of the hypervariable or CDR regions or framework regions is contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

### TABLE 1

### Original Exemplary Preferred Residue Substitutions

Ala (A) val; leu; ile val Arg (R) lys; gln; asn lys Asn (N) gln; his; asp, lys; gln arg Asp (D) glu; asn glu Cys (C) ser; ala ser Gln (Q) asn; glu asn Glu (E) asp; gln asp Gly (G) ala His (H) asn; gln; lys; arg Ile (I) leu; val; met; ala; leu phe; norleucine Leu (L) norleucine; ile; val; ile met; ala; phe Lys (K) arg; gln; asn arg Met (M) leu; phe; ile leu Phe (F) leu; val; ile; ala; tyr Pro (P) ala Ser (S) thru Thr (T) ser ser Trp (W) tyr; phe tyr Tyr (Y) trp; phe; thr; ser phe Val (V) ile; leu; met; phe; leu ala; norleucine

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions involve replacing a member of one of these classes with a member of another class.

Any cysteine residue not involved in maintaining the proper conformation of the humanized or variant antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

Affinity maturation involves preparing and screening antibody variants that have substitutions within the CDRs of a parent antibody and selecting variants that have improved biological properties such as binding affinity relative to the parent antibody. A convenient way for generating such substitutional variants is affinity maturation using phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity).

Alanine scanning mutagenesis can be performed to identify hypervariable region residues that contribute significantly to antigen binding. Alternatively, or in addition, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Antibody variants can also be produced that have a modified glycosylation pattern relative to the parent antibody, for example, deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. The presence of either of these tripeptide sequences in a polypeptide creates a potential glycosylation site. Thus, N-linked glycosylation sites may be added to an antibody by altering the amino acid sequence such that it contains one or more of these tripeptide sequences. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. O-linked glycosylation sites may be added to an antibody by inserting or substituting one or more serine or threonine residues to the sequence of the original antibody.

Humanized or human antibodies to M-CSF can also be produced using transgenic animals that have no endogenous immunoglobulin production and are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/741 also discloses transgenic non-primate mammalian hosts capable of mounting an immune response to an immunogen, wherein the antibodies have primate constant and/or variable regions, and wherein the endogenous immunoglobulin encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions.

Using a transgenic animal described above, an immune response can be produced to a selected antigenic molecule, and antibody producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. This publication discloses monoclonal antibodies against a variety of antigenic molecules including IL 6, IL 8, TNFa, human CD4, L selectin, gp39, and tetanus toxin. The monoclonal antibodies can be tested for the ability to inhibit or neutralize the biological activity or physiological effect of the corresponding protein. WO 96/33735 discloses that monoclonal antibodies against IL-8, derived from immune cells of transgenic mice immunized with IL-8, blocked IL-8 induced functions of neutrophils. Human monoclonal antibodies with specificity for the antigen used to immunize transgenic animals are also disclosed in WO 96/34096 and U.S. patent application no. 20030194404; and U.S. patent application no. 20030031667)

See also Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Pat. No. 5,591,669, U.S. Patent No. 5,589,369, U.S. Patent No. 5,545,807; and U.S patent application no. 20020199213. U.S. patent application no. and 20030092125 describes methods for biasing the immune response of an animal to the desired epitope. Human antibodies may also be generated by *in vitro* activated B cells (see U.S. Pat. Nos. 5,567,610 and 5,229,275).

The development of technologies for making repertoires of recombinant human antibody genes, and the display of the encoded antibody fragments on the surface of filamentous bacteriophage, has provided a means for making human antibodies directly. The antibodies produced by phage technology are produced as antigen binding fragments-usually Fv or Fab fragments-in bacteria and thus lack effector functions. Effector functions can be introduced by one of two strategies: The fragments can be engineered either into complete antibodies for expression in mammalian cells, or into bispecific antibody fragments with a second binding site capable of triggering an effector function.

Typically, the Fd fragment (V_{H}-C_{H}1) and light chain (V_{L}-C_{L}) of antibodies are separately cloned by PCR and recombined randomly in combinatorial phage display libraries, which can then be selected for binding to a particular antigen. The Fab fragments are expressed on the phage surface, i.e., physically linked to the genes that encode them. Thus, selection of Fab by antigen binding co-selects for the Fab encoding sequences, which can be amplified subsequently. By several rounds of antigen binding and re-amplification, a procedure termed panning, Fab specific for the antigen are enriched and finally isolated.

In 1994, an approach for the humanization of antibodies, called "guided selection", was described. Guided selection utilizes the power of the phage display technique for the humanization of mouse monoclonal antibody (See Jespers, L. S., et al., Bio/Technology 12, 899-903 (1994)). For this, the Fd fragment of the mouse monoclonal antibody can be displayed in combination with a human light chain library, and the resulting hybrid Fab library may then be selected with antigen. The mouse Fd fragment thereby provides a template to guide the selection. Subsequently, the selected human light chains are combined with a human Fd fragment library. Selection of the resulting library yields entirely human Fab.

A variety of procedures have been described for deriving human antibodies from phage-display libraries (See, for example, Hoogenboom et al., J. Mol. Biol., 227:381 (1991); Marks et al., J. Mol. Biol, 222:581-597 (1991); U.S. Pat. Nos. 5,565,332 and 5,573,905; Clackson, T., and Wells, J. A., TIBTECH 12, 173-184 (1994)). In particular, in vitro selection and evolution of antibodies derived from phage display libraries has become a powerful tool (See Burton, D. R., and Barbas III, C. F., Adv. Immunol. 57, 191-280 (1994); and, Winter, G., et al., Annu. Rev. Immunol. 12, 433-455 (1994); U.S. patent application no. 20020004215 and WO92/01047; U.S. patent application no. 20030190317 published October 9, 2003 and U.S. Patent No. 6,054,287; U.S. Patent No. 5,877,293.

Watkins, "Screening of Phage-Expressed Antibody Libraries by Capture Lift," Methods in Molecular Biology, Antibody Phage Display: Methods and Protocols 178: 187-193, and U.S. patent application no. 200120030044772 published March 6, 2003 describe methods for screening phage-expressed antibody libraries or other binding molecules by capture lift, a method involving immobilization of the candidate binding molecules on a solid support.

The antibody products may be screened for activity as an MCSF antagonist and for suitability in the treatment methods of the invention using assays as described in the section entitled "Screening Methods" herein or using any suitable assays known in the art.

### M-CSF Muteins

"Fragment" as used herein means a portion of the intact native molecule; for example, a fragment polypeptide is a fragment of the native polypeptide in which one or more amino acids from either the N-terminal or C-terminal have been deleted.

"Mutein" as used herein with respect to polypeptides means a variant of the intact native molecule or a variant of a fragment of the native molecule, in which one or more amino acids have been substituted, inserted or deleted. Such substitutions, insertions or deletions can be at the N-terminus, C-terminus or internal to the molecule. Thus the term "muteins" includes within its scope fragments of the native molecule. Insertional muteins include fusions at the N- or C-terminus, e.g. fusion to the Fc portion of an immunoglobulin to increase half-life

Preferred muteins exhibit at least about 65%, 70%. 75%, 80%, 85%, 90%, 95%, 97% or more sequence identity (homology) to the native polypeptide, as determined by the Smith-Waterman homology search algorithm (Meth. Mol. Biol. 70:173-187 (1997)) as implemented in the MSPRCH program (Oxford Molecular) using an affine gap search with the following search parameters: gap open penalty of 12, and gap extension penalty of 1. Other well-known and routinely used homology/identity scanning algorithm programs include Pearson and Lipman, PNAS USA, 85:2444-2448 (1988); Lipman and Pearson, Science, 222:1435 (1985); Devereaux et al., Nuc. Acids Res., 12:387-395 (1984); or the BLASTP, BLASTN or BLASTX algorithms of Altschul, et al., Mol. Biol., 215:403-410 (1990). Computerized programs using these algorithms are also available and include, but are not limited to: GAP, BESTFIT, BLAST, FASTA and TFASTA, which are commercially available from the Genetics Computing Group (GCG) package, Version 8, Madison Wis., USA; and CLUSTAL in the PC/Gene program by Intellegenetics, Mountain View Calif. Preferably, the percentage of sequence identity is determined by using the default parameters determined by the program.

"Modification" as used herein means any modification of the native polypeptide, fragment or mutein, such as glycosylation, phosphorylation, polymer conjugation (such as with polyethylene glycol), or other addition of foreign moieties, so long as the desired activity (agonist or antagonist) is retained.

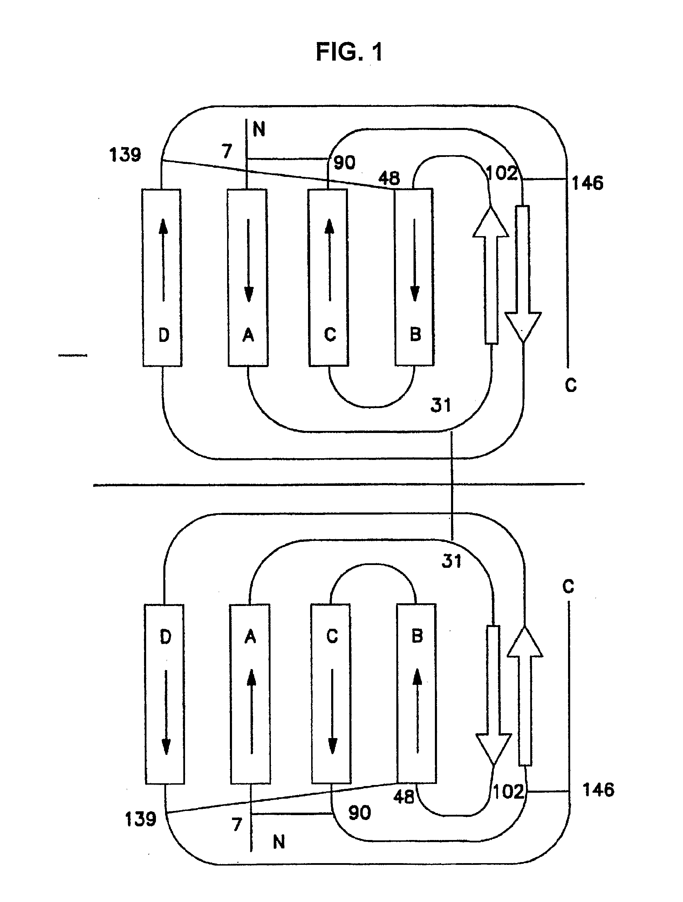

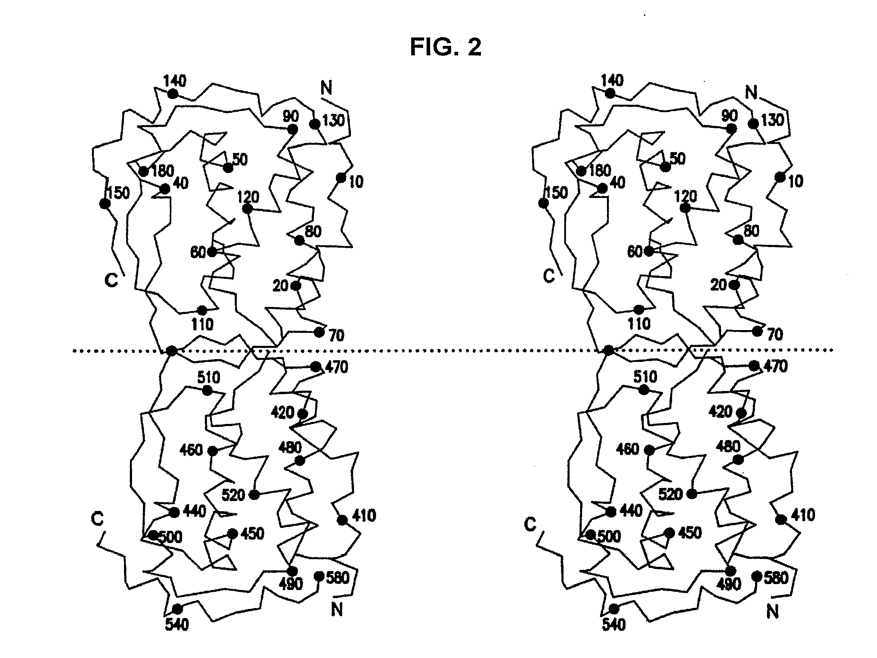

U.S. Patent No. 6,025,146, and Koths, Mol. Reprod. Dev. 1997 Jan;46(1):31-38, describe the crystallization of M-CSF alone and M-CSF complexed to MCSF-R, and characterize the three-dimensional structure of M-CSF as well as residues involved in receptor-binding. U.S. Patent No. 6,025,146 also describes methods for selecting candidate amino acid substitutions in M-CSF, based on structural information. FIG. 1 is a topology diagram showing the disulfide bonds in truncated dimeric M-CSF; FIG. 2 is a stereodiagram of the C-alpha backbone with every tenth residue labelled and with the non-crystallographic symmetry axis indicated by a dotted line. The overall topology of this form of M-CSF as shown in FIG. 1 is that of an antiparallel four alpha-helical bundle, in which the helices run up-up-down-down, unlike the more commonly observed up-down-up-down connectivity of most four helical bundles. A long crossover connection links helix A to helix B and a similar connection is found between helices C and D. In the disulfide-linked dimeric form, the bundles are linked end-to-end, forming an extremely flat, elongated structure (approximate dimensions 85 x 35 x 25 Ǻ). There are three intramolecular disulfide bonds in each monomer (Cys7-Cys90, Cys48-Cys139, Cys102-Cys146) all of which are at the distal end of the molecule. One interchain disulfide bond (Cys31--Cys31) is located at the dimer interface with the noncrystallographic two-fold symmetry axis passing through it as shown in FIG. 2. Mutation experiments indicate that all of the cysteine residues in this form of M-CSF may be necessary for full biological activity. The structure described herein suggests that their role is primarily structural rather than being related to receptor recognition. U.S. Patent No. 6,025,146 provides the three-dimensional structure of the truncated recombinant M-CSF α dimer as identified by the alpha-carbon positions of the amino acid residues in the sequence.

Specific residues in helices A, C, and D appear to be involved in the specificity of the receptor-binding interaction. Since M-CSFβ has intrachain disulfide bonds involving cysteines 157 and/or 159, the C-terminal region of M-CSF likely extends from the "rear" of the structure, providing a variable-length "tether" for membrane-bound forms of M-CSF. Thus, the "front" or receptor-binding region of M-CSF is on the opposite side of the molecules, consisting of solvent-accessible residues in or near helices A, C, and D, including residues from about 6 to 26, 71 to 90, and 110 to 130, respectively, of native M-CSF. Altering solvent accessible residues in these regions by site directed mutagenesis to increase or decrease side-chain interactions with the receptor may generate M-CSF agonists or antagonists. Residues having a solvent accessible surface area of greater than about 0.25 and preferably greater than about 0.4 are preferred based on normalization of the surface area of the amino acid accessible when in the trypeptide gly-x-gly (Kabsch, W. et al., Biopolymers 22:2577 (1983)). Preferably residues are chosen which do not interact with other parts of the protein such as the dimer interface in order to maintain the relative orientation of monomers and to avoid disturbing the process of protein folding. An optional additional consideration is selecting residues not conserved between human and mouse M-CSF, which does not recognize the human M-CSF receptor. Candidate amino acids are preferably selected for substitution with non-conservative amino acids, so as to disrupt hydrogen bonding and/or hydrophobic interactions with MCSF-R residues. For example, changing one or more histidines to non-hydrogen-donor amino acids of similar size may create an M-CSF with altered receptor binding ability. Preferred amino acids for substitution include but are not limited to: H15; Q79; R86; E115; E41; K93; D99; L55; S18; Q20; I75; V78; L85; D69; N70; H9; N63; and T34. M-CSF residues important in receptor signaling are believed to be composed of discontinuous regions of M-CSF. To minimize the likelihood of antibody formation to potentially administered M-CSF-based proteinaceous drugs, it is desirable to retain the solvent-accessible parental M-CSF residues (to resemble the native molecule) whenever possible.

Mutagenesis of amino acids H15 and H9 in the N-terminal/A helix region resulted in muteins with significantly lower biological activity and significantly lower MCSF-R binding ability. These results indicated that the reduced biological activity was due to decreased receptor binding affinity; thus, these histidine amino acids represent contacts that are important for M-CSF receptor binding affinity and should be left unchanged if full receptor-binding ability is desired. Nearby solvent accessible residues such as Y6 and S13 and others may also represent M-CSF receptor contact residues. A double mutant of M-CSF (Q20A, V78K) was constructed to test the importance of solvent accessible residues in the central portion of helices A and C. This double mutein had slightly lower (8-10 fold) biological activity and correspondingly lower receptor-binding activity. Mutagenesis of residues Q17, R21, E115 and E119 changed side chain properties of solvent-accessible amino acids in the areas of interest but did not affect biological specific activity, suggesting that these residues need not be altered in muteins designed to have antagonist activity.

In one embodiment, the disclosure contemplates M-CSF muteins in which residues of helices A and/or C and/or D involved in receptor-binding (for example, amino acids 6 to 26, 71 to 90 and/or 110 to 130) have been mutated non-conservatively. Such muteins preferably retain at least 65%, 70%, 75%, 80%, 85% or 90% similarity (i.e. amino acids that are identical or have similar properties) to the native sequence within helices A, C or D, but have higher similarity to the native sequence in the remainder of the polypeptide, e.g. , at least 95%, 98% or 99% similarity. In addition, residues that support the three-dimensional confirmation of the receptor-binding site may be mutated non-conservatively.

In another embodiment, the M-CSF mutein is a monomeric form of M-CSF. The dimeric form of M-CSF is the biologically active form, and monomeric forms of M-CSF are generally not active. Disulfide bonding of the monomers appears to occur through the Cys31-Cys31 interchain linkage. Thus, it is contemplated that monomeric forms of M-CSF may be suitable for use as antagonists. Such forms include muteins comprising cysteine deletions and/or cysteine replacements (e.g., cysteine to alanine substitutions) of Cys31 and/or other cysteines, or muteins in which the cysteine(s), particularly Cys31, have been chemically modified so that they are not available for disulfide bonding.

In yet another embodiment, the M-CSF mutein comprises one or more of helices A, C or D, or portions thereof involved in receptor-binding, alone or fused to other polypeptides that allow display of the fragments in proper three-dimensional conformation.

Muteins containing any desired conservative and/or non-conservative muteins are readily prepared using techniques well known in the art, including recombinant production or chemical synthesis.

Conservative substitutions, particularly substitutions outside of regions directly involved in ligand-receptor binding, are not expected to significantly change the binding properties of the M-CSF muteins (or M-CSFR muteins). Amino acids can be classified according to physical properties and contribution to secondary and tertiary protein structure. A conservative substitution is recognized in the art as a substitution of one amino acid for another amino acid that has similar properties. Exemplary conservative substitutions are set out in Table 2 (from WO 97/09433, page 10, published March 13, 1997 (PCT/GB96/02197, filed 9/6/96), immediately below.

**Table 2**

| **Conservative Substitutions I** | |
|---|---|
| SIDE CHAIN | |
| CHARACTERISTIC | AMINO ACID |
| Aliphatic | |
| Non-polar | GA P I L V |
| Polar-uncharged | C S T M N Q |
| Polar-charged | D E K R |
| Aromatic | H F W Y |
| Other | N Q D E |

Alternatively, conservative amino acids can be grouped as described in Lehninger, (Biochemistry, Second Edition; Worth Publishers, Inc. NY:NY (1975), pp.71-77) as set out in Table 3, immediately below.

**Table 3**

| **Conservative Substitutions II** | |
|---|---|
| SIDE CHAIN | |
| CHARACTERISTIC | AMINO ACID |
| Non-polar (hydrophobic) | |
| A. Aliphatic: | A L I V P |
| B. Aromatic: | F W |
| C. Sulfur-containing: | M |
| D. Borderline: | G |
| Uncharged-polar | |
| A. Hydroxyl: | S T Y |
| B. Amides: | N Q |
| C. Sulfhydryl: | C |
| D. Borderline: | G |
| Positively Charged (Basic): | K R H |
| Negatively Charged (Acidic): | D E |

As still an another alternative, exemplary conservative substitutions are set out in Table 4, immediately below.

| | **Table 4** |
|---|---|
| | **Conservative Substitutions III** |
| Original Residue | Exemplary Substitution |
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

The availability of a DNA sequence encoding M-CSF permits the use of various expression systems to produce the desired polypeptides. Construction of expression vectors and recombinant production from the appropriate DNA sequences are performed by methods well known in the art. These techniques and various other techniques are generally performed according to Sambrook et al., Molecular Cloning--A Laboratory Manual, Cold Spring Harbor Laboratory, Cold Spring Harbor, N.Y. (1989), and Kriegler, M., Gene Transfer and Expression, A Laboratory Manual, Stockton Press, New York (1990).

Certain modifications to the primary sequence of M-CSF can be made by deletion, addition, or alteration of the amino acids encoded by the DNA sequence without destroying the desired structure (e.g., the receptor binding ability of M-CSF) in accordance with well-known recombinant DNA techniques. Further, a skilled artisan will appreciate that individual amino acids may be substituted or modified by oxidation, reduction or other modification, and the polypeptide may be cleaved to obtain fragments that retain the active binding site and structural information. Such substitutions and alterations result in polypeptides having an amino acid sequence which falls within the definition of polypeptide "having substantially the same amino acid sequence as the mature M-CSFα (SEQ ID NO: 2), M-CSFβ (SEQ ID NO: 4), and M-CSFγ (SEQ ID NO: 6)polypeptides."

Polypeptides may be synthesized using standard solution phase or solid phase peptide synthesis techniques known in the art. In solution phase synthesis, a wide variety of coupling methods and protecting groups may be used (see Gross and Meienhofer, eds., "The Peptides: Analysis, Synthesis, Biology," Vol. 1-4 (Academic Press, 1979); Bodansky and Bodansky, "The Practice of Peptide Synthesis," 2d ed. (Springer Verlag, 1994)). In addition, intermediate purification and linear scale up are possible. Those of ordinary skill in the art will appreciate that solution synthesis requires consideration of main chain and side chain protecting groups and activation method as well as segment selection to minimize racemization.

Solid-phase peptide synthesis may generally be performed according to the method of Merrifield et al., J. Am. Chem. Soc. 85:2149, 1963, which involves assembling a linear peptide chain on a resin support using protected amino acids. Solid phase peptide synthesis typically utilizes either the Boc or Fmoc strategy. The Boc strategy uses a 1% cross-linked polystyrene resin. The standard protecting group for α-amino functions is the tert-butyloxycarbonyl (Boc) group. This group can be removed with dilute solutions of strong acids such as 25% trifluoroacetic acid (TFA). The next Boc-amino acid is typically coupled to the amino acyl resin using dicyclohexylcarbodiimide (DCC). Following completion of the assembly, the peptide-resin is treated with anhydrous HF to cleave the benzyl ester link and liberate the free peptide. Side-chain functional groups are usually blocked during synthesis by benzyl-derived blocking groups, which are also cleaved by HF. The free peptide is then extracted from the resin with a suitable solvent, purified and characterized. Newly synthesized peptides can be purified, for example, by gel filtration, HPLC, partition chromatography and/or ion-exchange chromatography, and may be characterized by, for example, mass spectrometry or amino acid sequence analysis. In the Boc strategy, C-terminal amidated peptides can be obtained using benzhydrylamine or methylbenzhydrylamine resins, which yield peptide amides directly upon cleavage with HF. An alternative approach using 9-fluorenylmethyloxycarbonyl (Fmoc) uses different reagents which allow the side-chain protecting groups and the peptide-resin link to be completely stable to the secondary amines used for cleaving the N-α-Fmoc group. The side-chain protection and the peptide-resin link are cleaved by mild acidolysis. The repeated contact with base makes the Merrifield resin unsuitable for Fmoc chemistry, and p-alkoxybenzyl esters linked to the resin are generally used. Deprotection and cleavage are generally accomplished using TFA. Acetylation of the N-terminal can be accomplished by reacting the final peptide with acetic anhydride before cleavage from the resin. C-amidation is accomplished using an appropriate resin such as methylbenzhydrylamine resin using the Boc technology.

In general, modifications of the genes encoding the M-CSF polypeptide are readily accomplished by a variety of well-known techniques, such as site-directed mutagenesis (see, Gillman and Smith, Gene 8:81-97 (1979) and Roberts, S. et al., Nature 328:731-734 (1987) and U.S. Pat. No. 5,032,676). Most modifications are evaluated by screening in a suitable assay for the desired characteristic. For instance, a change in the M-CSF receptor-binding character of the polypeptide can be detected by competitive assays with an appropriate reference polypeptides or by the bioassays described in U.S. Pat. No. 4,847,201.

Insertional variants of the present invention are those in which one or more amino acid residues are introduced into a predetermined site in the M-CSF. For instance, insertional variants can be fusions of heterologous proteins or polypeptides to the amino or carboxyl terminus of the subunits. Substitutional variants are those in which at least one residue has been removed and a different residue inserted in its place. Non-natural amino acids (i.e., amino acids not normally found in native proteins), as well as isosteric analogs (amino acid or otherwise) are also suitable for use in this invention. Examples of suitable substitutions are well known in the art, such as the Glu->Asp, Ser->Cys, and Cys->Ser, His->alanine for example. Another class of variants are deletional variants, which are characterized by the removal of one or more amino acid residues from the M-CSF.

Other variants of the present invention may be produced by chemically modifying amino acids of the native protein (e.g., diethylpyrocarbonate treatment which modifies histidine residues). Preferred or chemical modifications which are specific for certain amino acid side chains. Specificity may also be achieved by blocking other side chains with antibodies directed to the side chains to be protected. Chemical modification includes such reactions as oxidation, reduction, amidation, deamidation, or substitution of bulky groups such as polysaccharides or polyethylene glycol (see e.g., U.S. Pat. No. 4,179,337 and WO91/21029).

Exemplary modifications include the modification of lysinyl and amino terminal residues by reaction with succinic or other carboxylic acid anhydrides. Modification with these agents has the effect of reversing the charge of the lysinyl residues. Other suitable reagents for modifying amino-containing residues include imidoesters such as methyl picolinimidate; pyridoxal phosphate; pyridoxal chloroborohydride; trinitrobenzenesulfonic acid; O-methylisourea, 2,4-pentanedione; and transaminase-catalyzed reaction with glyoxylate, and N-hydroxysuccinamide esters of polyethylenene glycol or other bulky substitutions.

Arginyl residues may be modified by reaction with a number of reagents, including phenylglyoxal, 2,3-butanedione, 1,2-cyclohexanedione, and ninhydrin. Modification of arginine residues requires that the reaction be performed in alkaline conditions because of the high pKₐ of the guanidine functional group. Furthermore, these reagents may react with the groups of lysine as well as the arginine epsilon-amino group.

Tyrosyl residues may also be modified with particular interest in introducing spectral labels into tyrosyl residues by reaction with aromatic diazonium compounds or tetranitromethane are used to form O-acetyl tyrosyl species and 3-nitro derivatives, respectively. Tyrosyl residues may also be iodinated using¹²⁵ I or ¹³¹I to prepare labeled proteins for use in radioimmunoassay.

Carboxyl side groups (aspartyl or glutamyl) may be selectively modified by reaction with carbodiimides (R--N.dbd.C.dbd.N--R.sup.1), where R and R₁ are different alkyl groups, such as 1-cyclohexyl-3-(2-morpholinyl-4-ethyl)carbodiimide or 1-ethyl-3-(4-azonia-4,4-dimethylpentyl)carbodiimide. Furthermore, aspartyl and glutamyl residues are converted to asparaginyl and glutaminyl residues by reaction with ammonium ions.

Conversely, glutaminyl and asparaginyl residues may be deamidated to the corresponding glutamyl and aspartyl residues, respectively, under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T. E. Creighton, Proteins: Structure and Molecular Properties, W. H. Freeman & Co., San Francisco, pp. 79-86 (1983)), acetylation of the N-terminal amine, and amidation of any C-terminal carboxyl group.

A number of methods can be used to determine the similarity of M-CSF muteins to the native M-CSF protein. For example, percent homology is calculated as the percentage of amino acid residues in the smaller of two sequences which align with identical amino acid residue in the sequence being compared, when four gaps in a length of 100 amino acids may be introduced to maximize alignment (Dayhoff, in Atlas of Protein Sequence and Structure, Vol. 5, p. 124, National Biochemical Research Foundation, Washington, D.C. (1972)). Sequence alignment of polypeptides for purposes of sequence comparison also can be done using a variety of multiple alignment servers, most of which are presently available on the Internet, e.g., Clustal W, MAP, PIMA, Block Maker, MSA, MEME, and Match-Box. Preferably Clustal W (Higgins et al., Gene (1988) 73:237-244; Higgins et al., Meth. Enzymol.(1996) 266:383-402) is employed for sequence alignment of polypeptides (and also, polynucleotides). Similarly, the program BLASTP compares an amino acid query sequence against a protein database, and TBLASTN compares a protein query sequence against a nucleotide sequence database dynamically translated in all six reading frames (both strands), and can be employed in the invention. Determinations of whether two amino acid sequences are substantially homologous (i.e., similar or identical) can also be based on FASTA searches in accordance with Pearson et al., Proc. Natl. Acad. Sci. USA 85:2444-2448 (1988).

In particular, preferred methods to determine identity and/or similarity are designed to give the largest match between the sequences tested. Methods to determine identity and similarity are codified in publicly available computer programs (e.g., such as those previously described). Preferred computer program methods to determine identity and similarity between two sequences include, but are not limited to, the GCG program package, including GAP (Devereux et al., Nucleic Acids Research (1984) 12(1):387; Genetics Computer Group, University of Wisconsin, Madison, WI), BLASTP, BLASTN, and FASTA (Altschul et al., J. Molec. Biol. (1990) 215:403-410). The BLAST X program is publicly available from the National Center for Biotechnology Information (NCBI) and other sources (Altschul et al., BLAST Manual, NCB NLM NIH Bethesda, MD 20894; Altschul et al., J. Mol. Biol. (1990) 215:403-410). The well known Smith Waterman algorithm may also be used to determine identity. In comparing polynucleotide sequences using the GAP program, the following default parameters are preferred: comparison matrix: match = +10, mismatch = 0, with a gap penalty of 50 and a gap length penalty of 3 (Needleman et al., J. Mol Biol. (1970) 48:443-453).

The relatedness of proteins can also be characterized through the relatedness of their encoding nucleic acids. Methods to determine identity and/or similarity of polynucleotide sequences are described above. In addition, methods to determine similarity of polynucleotide sequences through testing their ability to hybridize under moderately or highly stringent conditions may be determined as follows. Exemplary moderately stringent hybridization conditions are as follows: hybridization at 42°C in a hybridization solution comprising 50% formamide, 1% SDS, 1 M NaCl, 10% Dextran sulfate, and washing twice for 30 minutes at 60°C in a wash solution comprising 0.1x SSC and 1% SDS. Highly stringent conditions include washes at 68°C in a wash solution comprising 0.1x SSC and 1% SDS. It is understood in the art that conditions of equivalent stringency can be achieved through variation of temperature and buffer, or salt concentration as described in the art (Ausubel, et al. (Eds.), Protocols in Molecular Biology, John Wiley & Sons (1994), pp. 6.0.3 to 6.4.10). Modifications in hybridization conditions can be empirically determined or precisely calculated based on the length and the percentage of guanosine/cytosine (GC) base pairing of the probe. The hybridization conditions can be calculated as described in Sambrook et al., (Eds.), Molecular Cloning: A Laboratory Manual, Cold Spring Harbor Laboratory Press: Cold Spring Harbor, New York (1989), pp. 9.47 to 9.51.

Exemplary M-CSFR fragments according to the invention may comprise one or more, or two or more, of domains involved in M-CSF/receptor binding (believed to be domains 1, 2 and 3). Preferred M-CSFR fragments comprises all three of domains 1, 2 and 3 of M-CSFR. Additional mutations and/or modifications to such fragments or to the entire extracellular domain of M-CSFR are contemplated and may be produced as described above in the section on M-CSF muteins.

### M-CSFR Antibodies

The invention also provides antibodies to M-CSFR that may be used as MCSF antagonists according to the methods of the invention.

M-CSFR (SEQ ID NO: 8) is a membrane spanning molecule with five extracellular immunoglobulin-like domains (of which domains 1-3 are believed to be involved in ligand-receptor binding), a transmembrane domain and an intracellular interrupted Src related tyrosine kinase domain. With reference to SEQ ID NO: 8, the aforementioned domains are located as follows: Ig domain 1: amino acids 27-102; Ig domain 2: amino acids 112-196; Ig domain 3: amino acids 215-285; Ig domain 4: amino acids 308-399; Ig domain 5: amino acids 410-492; transmembrane domain: amino acids 515-537; and kinase domain: amino acids 582-910. A "typical" immunoglobulin-like domain contains a loop structure usually anchored by a disulfide bond between two cysteines at the extremity of each loop. In M-CSF-R, these cysteines forming the Ig-like loops are at the following amino acid positions: Domain 1: 42, 84; Domain 2: 127, 177; Domain 3: 224, 278; Domain 4: no cysteins involved; Domain 5: 419, 485.

The intact extracellular portion of M-CSFR or any fragment thereof that retains antigenicity, for example, one or more of the Ig-like loops, may be used to raise antibodies that would bind to the native receptor. Polyclonal, monoclonal, chimeric, CDR grafted, humanized, fully human antibodies and antigen-binding fragments thereof may be prepared as described above for antibodies to M-CSF. The antibody products may be screened for activity as an MCSF antagonist and for suitability in the treatment methods of the invention using assays as described in the section entitled "Screening Methods" herein or using any suitable assays known in the art.

### Soluble M-CSFR

The biological function of M-CSF in vivo takes place through binding and activation of the M-CSF receptor, also referred to as the *c*-*fms* gene product. Recombinant human soluble M-CSF receptor (rhsM-CSFR), representing amino acids 20 to 511 of SEQ ID NO: 8 (Coussens, L et al., Nature, 320:277 (1986)) was used as an in vitro assay reagent to test the receptor-binding ability of M-CSF proteins. To generate a soluble form of the transmembrane receptor, only the extracellular domain of the human M-CSF receptor was expressed in a baculovirus/insect cell recombinant expression system. In order to purify the soluble receptor without adversely effecting tertiary or quaternary structure, non-denaturing chromatographic methods were chosen, as described below. Other choices exist for the purification of the recombinant receptor. Affinity chromatography may be employed when either a suitable antibody to or ligand for the receptor are available. Alternatively, "tags" may be added to the C-terminus of the recombinant receptor, i.e., KT3 antibody recognition sequence, and purified by an anti-tag antibody, i.e., KT3, column, for use in affinity chromatography. In expression systems in which the rhsM-CSFR is glycosylated, lectin chromatography can be used to enrich for specific glycoproteins.

One or more of the aformentioend Ig-like loops within the extracellular domain of the receptor may be sufficient to inhibit interaction between M-CSF and M-CSFR. Thus fragments of the extracellular domain of M-CSFR and muteins thereof may be easily prepared using recombinant or chemical synthetic means well known in the art. The products may be screened for activity as an MCSF antagonist and for suitability in the treatment methods of the invention using assays as described in the section entitled "Screening Methods" herein or using any suitable assays known in the art.

### Gene Therapy

Delivery of a therapeutic protein to appropriate cells can be effected via gene therapy ex vivo, in situ, or in vivo by use of any suitable approach known in the art, including by use of physical DNA transfer methods (e.g., liposomes or chemical treatments) or by use of viral vectors (e.g., adenovirus, adeno-associated virus, or a retrovirus). For example, for in vivo therapy, a nucleic acid encoding the desired protein, either alone or in conjunction with a vector, liposome, or precipitate may be injected directly into the subject, and in some embodiments, may be injected at the site where the expression of the protein compound is desired. For ex vivo treatment, the subject's cells are removed, the nucleic acid is introduced into these cells, and the modified cells are returned to the subject either directly or, for example, encapsulated within porous membranes which are implanted into the patient. See, e.g. U.S. Pat. Nos. 4,892,538 and 5,283,187. There are a variety of techniques available for introducing nucleic acids into viable cells. The techniques vary depending upon whether the nucleic acid is transferred into cultured cells in vitro, or in vivo in the cells of the intended host. Techniques suitable for the transfer of nucleic acid into mammalian cells in vitro include the use of liposomes, electroporation, microinjection, cell fusion, DEAE-dextran, and calcium phosphate precipitation. A commonly used vector for ex vivo delivery of a nucleic acid is a retrovirus.

Other in vivo nucleic acid transfer techniques include transfection with viral vectors (such as adenovirus, Herpes simplex I virus, or adeno-associated virus) and lipid-based systems. The nucleic acid and transfection agent are optionally associated with a microparticle. Exemplary transfection agents include calcium phosphate or calcium chloride co-precipitation, DEAE-dextran-mediated transfection, quaternary ammonium amphiphile DOTMA ((dioleoyloxypropyl) trimethylammonium bromide, commercialized as Lipofectin by GIBCO-BRL))(Felgner et al, (1987) Proc. Natl. Acad. Sci. USA 84, 7413-7417; Malone et al. (1989) Proc. Natl Acad. Sci. USA 86 6077-6081); lipophilic glutamate diesters with pendent trimethylammonium heads (Ito et al. (1990) Biochem. Biophys. Acta 1023, 124-132); the metabolizable parent lipids such as the cationic lipid dioctadecylamido glycylspermine (DOGS, Transfectam, Promega) and dipalmitoylphosphatidyl ethanolamylspermine (DPPES)(J. P. Behr (1986) Tetrahedron Lett. 27, 5861-5864; J. P. Behr et al. (1989) Proc. Natl. Acad. Sci. USA 86, 6982-6986); metabolizable quaternary ammonium salts (DOTB, N-(1-[2,3-dioleoyloxy]propyl)-N,N,N-trimethylammonium methylsulfate (DOTAP)(Boehringer Mannheim), polyethyleneimine (PEI), dioleoyl esters, ChoTB, ChoSC, DOSC)(Leventis et al. (1990) Biochim. Inter. 22, 235-241); 3beta[N-(N', N'-dimethylaminoethane)-carbamoyl]cholesterol (DC-Chol), dioleoylphosphatidyl ethanolamine (DOPE)/3beta[N-(N',N'-dimethylaminoethane)-carbamoyl]cholesterolDC-Chol in one to one mixtures (Gao et al., (1991) Biochim. Biophys. Acta 1065, 8-14), spermine, spermidine, lipopolyamines (Behr et al., Bioconjugate Chem, 1994, 5: 382-389), lipophilic polylysines (LPLL) (Zhou et al., (1991) Biochim. Biophys. Acta 939, 8-18), [[(1,1,3,3-tetramethylbutyl)cre- soxy]ethoxy]ethyl]dimethylbe nzylammonium hydroxide (DEBDA hydroxide) with excess phosphatidylcholine/cholesterol (Ballas et al., (1988) Biochim. Biophys. Acta 939, 8-18), cetyltrimethylammonium bromide (CTAB)/DOPE mixtures (Pinnaduwage et al, (1989) Biochim. Biophys. Acta 985, 33-37), lipophilic diester of glutamic acid (TMAG) with DOPE, CTAB, DEBDA, didodecylammonium bromide (DDAB), and stearylamine in admixture with phosphatidylethanolamine (Rose et al., (1991) Biotechnique 10, 520-525), DDAB/DOPE (TransfectACE, GIBCO BRL), and oligogalactose bearing lipids. Exemplary transfection enhancer agents that increase the efficiency of transfer include, for example, DEAE-dextran, polybrene, lysosome-disruptive peptide (Ohmori N I et al, Biochem Biophys Res Commun Jun. 27, 1997;235(3):726-9), chondroitan-based proteoglycans, sulfated proteoglycans, polyethylenimine, polylysine (Pollard H et al. J Biol Chem, 1998 273 (13):7507-11), integrin-binding peptide CYGGRGDTP, linear dextran nonasaccharide, glycerol, cholesteryl groups tethered at the 3'-terminal internucleoside link of an oligonucleotide (Letsinger, R. L. 1989 Proc Natl Acad Sci USA 86: (17):6553-6), lysophosphatide, lysophosphatidylcholine, lysophosphatidylethanolamine, and 1-oleoyl lysophosphatidylcho line.

In some situations it may be desirable to deliver the nucleic acid with an agent that directs the nucleic acid-containing vector to target cells. Such "targeting" molecules include antibodies specific for a cell-surface membrane protein on the target cell, or a ligand for a receptor on the target cell. Where liposomes are employed, proteins which bind to a cell-surface membrane protein associated with endocytosis may be used for targeting and/or to facilitate uptake. Examples of such proteins include capsid proteins and fragments thereof tropic for a particular cell type, antibodies for proteins which undergo internalization in cycling, and proteins that target intracellular localization and enhance intracellular half-life. In other embodiments, receptor-mediated endocytosis can be used. Such methods are described, for example, in Wu et al., 1987 or Wagner et al., 1990. For review of the currently known gene marking and gene therapy protocols, see Anderson 1992. See also WO 93/25673 and the references cited therein. For additional reviews of gene therapy technology, see Friedmann, Science, 244: 1275-1281 (1989); Anderson, Nature, supplement to vol. 392, no 6679, pp. 25-30 (1998); Verma, Scientific American: 68-84 (1990); and Miller, Nature, 357: 455460 (1992).Antisense

Antisense compounds and methods of using them are also provided by the present invention. The level of M-CSF or M-CSFR activity may be reduced by using well-known antisense, gene "knock-out," ribozyme and/or triple helix methods to decrease the level gene expression. Techniques for the production and use of such molecules are well known to those of skill in the art.

Antisense compounds may block the translation of mRNA by hybridizing to targeted mRNA and preventing protein translation and include oligonucleotides complementary to the target gene mRNA. Targeting may be to the coding region or more preferably to transcribed, untranslated region(s). Absolute complementarity is not required, only sufficient complementarity to be able to hybridize with the endogenous RNA or DNA and forming a stable duplex or triplex. The ability to hybridize depends on both the degree of complementarity and the length of the antisense nucleic acid. Oligonucleotides complementary to non-coding regions of the gene of interest can also be used in an antisense approach to inhibit translation of endogenous mRNA. Antisense nucleic acids are preferably oligonucleotides ranging from 6 to about 50 nucleotides in length. In specific aspects, the oligonucleotide is at least 10 nucleotides, at least 17 nucleotides, or at least 20 nucleotides.

In vitro studies are first performed to quantitate the ability of the candidate antisense oligonucleotide to inhibit target gene expression. It is preferred that these studies utilize controls that distinguish between antisense gene inhibition and nonspecific biological effects of oligonucleotides. It is also preferred that these studies compare levels of the target RNA or protein with that of an internal control RNA or protein.

The oligonucleotides can be DNA or RNA or chimeric mixtures or derivatives or modified versions thereof, and may be single-stranded or double-stranded (e.g., RNAi). The oligonucleotide can be modified at the base moiety, sugar moiety, or phosphate backbone, for example, to improve stability of the molecule or hybridization.. See, for example, WO 03/088921; Dean, Curr Opin Biotechnol. 12(6):622-5, 2001; Geary et al, Curr Opin Investig Drugs. 2(4):562-573, 2001. The oligonucleotide may be conjugated to other moieties, including peptides (e.g., for targeting host cells in vivo), or agents facilitating transport across the cell membrane (see, e.g., Letsinger, et al., 1989, Proc. Natl. Acad. Sci. U.S.A. 86:6553-6556; Lemaitre, et al., 1987, Proc. Natl. Acad. Sci. U.S.A. 84:648-652; PCT Publication No. WO88/09810, published Dec. 15, 1988) or the blood-brain barrier (see, e.g., PCT Publication No. WO89/10134, published Apr. 25, 1988), hybridization-triggered cleavage agents (see, e.g., Krol et al., 1988, BioTechniques 6:958-976) or intercalating agents (see, e.g., Zon, 1988, Pharm. Res. 5:539-549). The antisense compound may also be an alpha-anomeric oligonucleotide which forms specific double-stranded hybrids with complementary RNA that run parallel to each other (Gautier, et al., 1987, Nucl. Acids Res. 15:6625-6641). The oligonucleotide may be a 2'-( )-methylribonucleotide (Inoue, et al., 1987, Nucl. Acids Res. 15:6131-6148), or a chimeric RNA-DNA analogue (Inoue, et al., 1987, FEBS Lett. 215:327-330).

Antisense molecules should be delivered to cells that express the target gene in vivo. A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically. In one approach, intracellular concentrations of the antisense sufficient to suppress translation of endogenous mRNAs may be accomplished with a recombinant DNA construct in which the antisense oligonucleotide is placed under the control of a strong promoter. The use of such a construct to transfect target cells in the patient will result in the transcription of sufficient amounts of single stranded RNAs that will form complementary base pairs with the endogenous target gene transcripts and thereby prevent translation of the target gene mRNA. For example, a vector can be introduced e.g., such that it is taken up by a cell and directs the transcription of an antisense RNA. Such a vector can remain episomal or become chromosomally integrated, as long as it can be transcribed to produce the desired antisense RNA.

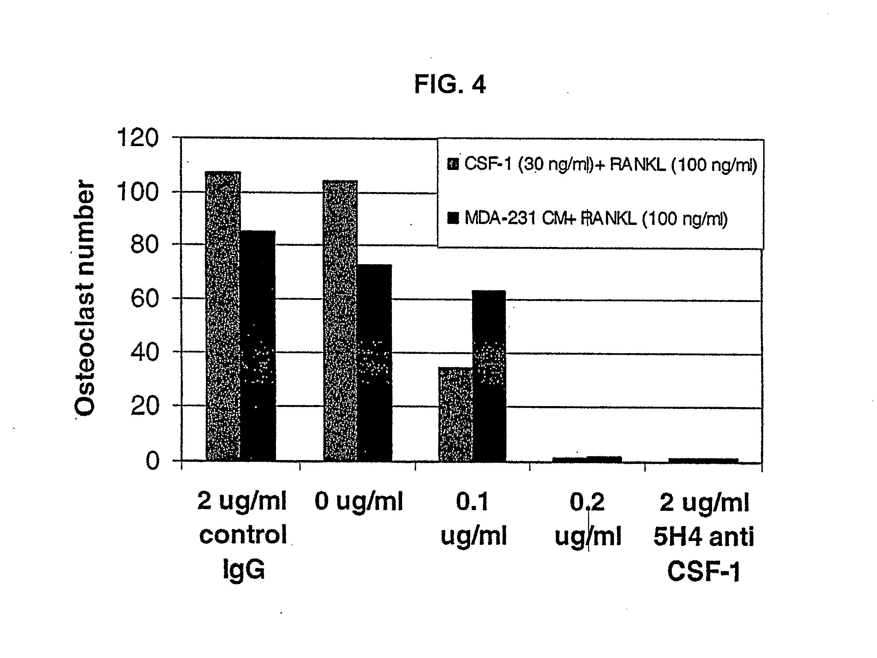

Ribozyme molecules designed to catalytically cleave target gene mRNA transcripts can also be used to prevent translation of target gene mRNA and, therefore, expression of target gene product. (See, e.g., PCT International Publication WO90/11364, published Oct. 4, 1990; Sarver, et al., 1990, Science 247, 1222-1225). Ribozymes are enzymatic RNA molecules capable of catalyzing the specific cleavage of RNA. (For a review, see Rossi, 1994, Current Biology 4:469-471). The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, preferably at a site closer to the 5' end, and must include the well known catalytic sequence responsible for mRNA cleavage. For this sequence, see, e.g., U.S. Pat. No. 5,093,246. Hammerhead ribozymes, which cleave mRNAs at locations dictated by specific flanking regions that form complementary base pairs with the target mRNA, may also be used. The construction and production of hammerhead ribozymes is known in the art and is described more fully in Myers, 1995, Molecular Biology and Biotechnology: A Comprehensive Desk Reference, VCH Publishers, New York, (see especially FIG. 4, page 833) and in Haseloff and Gerlach, 1988, Nature, 334:585-591. RNA endoribonucleases (also known as "Cech-type ribozymes"), may also be used. Such ribozymes such as the one that occurs naturally in Tetrahymena thermophila (known as the IVS, or L-19 IVS RNA) have been described in Zaug, et al., 1984, Science, 224:574-578; Zaug and Cech, 1986, Science, 231:470-475; Zaug, et al., 1986, Nature, 324:429-433; published International patent application No. WO 88/04300; and Been and Cech, 1986, Cell, 47:207-216. The Cech-type ribozymes have an eight base pair active site which hybridizes to a target RNA sequence. Ribozymes can also be composed of modified oligonucleotides (e.g., for improved stability, targeting, etc.) and should be delivered to cells that express the target gene in vivo. Because ribozymes unlike antisense molecules, are catalytic, a lower intracellular concentration is required for efficiency.

Endogenous target gene expression can also be reduced by inactivating or "knocking out" the target gene or its promoter using targeted homologous recombination (e.g., see Smithies, et al., 1985, Nature 317:230-234; Thomas and Capecchi, 1987, Cell 51:503-512; Thompson, et al., 1989, Cell 5:313-321). For example, a mutant, non-functional target gene (or a completely unrelated DNA sequence) flanked by DNA homologous to the endogenous target gene (either the coding regions or regulatory regions of the target gene) can be used, with or without a selectable marker and/or a negative selectable marker, to transfect cells that express the target gene in vivo. Insertion of the DNA construct, via targeted homologous recombination, results in inactivation of the target gene.

Alternatively, endogenous target gene expression can be reduced by targeting deoxyribonucleotide sequences complementary to the regulatory region of the target gene (i.e., the target gene promoter and/or enhancers) to form triple helical structures that prevent transcription of the target gene in target cells in the body. (See generally, Helene, 1991, Anticancer Drug Des., 6(6):569-584; Helene, et al., 1992, Ann. N.Y. Acad. Sci., 660:27-36; and Maher, 1992, Bioassays 14(12):807-815).

Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC.sup.+triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex. Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

If antisense, ribozyme or triple helix therapy reduces target gene expression to levels that are undesirably low, replacement protein may be administered. Alternatively, gene therapy to increase gene expression may be carried out using modified nucleic acids that do not contain sequences susceptible to whatever antisense, ribozyme, or triple helix treatments are being utilized.

Anti-sense RNA and DNA, ribozyme and triple helix molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by in vitro and in vivo transcription of DNA sequences encoding the therapeutic RNA molecule.

Chemical synthetic methods include use of a commercially available automated DNA synthesizer; phosphorothioate oligonucleotides may be synthesized by the method of Stein, et al. (1988, Nucl. Acids Res. 16:3209), and methylphosphonate oligonucleotides can be prepared by use of controlled pore glass polymer supports (Sarin, et al., 1988, Proc. Natl. Acad. Sci. U.S.A. 85:7448-7451).

### Peptides and Small Molecules

As used herein, the term "protein" includes proteins, oligopeptides, polypeptides, peptides and the like. Additionally, the term protein may also refer to fragments, multimers or aggregates of intact molecules and/or fragments. Proteins may be naturally occurring or may be produced via recombinant DNA means or by chemical and/or enzymatic synthesis. See, e.g., Sambrook et al., Molecular Cloning: A Laboratory Manual, Cold Spring Harbor Laboratories (3rd ed. 2001).

The term "peptides" is used to refer to relatively short chains of amino acids, preferably between 6 and 100 amino acids in length.

Peptides can be screened for desired binding activity (e.g., binding to M-CSF or M-CSFR) or biological activity using phage display techniques (See, for example, Scott et al. Science 249: 386 (1990); Devlin et al., Science 249: 404 (1990); U.S. Patent No. 5,223,409, issued June 29, 1993; U.S. Patent No. 5,733,731, issued March 31, 1998; U.S. Patent No. 5,498,530, issued March 12, 1996; U.S. Patent No. 5,432,018, issued July 11, 1995; U.S. Patent No. 5,338,665, issued August 16, 1994; U.S. Patent No. 5,922,545, issued July 13, 1999; WO 96/40987, published December 19, 1996; and WO 98/15833, published April 16, 1998). In such libraries, peptide sequences are displayed by fusion with coat proteins of filamentous phage. Typically, the displayed peptides are affinity-eluted against an antibody-immobilized extracellular domain of a receptor. The retained phages may be enriched by successive rounds of affinity purification and re-propagation. The best binding peptides may be sequenced to identify key residues within one or more structurally related families of peptides.

Peptides can also be screened using other methods, such as generating libraries of peptides fused to the carboxyl terminus of the lac repressor and expressed in *E. coli,* or displaying peptides on the outer membrane of *E. coli* by fusion with a peptidoglycan-associated lipoprotein (PAL). In another method, translation of random RNA is halted prior to ribosome release, resulting in a library of polypeptides with their associated RNA still attached. Other methods employ chemical linkage of peptides to RNA (See, for example, Roberts and Szostak, Proc Natl Acad Sci USA, 94: 12297-303 (1997)). Chemically derived peptide libraries have been developed in which peptides are immobilized on stable, non-biological materials, such as polyethylene rods or solvent-permeable resins. Other chemically derived peptide libraries uses photolithography to scan peptides immobilized on glass slides. Chemical-peptide screening may be advantageous in that it allows use of D-amino acids and other unnatural analogues, as well as non-peptide elements. Biological and chemical methods are reviewed in Wells and Lowman, Curr Opin Biotechnol 3: 355-62 (1992).

Peptides may be further modified by mutagenesis at the DNA level. Mutagenesis libraries may be created and screened to further optimize the sequence of the best binders (Lowman, Ann Rev Biophys Biomol Struct 26: 401-24 (1997)). Structural analysis of protein-protein interaction may also be used to suggest peptides that mimic the binding activity of large protein ligands. In such an analysis, the crystal structure may suggest the identity and relative orientation of critical residues of the large protein ligand, from which a peptide may be designed (See, e.g., Takasaki et al., Nature Biotech 15: 1266-70 (1997)).

Random peptides or peptides derived from antigen-binding CDRs may be fused in a linear fashion or as part of a three-dimensional scaffold, e.g. an antibody or portion thereof such as a constant region, to create new macromolecules that bind the desired antigen. Multiple copies of the same peptide or different peptides may be included.

As used herein, the term "peptidomimetic" is a non-peptide compound that comprises an assembly of amino acid side chains, or pharmacophores, or suitable derivatives thereof, that are supported on a scaffold such that the spatial orientation of the pharmacophores substantially mimic the bioactive conformation of a natural peptide. For example, a peptidomimetic may lack amino acids or peptide bonds but retain the particular three-dimensional arrangement of peptide chain groups from the parent peptide that is required for binding activity. The scaffold may comprise a bicyclic, tricyclic or higher polycyclic carbon or heteroatom skeleton, or may be based on one or more ring structures (e.g., pyridine, indazole, etc.) or amide bonds. This scaffold may be linked by spacers to an acidic group (e.g. a carboxylic acid functional group) at one end and a basic group (e.g. an N-containing moiety such as amidine or guanidine) at the other end of the core. Exemplary techniques for synthesizing peptidomimetics are described in U.S. patent application no. 20030199531 published October 23, 2003, U.S. Patent Application No. 20030139348 published July 24, 2003.

In addition to antibodies and other proteins, this invention also contemplates alternative M-CSF antagonists including, but not limited to, small molecules that are also effective in treating cancer metastasis and/or bone loss associated with cancer metastasis. Such small molecules may be identified by assaying their capacity to bind to M-CSF and/or to inhibit the interaction between M-CSF and M-CSFR.

Methods for measuring the binding of M-CSF with small molecules are readily available in the art and include, for example, competition assays whereby the small molecule interferes with the interaction between M-CSF and its receptor (M-CSFR) or an anti M-CSF antibody. Alternatively, direct binding assays may be utilized to measure the interaction of a small molecule with M-CSF. By way of example, an ELISA assay may be employed whereby M-CSF is adsorbed onto an insoluble matrix such as a tissue culture plate or bead. A labeled M-CSFR or anti-M-CSF antibody is blocked from binding to M-CSF by inclusion of the small molecule of interest. Alternatively, the binding of a small molecule to M-CSF may be determined by a fluorescence activated cell sorting (FACS) assay. By this method, cells expressing M-CSF are incubated with a fluorescent tagged anti-M-CSF antibody or an anti-M-CSF antibody in the presence of a fluorescent tagged secondary antibody. Binding of a small molecule to M-CSF may be assessed by a dose dependent decrease in fluorescence bound to the M-CSF expressing cells. Similarly, direct binding of a small molecule may be assessed by labeling, e.g. radio labeling or fluorescent tagging, the small molecule, incubating with immobilized M-CSF or M-CSF expressing cells and assaying for the radioactivity or fluorescence of the bound small molecule.

### Screening Methods

Effective therapeutics depend on identifying efficacious agents devoid of significant toxicity. Compounds potentially useful in preventing or treating bone loss associated with cancer metastasis may be screened using various assays. For instance, a candidate antagonist may first be characterized in a cultured cell system to determine its ability to neutralize M-CSF in inducing osteoclastogenesis. Such a system may include the co-culture of mouse calvarial osteoblasts and spleen cells (Suda et al., Modulation of osteoclast differentiation. Endocr. Rev. 13: 66 80, 1992; Martin and Udagawa, Trends Endocrinol. Metab. 9: 6-12, 1998), the co-culture of mouse stromal cell lines (e.g., MC3T3-G2/PA6 and ST2) and mouse spleen cells (Udagawa et al., Endocrinology 125: 1805 13, 1989), and the co-culture of ST2 cells and bone marrow cells, peripheral blood mononuclear cells or alveolar macrophages (Udagawa et al., Proc. Natl. Acad. Sci. USA 87: 7260 4, 1990; Sasaki et al., Cancer Res. 58: 462 7, 1998; Mancino et al., J. Surg. Res.100: 18-24, 2001). In the absence of any M-CSF antagonist, multinucleated cells formed in such co-cultures satisfy the major criteria of osteoclasts such as tartrate resistant acid phosphatase (TRAP, a marker enzyme of osteoclasts) activity, calcitonin receptors, p60C-STC, vitronectin receptors, and the ability to form resorption pits on bone and dentine slices. The presence of an effective M-CSF antagonist inhibits the formation of such multinucleated cells.

In addition to the above co-culture systems, the ability of a candidate M-CSF antagonist in inhibiting osteoclastogenesis may be assayed in a stromal cell-free or osteoblast-free system. The M-CSF required for osteoclastogenesis may be provided by co-cultured metastatic cancer cells (e.g., MDA 231) or conditioned medium from these cancer cells (Mancino et al., J. Surg. Res. 0: 18-24, 2001) or by addition of purified M-CSF.

Efficacy of a given M-CSF antagonist in preventing or treating bone loss associated with cancer metastasis may also be tested in any of the animal bone metastasis model systems familiar to those skilled in the art. Such model systems include those involving direct injection of tumor cells into the medullary cavity of bones (Ingall, Proc. Soc. Exp. Biol. Med., 117: 819-22, 1964; Falasko, Clin. Orthop. 169: 20 7, 1982), into the rat abdominal aorta (Powles et al., Br. J. Cancer 28: 316 21, 1973), into the mouse lateral tail vein or into the mouse left ventricle (Auguello et al., Cancer Res. 48: 6876 81, 1988). In the absence of an effective M-CSF antagonist, osteolytic bone metastases formed from injected tumor cells may be determined by radiographs (areas of osteolytic bone lesions) or histochemistry (bone and soft tissues). Sasaki et al., Cancer Res. 55: 3551 7, 1995; Yoneda et al., J. Clin. Invest. 99: 2509 17, 1997. Clohisy and Ramnaraine, Orthop Res. 16: 660 6, 1998. Yin et al., J. Clin. Invest. 103: 197 206, 1999. In the presence of an effective M-CSF antigonist, osteolytic bone metastases may be prevented, or inhibited to result in fewer and/or smaller metastases.

The M-CSF antagonists of the present invention may also be useful in preventing or treating cancer metastasis. The effectiveness of a candidate M-CSF antagonist in preventing or treating cancer metastasis may be screened using a human amnionic basement membrane invasion model as described in Filderman et al., Cancer Res 52: 36616, 1992. In addition, any of the animal model systems for metastasis of various types of cancers may also be used. Such model systems include, but are not limited to, those described in Wenger et al., Clin. Exp. Metastasis 19: 169 73, 2002; Yi et al., Cancer Res. 62: 917 23, 2002; Tsutsumi et al., Cancer Lett 169: 77-85, 2001; Tsingotjidou et al., Anticancer Res. 21: 971 8, 2001; Wakabayashi et al., Oncology 59: 75 80, 2000; Culp and Kogerman, Front Biosci. 3:D672 83, 1998; Runge et al., Invest Radiol. 32: 212 7; Shioda et al., J. Surg. Oncol. 64: 122 6, 1997; Ma et al., Invest Ophthalmol Vis Sci. 37: 2293 301, 1996; Kuruppu et al., J Gastroenterol Hepatol. 11: 26 32, 1996. In the presence of an effective M-CSF antigonist, cancer metastases may be prevented, or inhibited to result in fewer and/or smaller metastases.

Identification of additional M-CSF antagonists may be achieved by using any of a number of known methods for identifying and obtaining proteins that specifically interact with other proteins or polypeptides, for example, a yeast two hybrid screening system such as that described in U.S. Patent No. 5,283,173 or the equivalent may be utilized. In one embodiment of the present invention, a cDNA encoding M-CSF, or a fragment thereof, may be cloned into a two hybrid bait vector and used to screen a complementary target library for a protein having M-CSF binding activity.

The anti-tumor activity of a particular M-CSF antagonist, or combination of M-CSF antagonists, may be evaluated *in vivo* using a suitable animal model. For example, xenogenic lymphoma cancer models wherein human lymphoma cells are introduced into immune compromised animals, such as nude or SCID mice. Efficacy may be predicted using assays which measure inhibition of tumor formation, tumor regression or metastasis, and the like.

The amino acid sequence information provided by the instant invention also makes possible the identification of binding partner compounds with which M-CSF or M-CSFR polypeptides or polynucleotides will interact. Methods to identify binding partner compounds include solution assays, in vitro assays wherein M-CSF or M-CSFR polypeptides are immobilized, and cell based assays. Identification of binding partner compounds of M-CSF or M-CSFR polypeptides provides candidates for therapeutic or prophylactic intervention in pathologies associated with normal or aberrant biological activity of M-CSF or M-CSFR.

The disclosre includes several assay systems for identifying binding partners for M-CSF or M-CSFR polypeptides. In solution assays, methods of the invention comprise the steps of (a) contacting M-CSF or M-CSFR polypeptides with one or more candidate binding partner compounds and (b) identifying the compounds that bind to the M-CSF or M-CSFR polypeptide(s). Identification of the compounds that bind to M-CSF or M-CSFR polypeptides can be achieved by isolating the M-CSF or M-CSFR polypeptide/binding partner complex, and separating the M-CSF or M-CSFR polypeptide from the binding partner compound. An additional step of characterizing the physical, biological, and/or biochemical properties of the binding partner compound is also comprehended in another embodiment of the invention. In one aspect, the M-CSF or M-CSFR polypeptide/binding partner complex is isolated using an antibody immunospecific for either the M-CSF or M-CSFR polypeptide or the candidate binding partner compound.

In still other embodiments, either the M-CSF or M-CSFR polypeptide or the candidate binding partner compound comprises a label or tag that facilitates its isolation, and methods of the disclosure to identify binding partner compounds include a step of isolating the M-CSF or M-CSFR polypeptide/binding partner complex through interaction with the label or tag. An exemplary tag of this type is a poly-histidine sequence, generally around six histidine residues, that permits isolation of a compound so labeled using nickel chelation. Other labels and tags, such as the FLAG® tag (Eastman Kodak, Rochester, NY), well known and routinely used in the art, are embraced by the disclosure.

In one variation of an *in vitro* assay, disclosed herein is a method comprising the steps of (a) contacting an immobilized M-CSF or M-CSFR polypeptide with a candidate binding partner compound and (b) detecting binding of the candidate compound to the M-CSF or M-CSFR polypeptide. In an alternative embodiment, the candidate binding partner compound is immobilized and binding of M-CSF or M-CSFR polypeptide is detected. Immobilization is accomplished using any of the methods well known in the art, including covalent bonding to a support, a bead, or a chromatographic resin, as well as non-covalent, high affinity interaction such as antibody binding, or use of streptavidin/biotin binding wherein the immobilized compound includes a biotin moiety. Detection of binding can be accomplished (i) using a radioactive label on the compound that is not immobilized, (ii) using a fluorescent label on the non-immobilized compound, (iii) using an antibody immunospecific for the non-immobilized compound, (iv) using a label on the non-immobilized compound that excites a fluorescent support to which the immobilized compound is attached, as well as other techniques well known and routinely practiced in the art.

Agents, e.g., antibodies or organic/inorganic chemical compounds that modulate (i.e., increase, decrease, or block) the activity or expression of M-CSF or M-CSFR polypeptides may be identified by incubating a putative modulator with a cell expressing a M-CSF or M-CSFR polypeptide or polynucleotide and determining the effect of the putative modulator on the activity or expression of the M-CSF or M-CSFR polypeptide or polynucleotide. The selectivity of a compound that modulates the activity of a M-CSF or M-CSFR polypeptide or polynucleotide can be evaluated by comparing its effects on the M-CSF or M-CSFR polypeptide or polynucleotide to its effect on other related compounds. Selective modulators may include, for example, antibodies and other proteins, peptides, or organic molecules which specifically bind to M-CSF or M-CSFR polypeptides or to a nucleic acid encoding a M-CSF or M-CSFR polypeptide. Modulators of M-CSF or M-CSFR polypeptide activity will be therapeutically useful in treatment of diseases and physiological conditions in which normal or aberrant activity of M-CSF or M-CSFR polypeptide is involved.

Methods of the disclosure to identify modulators include variations on any of the methods described above to identify binding partner compounds, the variations including techniques wherein a binding partner compound has been identified and the binding assay is carried out in the presence and absence of a candidate modulator. A modulator is identified in those instances where binding between M-CSF or M-CSFR polypeptides and the binding partner compound changes in the presence of the candidate modulator compared to binding in the absence of the candidate modulator compound. A modulator that increases binding between a M-CSF or M-CSFR polypeptide and the binding partner compound is described as an enhancer or activator, and a modulator that decreases binding between a M-CSF or M-CSFR polypeptide and the binding partner compound is described as an inhibitor.

The disclosure also comprehends high throughput screening (HTS) assays to identify compounds that interact with or inhibit biological activity (i.e., inhibit enzymatic activity, binding activity, etc.) of a M-CSF or M-CSFR polypeptide. HTS assays permit screening of large numbers of compounds in an efficient manner. Cell-based HTS systems are contemplated to investigate the interaction between M-CSF or M-CSFR polypeptides and their binding partners. HTS assays are designed to identify "hits" or "lead compounds" having the desired property, from which modifications can be designed to improve the desired property. Chemical modification of the "hit" or "lead compound" is often based on an identifiable structure/activity relationship between the "hit" and M-CSF or M-CSFR polypeptides.

Another aspect of the present disclosure is directed to methods of identifying compounds that bind to either a M-CSF or M-CSFR polypeptide or nucleic acid molecules encoding a M-CSF or M-CSFR polypeptide, comprising contacting a M-CSF or M-CSFR polypeptide, or a nucleic acid molecule encoding the same, with a compound, and determining whether the compound binds the M-CSF or M-CSFR polypeptide or a nucleic acid molecule encoding the same. Binding can be determined by binding assays which are well known to the skilled artisan, including, but not limited to, gel-shift assays, Western blots, radiolabeled competition assay, phage-based expression cloning, co-fractionation by chromatography, co-precipitation, cross linking, interaction trap/two-hybrid analysis, southwestern analysis, ELISA, and the like, which are described in, for example, Current Protocols in Molecular Biology (1999) John Wiley & Sons, NY. The compounds to be screened (which may include compounds which are suspected to bind a M-CSF or M-CSFR polypeptide, or a nucleic acid molecule encoding the same) include, but are not limited to, extracellular, intracellular, biologic or chemical origin. The methods of the disclosure also embrace ligands including substrates, adaptor or receptor molecules that are attached to a label, such as a radiolabel (e.g., ¹²⁵I, ³⁵S, ³²P, ³³P, ³H), a fluorescence label, a chemiluminescent label, an enzymic label or an immunogenic label. Modulators include, but are not limited to, non-peptide molecules such as non-peptide mimetics, non-peptide allosteric effectors, and peptides. The M-CSF or M-CSFR polypeptide or polynucleotide employed in such a test may either be free in solution, attached to a solid support, borne on a cell surface or located intracellularly or associated with a portion of a cell. One skilled in the art can, for example, measure the formation of complexes between a M-CSF or M-CSFR polypeptide and the compound being tested. Alternatively, one skilled in the art can examine the diminution in complex formation between a M-CSF or M-CSFR polypeptide and its substrate caused by the compound being tested.

Another aspect of the present disclosure is directed to methods of identifying compounds which modulate (i.e., decrease) activity of a M-CSF or M-CSFR polypeptide comprising contacting a M-CSF or M-CSFR polypeptide with a compound, and determining whether the compound modifies activity of the M-CSF or M-CSFR polypeptide. The activity in the presence of the test compared is measured to the activity in the absence of the test compound. Where the activity of the sample containing the test compound is higher than the activity in the sample lacking the test compound, the compound will have increased activity. Similarly, where the activity of the sample containing the test compound is lower than the activity in the sample lacking the test compound, the compound will have inhibited activity.

The present disclosure is particularly useful for screening compounds by using the M-CSF or M-CSFR polypeptides in any of a variety of drug screening techniques. The compounds to be screened (which may include compounds which are suspected to bind a M-CSF or M-CSFR polypeptide, or a nucleic acid molecule encoding the same) include, but are not limited to, extracellular, intracellular, biologic or chemical origin. The M-CSF or M-CSFR polypeptide or polynucleotide employed in such a test may either be free in solution, attached to a solid support, borne on a cell surface or located intracellularly or associated with a portion of a cell. One skilled in the art can, for example, measure the formation of complexes between a M-CSF or M-CSFR polypeptide and the compound being tested. Alternatively, one skilled in the art can examine the diminution in complex formation between a M-CSF or M-CSFR polypeptide and its substrate caused by the compound being tested.

The activity of M-CSF or M-CSFR polypeptides can be determined by, for example, examining their ability to bind chemically synthesized or naturally occurring peptide ligands. Alternatively, the activity of the M-CSF or M-CSFR polypeptides can be assayed by examining their ability to bind to adaptor molecules, receptor molecules, substrates or other ligands. The activity of the M-CSF or M-CSFR polypeptides can also be determined by examining the activity of effector molecules including, but not limited to other downstream enzymes that are activated by M-CSF or M-CSFR. Thus, modulators of M-CSF or M-CSFR polypeptide activity may alter a M-CSF or M-CSFR function, such as a binding property of a M-CSF or M-CSFR polypeptide. In various embodiments of the method, the assay may take the form of binding assays to natural binding partners, as well as other binding or function-based assays of M-CSF or M-CSFR activity that are generally known in the art. Biological activities of M-CSF or M-CSFR include, but are not limited to, the binding of a natural or an unnatural ligand, as well as any one of the functional activities of M-CSF or M-CSFR known in the art. Non-limiting examples of M-CSF or M-CSFR activities include proteolysis of substrates and binding to substrates, ligands, adaptor or receptor molecules.

The modulators of the disclosure exhibit a variety of chemical structures, which can be generally grouped into non-peptide mimetics of natural M-CSF or M-CSFR ligands, peptide and non-peptide allosteric effectors of M-CSF or M-CSFR, and peptides that may function as activators or inhibitors (competitive, uncompetitive and non-competitive) (e.g., antibody products) of M-CSF or M-CSFR. The disclosure does not restrict the sources for suitable modulators, which may be obtained from natural sources such as plant, animal or mineral extracts, or non-natural sources such as small molecule libraries, including the products of combinatorial chemical approaches to library construction, and peptide libraries.

Other assays can be used to examine enzymatic activity including, but not limited to, photometric, radiometric, HPLC, electrochemical, and the like, which are described in, for example, Enzyme Assays: A Practical Approach, eds. R. Eisenthal and M. J. Danson (1992) Oxford University Press.

cDNAs encoding M-CSF or M-CSFR polypeptides can be used in drug discovery programs; assays capable of testing thousands of unknown compounds per day in high-throughput screens (HTSs) are thoroughly documented. The literature is replete with examples of the use of radiolabeled ligands in HTS binding assays for drug discovery (see Williams, Medicinal Research Reviews (1991) 11, 147-184.; Sweetnam, et al., J. Natural Products (1993) 56, 441-455 for review). Immobilized M-CSF or M-CSFR are preferred for binding assay HTS because they allow for better specificity (higher relative purity), provide the ability to generate large amounts of M-CSF or M-CSFR material, and can be used in a broad variety of formats (see Hodgson, Bio/Technology (1992) 10:973-980)).

A variety of heterologous systems is available for functional expression of recombinant polypeptides that are well known to those skilled in the art. Such systems include bacteria (Strosberg, et al., Trends in Pharmacological Sciences (1992) 13:95-98), yeast (Pausch, Trends in Biotechnology (1997) 15:487-494), several kinds of insect cells (Vanden Broeck, Int. Rev. Cytology (1996) 164:189-268), amphibian cells (Jayawickreme et al., Current Opinion in Biotechnology (1997) 8: 629-634) and several mammalian cell lines (CHO, HEK293, COS, etc.; see Gerhardt, et al., Eur. J. Pharmacology (1997) 334:1-23). These examples do not preclude the use of other possible cell expression systems, including cell lines obtained from nematodes (PCT application WO 98/37177).

In preferred embodiments, methods of screening for compounds which modulate the activity of M-CSF or M-CSFR polypeptides comprise contacting test compounds with a M-CSF or M-CSFR polypeptide and assaying for the presence of a complex between the compound and the M-CSF or M-CSFR polypeptide. In such assays, the ligand is typically labeled. After suitable incubation, free ligand is separated from that present in bound form, and the amount of free or uncomplexed label is a measure of the ability of the particular compound to bind to the M-CSF or M-CSFR polypeptide

In another embodiment, high throughput screening for compounds having suitable binding affinity to a M-CSF or M-CSFR polypeptide is employed. Briefly, large numbers of different small peptide test compounds are synthesized on a solid substrate. The peptide test compounds are contacted with a M-CSF or M-CSFR polypeptide and washed. Bound M-CSF or M-CSFR polypeptides are then detected by methods well known in the art. Purified polypeptides of the disclosure can also be coated directly onto plates for use in the aforementioned drug screening techniques. In addition, non-neutralizing antibodies can be used to capture the protein and immobilize it on the solid support.

Generally, an expressed M-CSF or M-CSFR polypeptide can be used for HTS binding assays in conjunction with a substrate, ligand, adaptor or receptor molecule that is labeled with a suitable radioisotope, including, but not limited to, ¹²⁵I, ³H, ³⁵S or ³²P, by methods that are well known to those skilled in the art. Alternatively, the substrate, ligand, adaptor or receptor molecule may be labeled by well-known methods with a suitable fluorescent derivative (Baindur, et al., Drug Dev. Res., (1994) 33:373-398; Rogers, Drug Discovery Today (1997) 2:156-160). Radioactive ligand specifically bound to immobilized M-CSF or M-CSFR can be detected in HTS assays in one of several standard ways, including filtration of the M-CSF or M-CSFR -ligand complex to separate bound ligand from unbound ligand (Williams, Med. Res. Rev. (1991) 11, 147-184; Sweetnam, et al., J. Natural Products (1993) 56, 441-455). Alternative methods include a scintillation proximity assay (SPA) or a FlashPlate format in which such separation is unnecessary (Nakayama, Cur. Opinion Drug Disc. Dev. (1998) 1:85-91 Bossé, et al., J. Biomolecular Screening (1998) 3: 285-292.). Binding of fluorescent ligands can be detected in various ways, including fluorescence energy transfer (FRET), direct spectrophotofluorometric analysis of bound ligand, or fluorescence polarization (Rogers, Drug Discovery Today (1997) 2, 156-160; Hill, Cur. Opinion Drug Disc. Dev. (1998) 1, 92-97).

The disclosure contemplates a multitude of assays to screen and identify inhibitors of substrate, ligand, adaptor or receptor binding to M-CSF or M-CSFR. In one example, M-CSF or M-CSFR is immobilized and interaction with a binding partner is assessed in the presence and absence of a candidate modulator such as an inhibitor compound. In another example, interaction between M-CSF or M-CSFR and its binding partner is assessed in a solution assay, both in the presence and absence of a candidate inhibitor compound. In either assay, an inhibitor is identified as a compound that decreases binding between the M-CSF or M-CSFR and its binding partner. Another contemplated assay involves a variation of the di-hybrid assay wherein an inhibitor of protein/protein interactions is identified by detection of a positive signal in a transformed or transfected host cell, as described in PCT publication number WO 95/20652, published August 3, 1995.

Candidate modulators contemplated by the disclosure include compounds selected from libraries of either potential activators or potential inhibitors. There are a number of different libraries used for the identification of small molecule modulators, including: (1) chemical libraries, (2) natural product libraries, and (3) combinatorial libraries comprised of random peptides, oligonucleotides or organic molecules. Chemical libraries consist of random chemical structures, some of which are analogs of known compounds or analogs of compounds that have been identified as "hits" or "leads" in other drug discovery screens, some of which are derived from natural products, and some of which arise from non-directed synthetic organic chemistry. Natural product libraries are collections of microorganisms, animals, plants, or marine organisms that are used to create mixtures for screening by: (1) fermentation and extraction of broths from soil, plant or marine microorganisms or (2) extraction of plants or marine organisms. Natural product libraries include polyketides, non-ribosomal peptides, and variants (non-naturally occurring) thereof. For a review, see Science 282:63-68 (1998). Combinatorial libraries are composed of large numbers of peptides, oligonucleotides, or organic compounds as a mixture. These libraries are relatively easy to prepare by traditional automated synthesis methods, PCR, cloning, or proprietary synthetic methods. Of particular interest are non-peptide combinatorial libraries. Still other libraries of interest include peptide, protein, peptidomimetic, multiparallel synthetic collection, recombinatorial, and polypeptide libraries. For a review of combinatorial chemistry and libraries created therefrom, see Myers, Curr. Opin. Biotechnol. 8:701-707 (1997). Identification of modulators through use of the various libraries described herein permits modification of the candidate "hit" (or "lead") to optimize the capacity of the "hit" to modulate activity.

Still other candidate inhibitors contemplated by the disclosure can be designed and include soluble forms of binding partners, as well as such binding partners as chimeric, or fusion, proteins. A "binding partner" as used herein broadly encompasses non-peptide modulators, as well as peptide modulator including antibodies, antibody fragments, and modified compounds comprising antibody domains that are immunospecific for M-CSF or M-CSFR.

Other assays may be used to identify specific ligands of M-CSF or M-CSFR, including assays that identify ligands of the target protein through measuring direct binding of test ligands to the target protein, as well as assays that identify ligands of target proteins through affinity ultrafiltration with ion spray mass spectroscopy/HPLC methods or other physical and analytical methods. Alternatively, such binding interactions are evaluated indirectly using the yeast two-hybrid system described in Fields et al., Nature, 340:245-246 (1989), and Fields et al., Trends in Genetics, 10:286-292 (1994). The two-hybrid system is a genetic assay for detecting interactions between two proteins or polypeptides. It can be used to identify proteins that bind to a known protein of interest, or to delineate domains or residues critical for an interaction. Variations on this methodology have been developed to clone genes that encode DNA binding proteins, to identify peptides that bind to a protein, and to screen for drugs. The two-hybrid system exploits the ability of a pair of interacting proteins to bring a transcription activation domain into close proximity with a DNA binding domain that binds to an upstream activation sequence (UAS) of a reporter gene, and is generally performed in yeast. The assay requires the construction of two hybrid genes encoding (1) a DNA-binding domain that is fused to a first protein and (2) an activation domain fused to a second protein. The DNA-binding domain targets the first hybrid protein to the UAS of the reporter gene; however, because most proteins lack an activation domain, this DNA-binding hybrid protein does not activate transcription of the reporter gene. The second hybrid protein, which contains the activation domain, cannot by itself activate expression of the reporter gene because it does not bind the UAS. However, when both hybrid proteins are present, the noncovalent interaction of the first and second proteins tethers the activation domain to the UAS, activating transcription of the reporter gene. For example, when the first protein is M-CSF or M-CSFR, or subunit or fragment thereof, that is known to interact with another protein or nucleic acid, this assay can be used to detect agents that interfere with the binding interaction. Expression of the reporter gene is monitored as different test agents are added to the system. The presence of an inhibitory agent results in lack of a reporter signal.

The yeast two-hybrid assay can also be used to identify proteins that bind to M-CSF or M-CSFR. In an assay to identify proteins that bind to M-CSF or M-CSFR, or subunit or fragment thereof, a fusion polynucleotide encoding both a M-CSF or M-CSFR (or subunit or fragment) and a UAS binding domain (i.e., a first protein) may be used. In addition, a large number of hybrid genes each encoding a different second protein fused to an activation domain are produced and screened in the assay. Typically, the second protein is encoded by one or more members of a total cDNA or genomic DNA fusion library, with each second protein-coding region being fused to the activation domain. This system is applicable to a wide variety of proteins, and it is not even necessary to know the identity or function of the second binding protein. The system is highly sensitive and can detect interactions not revealed by other methods; even transient interactions may trigger transcription to produce a stable mRNA that can be repeatedly translated to yield the reporter protein.

Other assays may be used to search for agents that bind to the target protein. One such screening method to identify direct binding of test ligands to a target protein is described in U.S. Patent No. 5,585,277. This method relies on the principle that proteins generally exist as a mixture of folded and unfolded states, and continually alternate between the two states. When a test ligand binds to the folded form of a target protein (i.e., when the test ligand is a ligand of the target protein), the target protein molecule bound by the ligand remains in its folded state. Thus, the folded target protein is present to a greater extent in the presence of a test ligand which binds the target protein, than in the absence of a ligand. Binding of the ligand to the target protein can be determined by any method which distinguishes between the folded and unfolded states of the target protein. The function of the target protein need not be known in order for this assay to be performed. Virtually any agent can be assessed by this method as a test ligand, including, but not limited to, metals, polypeptides, proteins, lipids, polysaccharides, polynucleotides and small organic molecules.

Another method for identifying ligands of a target protein is described in Wieboldt et al., Anal. Chem., 69:1683-1691 (1997). This technique screens combinatorial libraries of 20-30 agents at a time in solution phase for binding to the target protein. Agents that bind to the target protein are separated from other library components by simple membrane washing. The specifically selected molecules that are retained on the filter are subsequently liberated from the target protein and analyzed by HPLC and pneumatically assisted electrospray (ion spray) ionization mass spectroscopy. This procedure selects library components with the greatest affinity for the target protein, and is particularly useful for small molecule libraries.

Other embodiments comprise using competitive screening assays in which neutralizing antibodies capable of binding a polypeptide of the invention specifically compete with a test compound for binding to the polypeptide. In this manner, the antibodies can be used to detect the presence of any peptide that shares one or more antigenic determinants with M-CSF or M-CSFR. Radiolabeled competitive binding studies are described in A.H. Lin et al. Antimicrobial Agents and Chemotherapy (1997) vol. 41, no. 10. pp. 2127-2131.

In other embodiments, the polypeptides of the disclosure are employed as a research tool for identification, characterization and purification of interacting, regulatory proteins. Appropriate labels are incorporated into the polypeptides of the invention by various methods known in the art and the polypeptides are used to capture interacting molecules. For example, molecules are incubated with the labeled polypeptides, washed to removed unbound polypeptides, and the polypeptide complex is quantified. Data obtained using different concentrations of polypeptide are used to calculate values for the number, affinity, and association of polypeptide with the protein complex.

Labeled polypeptides are also useful as reagents for the purification of molecules with which the polypeptide interacts including, but not limited to, inhibitors. In one embodiment of affinity purification, a polypeptide is covalently coupled to a chromatography column. Cells and their membranes are extracted, and various cellular subcomponents are passed over the column. Molecules bind to the column by virtue of their affinity to the polypeptide. The polypeptide-complex is recovered from the column, dissociated and the recovered molecule is subjected to protein sequencing. This amino acid sequence is then used to identify the captured molecule or to design degenerate oligonucleotides for cloning the corresponding gene from an appropriate cDNA library.

Alternatively, compounds may be identified which exhibit similar properties to the ligand for M-CSF or M-CSFR, but which are smaller and exhibit a longer half time than the endogenous ligand in a human or animal body. When an organic compound is designed, a molecule according to the invention is used as a "lead" compound. The design of mimetics to known pharmaceutically active compounds is a well-known approach in the development of pharmaceuticals based on such "lead" compounds. Mimetic design, synthesis and testing are generally used to avoid randomly screening a large number of molecules for a target property. Furthermore, structural data deriving from the analysis of the deduced amino acid sequences encoded by the polynucleotides of the present invention are useful to design new drugs, more specific and therefore with a higher pharmacological potency.

Computer modeling can be used to develop a putative tertiary structure of the proteins of the invention based on the available information on M-CSF or M-CSFR. Thus, novel ligands based on the predicted structure of M-CSF or M-CSFR can be designed.

Another aspect of the present disclosure is the use of the M-CSF or M-CSFR nucleotide sequences disclosed herein for identifying homologs in other animals. Any of the nucleotide sequences disclosed herein, or any portion thereof, can be used, for example, as probes to screen databases or nucleic acid libraries, such as, for example, genomic or cDNA libraries, to identify homologs, using screening procedures well known to those skilled in the art. Accordingly, homologs having at least 50%, more preferably at least 60%, more preferably at least 70%, more preferably at least 80%, more preferably at least 90%, more preferably at least 95%, and most preferably at least 100% homology with M-CSF or M-CSFR sequences can be identified.

### Combination Therapy

Having identified more than one M-CSF antagonist that is effective in an animal model, it may be further advantageous to mix two or more such M-CSF antagonists together to provide still improved efficacy against cancer metastasis and/or bone loss associated with cancer metastasis. Compositions comprising one or more M-CSF antagonist may be administered to persons or mammals suffering from, or predisposed to suffer from, cancer metastasis and/or bone loss associated with cancer metastasis.

Although M-CSF antagonist therapy may be useful for all stages of cancers, antibody therapy may be particularly appropriate in advanced or metastatic cancers. Combining the antibody therapy method a chemotherapeutic or radiation regimen may be preferred in patients that have not received chemotherapeutic treatment, whereas treatment with the antibody therapy may be indicated for patients who have received one or more chemotherapies. Additionally, antibody therapy can also enable the use of reduced dosages of concomitant chemotherapy, particularly in patients that do not tolerate the toxicity of the chemotherapeutic agent very well.

The method of the disclosure contemplates the administration of single anti-M-CSF and anti-M-CSFR antibodies, as well as combinations, or "cocktails", of different antibodies. Such antibody cocktails may have certain advantages inasmuch as they contain antibodies which exploit different effector mechanisms or combine directly cytotoxic antibodies with antibodies that rely on immune effector functionality. Such antibodies in combination may exhibit synergistic therapeutic effects. In addition, the administration of anti-M-CSF and anti-M-CSFR antibodies may be combined with other therapeutic agents and/or procedures, including but not limited to various chemotherapeutic agents, androgen-blockers, and immune modulators (e.g., IL-2, GM-CSF), Bisphosphonate(s) (e.g., Aredia; Zometa; Clodronate), surgery, radiation, chemotherapy, hormone therapy (e.g., Tamoxifen; anti-Androgen therapy), antibody therapy (e.g., RANKL/RANK neutralizing antibodies; PTHrP neutralizing antibody, anti-Her2 antibody, VEGF neutralizing antibody), therapeutic protein therapy (e.g., soluble RANKL receptor; OPG, and PDGF and MMP inhibitors), small molecule drug therapy (e.g., Src-kinase inhibitor), kinase inhibitors of growth factor receptors; oligonucleotides therapy (e.g., RANKL or RANK or PTHrP Anti-sense), gene therapy (e.g., RANKL or RANK inhibitors), peptide therapy (e.g. muteins of RANKL) as well as those proteins, peptides, compounds, and small molecules described herein.

Cancer chemotherapeutic agents include, without limitation, alkylating agents, such as carboplatin and cisplatin; nitrogen mustard alkylating agents; nitrosourea alkylating agents, such as carmustine (BCNU); antimetabolites, such as methotrexate; purine analog antimetabolites, mercaptopurine; pyrimidine analog antimetabolites, such as fluorouracil (5-FU) and gemcitabine; hormonal antineoplastics, such as goserelin, leuprolide, and tamoxifen; natural antineoplastics, such as aldesleukin, interleukin-2, docetaxel, etoposide (VP-16), interferon alfa, paclitaxel, and tretinoin (ATRA); antibiotic natural antineoplastics, such as bleomycin, dactinomycin, daunorubicin, doxorubicin, and mitomycin; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide, Schizophyllan, cytarabine, dacarbazine, thioinosine, thiotepa, tegafur, , neocarzinostatin, OK-432, bleomycin, furtulon, broxuridine, busulfan, honvan, peplomycin, , Bestatin (ubenimex), interferon- β, mepitiostane, mitobronitol, merphalan, laminin peptides, lentinan, Coriolus versicolor extract, tegafur/uracil, estramustine (estrogen/mechlorethamine).

Further, additional agents used as therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ hormones, such as luteinizing hormone releasing hormone (LHRH) and analogues and, gonadotropin releasing hormone (GnRH); growth factors, such as transforming growth factor- β (TGF- β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor- α & β (TNF- α & β); invasion inhibiting factor-2 (IIF-2); bone morphogenetic proteins 1-7 (BMP 1-7); somatostatin; thymosin- α -1; γ-globulin; superoxide dismutase (SOD); complement factors; anti-angiogenesis factors; antigenic materials; and pro-drugs.

### Administration and preparation

The present disclosure provides compounds, pharmaceutical formulations including the compounds, methods of preparing the pharmaceutical formulations, and methods of treating patients with the pharmaceutical formulations and compounds.

Such compositions can be in the form of, for example, granules, powders, tablets, capsules, syrup, suppositories, injections, emulsions, elixirs, suspensions or solutions. The instant compositions can be formulated for various routes of administration, for example, by oral administration, by nasal administration, by rectal administration, subcutaneous injection, intravenous injection, intramuscular injections, or intraperitoneal injection. The following dosage forms are given by way of example and should not be construed as limiting the instant invention.

For oral, buccal, and sublingual administration, powders, suspensions, granules, tablets, pills, capsules, gelcaps, and caplets are acceptable as solid dosage forms. These can be prepared, for example, by mixing one or more compounds of the instant invention, or pharmaceutically acceptable salts or tautomers thereof, with at least one additive such as a starch or other additive. Suitable additives are sucrose, lactose, cellulose sugar, mannitol, maltitol, dextran, starch, agar, alginates, chitins, chitosans, pectins, tragacanth gum, gum arabic, gelatins, collagens, casein, albumin, synthetic or semi-synthetic polymers or glycerides. Optionally, oral dosage forms can contain other ingredients to aid in administration, such as an inactive diluent, or lubricants such as magnesium stearate, or preservatives such as paraben or sorbic acid, or anti-oxidants such as ascorbic acid, tocopherol or cysteine, a disintegrating agent, binders, thickeners, buffers, sweeteners, flavoring agents or perfuming agents. Tablets and pills may be further treated with suitable coating materials known in the art.

Liquid dosage forms for oral administration may be in the form of pharmaceutically acceptable emulsions, syrups, elixirs, suspensions, and solutions, which may contain an inactive diluent, such as water. Pharmaceutical formulations and medicaments may be prepared as liquid suspensions or solutions using a sterile liquid, such as, but not limited to, an oil, water, an alcohol, and combinations of these. Pharmaceutically suitable surfactants, suspending agents, emulsifying agents, may be added for oral or parenteral administration.

As noted above, suspensions may include oils. Such oil include, but are not limited to, peanut oil, sesame oil, cottonseed oil, corn oil and olive oil. Suspension preparation may also contain esters of fatty acids such as ethyl oleate, isopropyl myristate, fatty acid glycerides and acetylated fatty acid glycerides. Suspension formulations may include alcohols, such as, but not limited to, ethanol, isopropyl alcohol, hexadecyl alcohol, glycerol and propylene glycol. Ethers, such as but not limited to, poly(ethyleneglycol), petroleum hydrocarbons such as mineral oil and petrolatum; and water may also be used in suspension formulations.

For nasal administration, the pharmaceutical formulations and medicaments may be a spray or aerosol containing an appropriate solvent(s) and optionally other compounds such as, but not limited to, stabilizers, antimicrobial agents, antioxidants, pH modifiers, surfactants, bioavailability modifiers and combinations of these. A propellant for an aerosol formulation may include compressed air, nitrogen, carbon dioxide, or a hydrocarbon based low boiling solvent.

Injectable dosage forms generally include aqueous suspensions or oil suspensions which may be prepared using a suitable dispersant or wetting agent and a suspending agent. Injectable forms may be in solution phase or in the form of a suspension, which is prepared with a solvent or diluent. Acceptable solvents or vehicles include sterilized water, Ringer's solution, or an isotonic aqueous saline solution. Alternatively, sterile oils may be employed as solvents or suspending agents. Preferably, the oil or fatty acid is non-volatile, including natural or synthetic oils, fatty acids, mono-, di- or tri-glycerides.

For injection, the pharmaceutical formulation and/or medicament may be a powder suitable for reconstitution with an appropriate solution as described above. Examples of these include, but are not limited to, freeze dried, rotary dried or spray dried powders, amorphous powders, granules, precipitates, or particulates. For injection, the formulations may optionally contain stabilizers, pH modifiers, surfactants, bioavailability modifiers and combinations of these.

For rectal administration, the pharmaceutical formulations and medicaments may be in the form of a suppository, an ointment, an enema, a tablet or a cream for release of compound in the intestines, sigmoid flexure and/or rectum. Rectal suppositories are prepared by mixing one or more compounds of the instant invention, or pharmaceutically acceptable salts or tautomers of the compound, with acceptable vehicles, for example, cocoa butter or polyethylene glycol, which is present in a solid phase at normal storing temperatures, and present in a liquid phase at those temperatures suitable to release a drug inside the body, such as in the rectum. Oils may also be employed in the preparation of formulations of the soft gelatin type and suppositories. Water, saline, aqueous dextrose and related sugar solutions, and glycerols may be employed in the preparation of suspension formulations which may also contain suspending agents such as pectins, carbomers, methyl cellulose, hydroxypropyl cellulose or carboxymethyl cellulose, as well as buffers and preservatives.

Besides those representative dosage forms described above, pharmaceutically acceptable excipients and carries are generally known to those skilled in the art and are thus included in the instant invention. Such excipients and carriers are described, for example, in "Remingtons Pharmaceutical Sciences" Mack Pub. Co., New Jersey (1991).

The formulations may be designed to be short-acting, fast-releasing, long-acting, and sustained-releasing as described below. Thus, the pharmaceutical formulations may also be formulated for controlled release or for slow release.

The instant compositions may also comprise, for example, micelles or liposomes, or some other encapsulated form, or may be administered in an extended release form to provide a prolonged storage and/or delivery effect. Therefore, the pharmaceutical formulations and medicaments may be compressed into pellets or cylinders and implanted intramuscularly or subcutaneously as depot injections or as implants such as stents. Such implants may employ known inert materials such as silicones and biodegradable polymers.

Specific dosages may be adjusted depending on conditions of disease, the age, body weight, general health conditions, sex, and diet of the subject, dose intervals, administration routes, excretion rate, and combinations of drugs. Any of the above dosage forms containing effective amounts are well within the bounds of routine experimentation and therefore, well within the scope of the instant invention.

By the present methods, compositions comprising M-CSF antagonists may be administered parenterally, topically, orally or locally for therapeutic treatment. Preferably, the compositions are administered orally or parenterally, i.e., intravenously, intraperitoneally, intradermally or intramuscularly. Thus, disclosed herein are methods which employ compositions for administration which comprise one or more M-CSF antagonists in a pharmaceutically acceptable carrier, preferably an aqueous carrier. A variety of aqueous carriers may be used, e.g., water, buffered water, 0.4% saline, 0.3% glycine and the like, and may include other proteins for enhanced stability, such as albumin, lipoprotein, globulin, etc., subjected to mild chemical modifications or the like.

M-CSF antagonists useful as therapeutics for cancer metastasis or bone loss associated with cancer metastasis will often be prepared substantially free of other naturally occurring immunoglobulins or other biological molecules. Preferred M-CSF antagonists will also exhibit minimal toxicity when administered to a mammal afflicted with, or predisposed to suffer from, cancer metastasis and/or bone loss associated with cancer metastasis.

The compositions disclosed herein may be sterilized by conventional, well known sterilization techniques. The resulting solutions may be packaged for use or filtered under aseptic conditions and lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride and stabilizers (e.g., 1 20% maltose, etc.).

The M-CSF antagonists of the present invention may also be administered via liposomes. Liposomes, which include emulsions, foams, micelles, insoluble monolayers, phospholipid dispersions, lamellar layers and the like, can serve as vehicles to target the M-CSF antagonists to a particular tissue as well as to increase the half life of the composition. A variety of methods are available for preparing liposomes, as described in, e.g., U.S. Patent Nos. 4,837,028 and 5,019,369.

The concentration of the M-CSF antagonist in these compositions can vary widely, i.e., from less than about 10%, usually at least about 25% to as much as 75% or 90% by weight and will be selected primarily by fluid volumes, viscosities, etc., in accordance with the particular mode of administration selected. Actual methods for preparing orally, topically and parenterally administrable compositions will be known or apparent to those skilled in the art and are described in detail in, for example, Remington's Pharmaceutical Science, 19th ed., Mack Publishing Co., Easton, PA (1995).

Determination of an effective amount of a composition of the invention to treat cancer metastasis and/or bone loss associated with cancer metastasis in a patient can be accomplished through standard empirical methods which are well known in the art. For example, the in vivo neutralizing activity of sera from a subject treated with a given dosage of M-CSF antagonist may be evaluated using an assay that determines the ability of the sera to block M-CSF induced proliferation and survival of murine monocytes (CD11b+ cell, a subset of CD11 cells, which expresses high levels of receptor to M-CSF) in vitro as described in Cenci et al., J Clin. Invest. 1055: 1279-87, 2000.

Compositions of the disclosure are administered to a mammal already suffering from, or predisposed to, cancer metastasis and/or bone loss associated with cancer metastasis in an amount sufficient to prevent or at least partially arrest the development of cancer metastasis and/or bone loss associated with cancer metastasis. An amount adequate to accomplish this is defined as a "therapeutically effective dose." Effective amounts of a M-CSF antagonist will vary and depend on the severity of the disease and the weight and general state of the patient being treated, but generally range from about 1.0 µg/kg to about 100 mg/kg body weight, with dosages of from about 10 µg/kg to about 10 mg/kg per application being more commonly used. Administration is daily, weekly or less frequently, as necessary depending on the response to the disease and the patient's tolerance of the therapy. Maintenance dosages over a prolonged period of time may be needed, and dosages may be adjusted as necessary.

Single or multiple administrations of the compositions can be carried out with the dose levels and pattern being selected by the treating physician. In any event, the formulations should provide a quantity of M-CSF antagonist sufficient to effectively prevent or minimize the severity of cancer metastasis and/or bone loss associated with cancer metastasis. The compositions may be administered alone or as an adjunct therapy in conjunction with other therapeutics known in the art for the treatment of cancer metastasis and/or bone loss associated with cancer metastasis.

### II. Immunotherapy

Anti-M-CSF and anti-M-CSFR antibodies useful in treating cancers include those which are capable of initiating a potent immune response against the tumor and those which are capable of direct cytotoxicity. In this regard, anti-M-CSF and anti-M-CSFR antibodies may elicit tumor cell lysis by either complement-mediated or antibody-dependent cell cytotoxicity (ADCC) mechanisms, both of which require an intact Fc portion of the immunoglobulin molecule for interaction with effector cell Fc receptor sites or complement proteins. In addition, anti-M-CSF and anti-M-CSFR antibodies that exert a direct biological effect on tumor growth are useful in the practice of the invention. Potential mechanisms by which such directly cytotoxic antibodies may act include inhibition of cell growth, modulation of cellular differentiation, modulation of tumor angiogenesis factor profiles, and the induction of apoptosis. The mechanism by which a particular anti-M-CSF and anti-M-CSFR antibody exerts an anti-tumor effect may be evaluated using any number of *in vitro* assays designed to determine ADCC, ADMMC, complement-mediated cell lysis, and so forth, as is generally known in the art.

In one embodiment, immunotherapy is carried out using antibodies that have a higher affinity for the membrane-bound form of M-CSF (M-CSFα) than for the secreted forms of M-CSF. For example, antibodies may be prepared that specifically bind at or around the cleavage site of M-CSFα or to the portion of M-CSFα adjacent to the membrane. Such antibodies may also beneficially inhibit cleavage and release of the soluble active portion of M-CSFα.

The anti-M-CSF and anti-M-CSFR antibodies may be administered in their "naked" or unconjugated form, or may have therapeutic agents conjugated to them. In one embodiment, anti-M-CSF and anti-M-CSFR antibodies are used as a radiosenitizer. In such embodiments, the anti-M-CSF and anti-M-CSFR antibodies are conjugated to a radiosensitizing agent. The term "radiosensitizer," as used herein, is defined as a molecule, preferably a low molecular weight molecule, administered to animals in therapeutically effective amounts to increase the sensitivity of the cells to be radiosensitized to electromagnetic radiation and/or to promote the treatment of diseases that are treatable with electromagnetic radiation. Diseases that are treatable with electromagnetic radiation include neoplastic diseases, benign and malignant tumors, and cancerous cells.

The terms "electromagnetic radiation" and "radiation" as used herein include, but are not limited to, radiation having the wavelength of 10⁻²⁰ to 100 meters. Preferred embodiments of the present invention employ the electromagnetic radiation of: gamma-radiation (10⁻²⁰ to 10⁻¹³ m), X-ray radiation (10⁻¹² to 10⁻⁹ m), ultraviolet light (10 nm to 400 nm), visible light (400 nm to 700 nm), infrared radiation (700 nm to 1.0 mm), and microwave radiation (1 mm to 30 cm).

Radiosensitizers are known to increase the sensitivity of cancerous cells to the toxic effects of electromagnetic radiation. Many cancer treatment protocols currently employ radiosensitizers activated by the electromagnetic radiation of X-rays. Examples of X-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5-iododeoxyuridine (IUdR), bromodeoxycytidine, fluorodeoxyuridine (FUdR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin(r), benzoporphyrin derivatives, NPe6, tin etioporphyrin (SnET2), pheoborbide-a, bacteriochlorophyll-a, naphthalocyanines, phthalocyanines, zinc phthalocyanine, and therapeutically effective analogs and derivatives of the same.

The anti-M-CSF and anti-M-CSFR antibodies used in the practice of a method of the invention may be formulated into pharmaceutical compositions comprising a carrier suitable for the desired delivery method. Suitable carriers include any material which, when combined with the anti-M-CSF and anti-M-CSFR antibodies, retains the anti-tumor function of the antibody and is nonreactive with the subject's immune systems. Examples include, but are not limited to, any of a number of standard pharmaceutical carriers such as sterile phosphate buffered saline solutions, bacteriostatic water, and the like.

The present invention further provides the above-described antibodies in detectably labeled form. Antibodies can be detectably labeled through the use of radioisotopes, affinity labels (such as biotin, avidin, etc.), enzymatic labels (such as horseradish peroxidase, alkaline phosphatase, etc.) fluorescent labels (such as FITC or rhodamine, etc.), paramagnetic atoms, and the like. Procedures for accomplishing such labeling are well known in the art; for example, see (Sternberger, L.A. et al., J. Histochem. Cytochem. 18:315 (1970); Bayer, E.A. et al., Meth. Enzym. 62:308 (1979); Engval, E. et al., Immunol. 109:129 (1972); Goding, J.W. J. Immunol. Meth. 13:215 (1976)).

The present invention contemplates the use of "naked" anti-M-CSF and anti-M-CSFR antibodies, as well as the use of immunoconjugates. Production of immunconjugates is described in U.S. Patent No. 6,306,393. Immnunoconjugates can be prepared by indirectly conjugating a therapeutic agent to an antibody component. General techniques are described in Shih et al., Int. J. Cancer 41:832-839 (1988); Shih et al., Int. J. Cancer 46:1101-1106 (1990); and Shih et al., U.S. Pat. No. 5,057,313. The general method involves reacting an antibody component having an oxidized carbohydrate portion with a carrier polymer that has at least one free amine function and that is loaded with a plurality of drug, toxin, chelator, boron addends, or other therapeutic agent. This reaction results in an initial Schiff base (imine) linkage, which can be stabilized by reduction to a secondary amine to form the final conjugate.

The carrier polymer is preferably an aminodextran or polypeptide of at least 50 amino acid residues, although other substantially equivalent polymer carriers can also be used. Preferably, the final immunoconjugate is soluble in an aqueous solution, such as mammalian serum, for ease of administration and effective targeting for use in therapy. Thus, solubilizing functions on the carrier polymer will enhance the serum solubility of the final immunoconjugate. In particular, an aminodextran will be preferred.

The process for preparing an inmmunoconjugate with an aminodextran carrier typically begins with a dextran polymer, advantageously a dextran of average molecular weight of about 10,000-100,000. The dextran is reacted with an oxidizing agent to affect a controlled oxidation of a portion of its carbohydrate rings to generate aldehyde groups. The oxidation is conveniently effected with glycolytic chemical reagents such as NaIO₄, according to conventional procedures.

The oxidized dextran is then reacted with a polyamine, preferably a diamine, and more preferably, a mono- or polyhydroxy diamine. Suitable amines include ethylene diamine, propylene diamine, or other like polymethylene diamines, diethylene triamine or like polyamines, 1,3-diamino-2-hydroxypropane, or other like hydroxylated diamines or polyamines, and the like. An excess of the amine relative to the aldehyde groups of the dextran is used to ensure substantially complete conversion of the aldehyde functions to Schiff base groups.

A reducing agent, such as NaBH₄, NaBH₃ CN or the like, is used to effect reductive stabilization of the resultant Schiff base intermediate. The resultant adduct can be purified by passage through a conventional sizing column to remove cross-linked dextrans.

Other conventional methods of derivatizing a dextran to introduce amine functions can also be used, e.g., reaction with cyanogen bromide, followed by reaction with a diamine.

The amninodextran is then reacted with a derivative of the particular drug, toxin, chelator, immunomodulator, boron addend, or other therapeutic agent to be loaded, in an activated form, preferably, a carboxyl-activated derivative, prepared by conventional means, e.g., using dicyclohexylcarbodiimide (DCC) or a water soluble variant thereof, to form an intermediate adduct.

Alternatively, polypeptide toxins such as pokeweed antiviral protein or ricin A-chain, and the like, can be coupled to aminodextran by glutaraldehyde condensation or by reaction of activated carboxyl groups on the protein with amines on the aminodextran.

Chelators for radiometals or magnetic resonance enhancers are well-known in the art. Typical are derivatives of ethylenediaminetetraacetic acid (EDTA) and diethylenetriaminepentaacetic acid (DTPA). These chelators typically have groups on the side chain by which the chelator can be attached to a carrier. Such groups include, *e.g.,* benzylisothiocyanate, by which the DTPA or EDTA can be coupled to the amine group of a carrier. Alternatively, carboxyl groups or amine groups on a chelator can be coupled to a carrier by activation or prior derivatization and then coupling, all by well-known means.

Boron addends, such as carboranes, can be attached to antibody components by conventional methods. For example, carboranes can be prepared with carboxyl functions on pendant side chains, as is well known in the art. Attachment of such carboranes to a carrier, e.g., aminodextran, can be achieved by activation of the carboxyl groups of the carboranes and condensation with amines on the carrier to produce an intermediate conjugate. Such intermediate conjugates are then attached to antibody components to produce therapeutically useful immunoconjugates, as described below.

A polypeptide carrier can be used instead of aminodextran, but the polypeptide carrier should have at least 50 amino acid residues in the chain, preferably 100-5000 amino acid residues. At least some of the amino acids should be lysine residues or glutamate or aspartate residues. The pendant amines of lysine residues and pendant carboxylates of glutamine and aspartate are convenient for attaching a drug, toxin, immunomodulator, chelator, boron addend or other therapeutic agent. Examples of suitable polypeptide carriers include polylysine, polyglutamic acid, polyaspartic acid, co-polymers thereof, and mixed polymers of these amino acids and others, e.g., serines, to confer desirable solubility properties on the resultant loaded carrier and immunoconjugate.

Conjugation of the intermediate conjugate with the antibody component is effected by oxidizing the carbohydrate portion of the antibody component and reacting the resulting aldehyde (and ketone) carbonyls with amine groups remaining on the carrier after loading with a drug, toxin, chelator, immunomodulator, boron addend, or other therapeutic agent. Alternatively, an intermediate conjugate can be attached to an oxidized antibody component via amine groups that have been introduced in the intermediate conjugate after loading with the therapeutic agent. Oxidation is conveniently effected either chemically, e.g., with NaIO₄ or other glycolytic reagent, or enzymatically, e.g., with neuraminidase and galactose oxidase. In the case of an aminodextran carrier, not all of the amines of the aminodextran are typically used for loading a therapeutic agent. The remaining amines of aminodextran condense with the oxidized antibody component to form Schiff base adducts, which are then reductively stabilized, normally with a borohydride reducing agent.

Analogous procedures are used to produce other immunoconjugates according to the invention. Loaded polypeptide carriers preferably have free lysine residues remaining for condensation with the oxidized carbohydrate portion of an antibody component. Carboxyls on the polypeptide carrier can, if necessary, be converted to amines by, e.g., activation with DCC and reaction with an excess of a diamme.

The final immunoconjugate is purified using conventional techniques, such as sizing chromatography on Sephacryl S-300 or affinity chromatography using one or more CD84Hy epitopes.

Alternatively, immunoconjugates can be prepared by directly conjugating an antibody component with a therapeutic agent. The general procedure is analogous to the indirect method of conjugation except that a therapeutic agent is directly attached to an oxidized antibody component.

It will be appreciated that other therapeutic agents can be substituted for the chelators described herein. Those of skill in the art will be able to devise conjugation schemes without undue experimentation.

As a further illustration, a therapeutic agent can be attached at the hinge region of a reduced antibody component via disulfide bond formation. For example, the tetanus toxoid peptides can be constructed with a single cysteine residue that is used to attach the peptide to an antibody component. As an alternative, such peptides can be attached to the antibody component using a heterobifunctional cross-linker, such as N-succinyl 3-(2-pyridyldithio)proprionate (SPDP). Yu et al., Int. J. Cancer56:244 (1994). General techniques for such conjugation are well-known in the art. See, for example, Wong, Chemistry Of Protein Conjugation and Cross-Linking (CRC Press 1991); Upeslacis et al., "Modification of Antibodies by Chemical Methods," in Monoclonal Antibodies: Principles and Applications, Birch et al. (eds.), pages 187-230 (Wiley-Liss, Inc. 1995); Price, "Production and Characterization of Synthetic Peptide-Derived Antibodies," in Monoclonal Antibodies: Production, Enineering and Clinical Application, Ritter et al. (eds.), pages 60-84 (Cambridge University Press 1995).

As described above, carbohydrate moieties in the Fc region of an antibody can be used to conjugate a therapeutic agent. However, the Fc region may be absent if an antibody fragment is used as the antibody component of the immunoconjugate. Nevertheless, it is possible to introduce a carbohydrate moiety into the light chain variable region of an antibody or antibody fragment. See, for example, Leung et al., J. Immunol. 154:5919 (1995); Hansen et al., U.S. Pat. No. 5,443,953. The engineered carbohydrate moiety is then used to attach a therapeutic agent.

In addition, those of skill in the art will recognize numerous possible variations of the conjugation methods. For example, the carbohydrate moiety can be used to attach polyethyleneglycol in order to extend the half-life of an intact antibody, or antigen-binding fragment thereof, in blood, lymph, or other extracellular fluids. Moreover, it is possible to construct a "divalent immunoconjugate" by attaching therapeutic agents to a carbohydrate moiety and to a free sulfhydryl group. Such a free sulfhydryl group may be located in the hinge region of the antibody component.

### Anti-M-CSF and Anti-M-CSFR Antibody Fusion Proteins

The present invention contemplates the use of fusion proteins comprising one or more anti-M-CSF and anti-M-CSFR antibody moieties and an immunomodulator or toxin moiety. Methods of making antibody fusion proteins are well known in the art. See, *e.g.,* U.S. Patent No. 6,306,393. Antibody fusion proteins comprising an interleukin-2 moiety are described by Boleti et al., Ann. Oncol. 6:945 (1995), Nicolet et al., Cancer Gene Ther. 2:161 (1995), Becker et al., Proc. Nat'l Acad. Sci. USA 93:7826 (1996), Hank et al., Clin. Cancer Res. 2:1951 (1996), and Hu et al., Cancer Res. 56:4998 (1996). In addition, Yang et al., Hum. Antibodies Hybridomas 6:129 (1995), describe a fusion protein that includes an F(ab')₂ fragment and a tumor necrosis factor alpha moiety.

Methods of making antibody-toxin fusion proteins in which a recombinant molecule comprises one or more antibody components and a toxin or chemotherapeutic agent also are known to those of skill in the art. For example, antibody-*Pseudomonas* exotoxin A fusion proteins have been described by Chaudhary et al., Nature 339:394 (1989), Brinkmann et al., Proc. Nat'l Acad. Sci. USA 88:8616 (1991), Batra et al., Proc. Nat'l Acad. Sci. USA 89:5867 (1992), Friedman et al., J. Immunol. 150:3054 (1993), Wels et al., Int. J. Can. 60:137 (1995), Fominaya et al., J. Biol. Chem. 271:10560 (1996), Kuan et al., Biochemistry 35:2872 (1996), and Schmidt et al., Int. J. Can. 65:538 (1996). Antibody-toxin fusion proteins containing a diphtheria toxin moiety have been described by Kreitman et al., Leukemia 7:553 (1993), Nicholls et al., J. Biol. Chem. 268:5302 (1993), Thompson et al., J. Biol. Chem. 270:28037 (1995), and Vallera et al., Blood 88:2342 (1996). Deonarain et al., Tumor Targeting 1:177 (1995), have described an antibody-toxin fusion protein having an RNase moiety, while Linardou et al., Cell Biophys. 24-25:243 (1994), produced an antibody-toxin fusion protein comprising a DNase I component. Gelonin was used as the toxin moiety in the antibody-toxin fusion protein of Wang et al., Abstracts of the 209th ACS National Meeting, Anaheim, Calif., Apr. 2-6, 1995, Part 1, BIOT005. As a further example, Dohlsten et al., Proc. Nat'l Acad. Sci. USA 91:8945 (1994), reported an antibody-toxin fusion protein comprising *Staphylococcal* enterotoxin-A.

Illustrative of toxins which are suitably employed in the preparation of such conjugates are ricin, abrin, ribonuclease, DNase I, Staphylococcal enterotoxin-A, pokeweed antiviral protein, gelonin, diphtherin toxin, Pseudomonas exotoxin, and Pseudomonas endotoxin. See, for example, Pastan et al., Cell 47:641 (1986), and Goldenberg, CA--A Cancer Journal for Clinicians 44:43 (1994). Other suitable toxins are known to those of skill in the art.

The invention is illustrated by the following examples, which are not intended to be limiting in any way.

### EXAMPLES

### EXAMPLE 1

This example shows that highly metastatic breast cancer cell lines express high levels of M-CSF. Using microarrays, the M-CSF gene expression by the highly metastatic cell line, MDA 231, was compared with that of the cell lines MCF7 and ZR751. There was a 6.9 fold increase when the M-CSF expression level in MDA 231 was compared with that in MCF7, and a 5.2 fold increase when the M-CSF expression level in MDA 231 was compared with that in ZR751.

### EXAMPLE 2

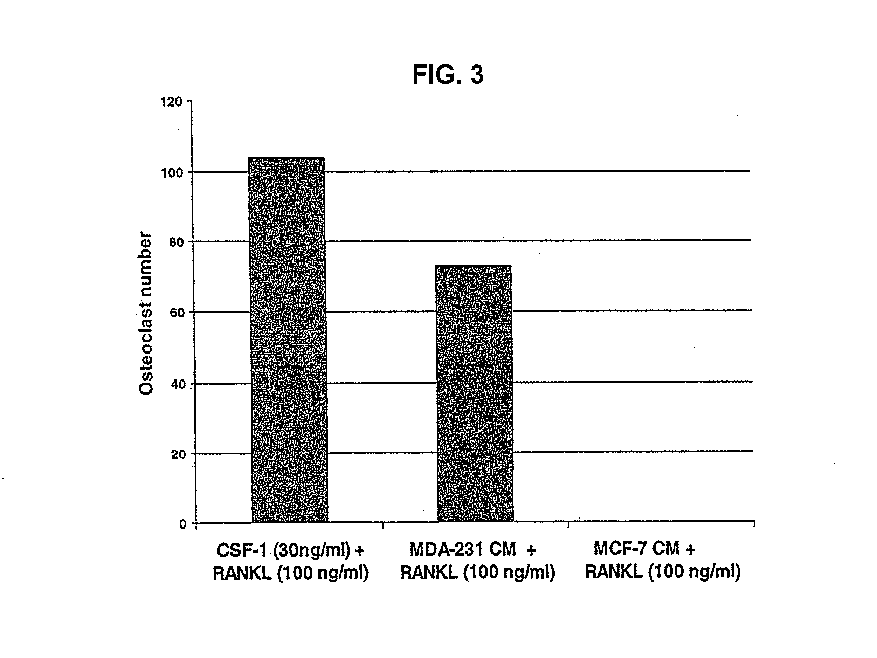

This example shows that purified M-CSF can be replaced by conditioned media (CM) from the metastatic cell line MDA 231 but not from the cell line MCF7 in *in vitro* assays of osteoclast formation (Figure 3).

Production of conditioned media (CM): MDA 231 or MCF7 cells were plated at a density of 1 x 10⁶ cells/10 cm dish in 8 mls of 50% DMEM/ 50% HAMs F12 containing 1 x ITS, (BD Biosciences, Lexington, Ky), a culture supplement containing insulin, human transferrin, and selenous acid. After 48 hours of incubation at 37°C in 5% CO₂, the media were collected and centrifuged for 10 minutes at 1500 RPM to remove any suspended cells. The supernatant was collected, filtered through a 0.2 nM filter, and used as CM.

Osteoclast assay: Bone marrow CD34⁺ cells were plated at a density of 15,000 cells/ 96 well in 100 µl of Alpha MEM containing 10% FCS, 1 X Pen/Strep and 1 x fungizone. The next day, 50 µl of media was removed from each well and replaced with 25 µl of Alpha MEM media and 75 µl of CM or 50% DMEM/ 50% HAMs F12 containing 1 x ITS. RANKL was added to each well at a final concentration of 100 ng/ml and 30 ng/ml M-CSF was added to the appropriate wells. The cells were incubated at 37°C in 5% CO₂ for 11 days. During that time fresh RANKL was added again after 6 days. After 11 days the cells were fixed and stained for tartrate resistant acid phosphatase using the Leukocyte acid phosphatase kit from Sigma.

Results: As shown in Figure 3, purified M-CSF can be replaced by conditioned media (CM) from the metastatic cell line MDA 231 but not from the cell line MCF7 in *in vitro* assays of osteoclast formation.

### EXAMPLE 3

This example shows that osteoclast induction by MDA 231 CM is neutralized by antibodies to M-CSF (Figure 4).

Bone marrow CD34⁺ cells were plated as described in Example 2. The next day 50 µl of media was removed from each well. 25 µl of 6x antibody 5H4 or Alpha MEM media was added to each well followed by 75 µl of CM or 50% DMEM/50% HAMs F12 containing 1 x ITS or alpha MEM media. 100 ng/ml RANKL was added to all wells, and 30 ng/ml M-CSF was added to half of the wells. The cells were incubated at 37°C in 5% CO₂ for 11 days. During that time fresh RANKL was added again after 6 days. After 11 days, the cells were fixed and stained for tartrate resistant acid phosphatase using the Leukocyte acid phosphatase kit from Sigma.

As shown in Figure 4, osteoclast induction by MDA 231 CM is neutralized by antibodies to M-CSF.

### EXAMPLE 4

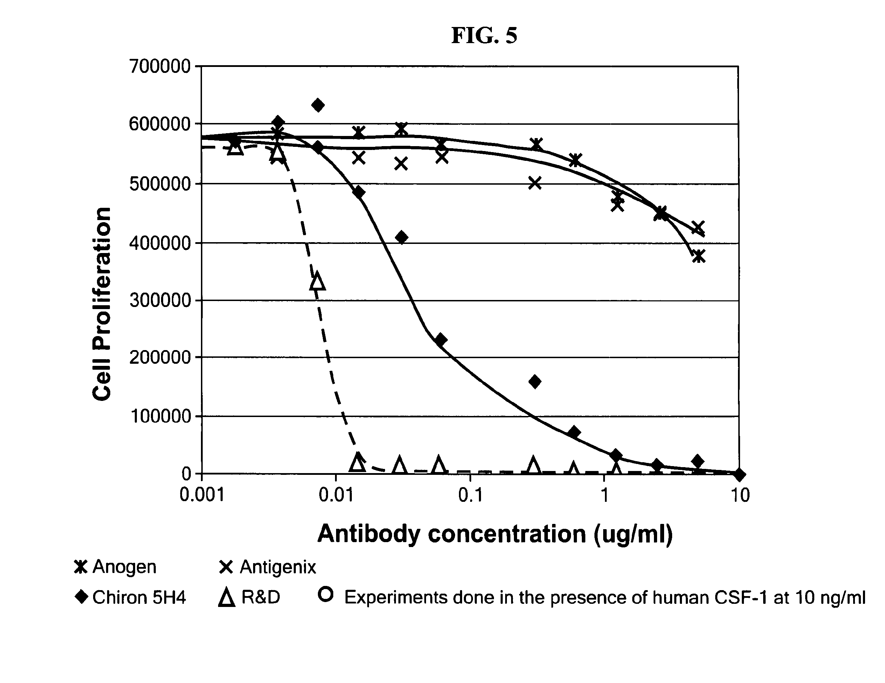

This example shows neutralizing activity of a monoclonal antibody 5H4 (American Type Culture Collection Accession No. HB10027) and other antibodies against human M-CSF (Figure 5).

To measure the neutralizing ability of the antibodies against the activity of human M-CSF on murine M NFS 60 cells (American Type Culture Collection Accession No. CRL-1838, available from ATCC in Rockville, MD, USA, derived from a myelogenous leukemia induced with the Cas-Br-MuLV wild mouse ecotropic retrovirous, responsive to both interleukin 3 and M-CSF and which contain a truncated c-myb proto-oncogene caused by the integration of a retrovirus), recombinant human CSF-1 (at 10 ng/ml final concentration) was incubated with various concentrations of antibodies for 1 hour at 37°C in 5% CO₂ in an incubator. Following the incubation, the mixture was added to the M NFS 60 culture in 96 well microtiter plates. The total assay volume per well was 100µl, with 10 ng/ml rhM-CSF, the indicated antibody concentration according to Figure 5, and cell density at 5,000 cells/well. After 72 hours culture in a CO₂ incubator at 37°C, cell proliferation was assayed by CeIITiter Glo Kit (Promega). All antibodies were raised against human M-CSF. Anogen refers to Anogen product catalog #MO C40048.A clone 116; Antigenix refers to Antigenix America product catalog #MC600520 clone M16; and R&D refers to R&D Systems product catalog #Mab216.

As shown in Figure 5, cell proliferation was most affected by antibody 5H4 treatment versus Anogen and Antigenix.

### EXAMPLE 5

This example shows that anti-M-CSF antibodies are highly effective in preventing severe osteolytic disease associated with cancer metastasis.

Experimental Design. To evaluate the efficacy of M-CSF antibodies as a therapeutic agent for the treatment of osteolysis, the highly metastatic human breast cancer cell line MDA-231 (3 x 10⁵) was injected into the tibia bone marrow cavity of female nude mice. The mice were at the age of 4-7 weeks old, and had an average weight of ∼20g. The mice were chipped for identification and underwent an acclimation period of at least 7 days prior to the start of the 8-week study.

The mice received a total dose of 1.5mpk (0.03mg per mouse) Buprenorphine subcutaneously at both flank 30 minutes before intra-tibia injection. Mice were anesthetized by isoflurane inhalation and the right hind leg was cleaned with 70% ethanol. Tumor cells (MDA-MB-231-luc, 3x10⁵) suspended in 10 µl of saline was injected into the right tibia bone marrow cavity using 50 or 100 µl micro-syringe.

For antibody treatment, groups were chosen as follows:
1. murine IgG1 isotype control
2. 5H4 murine IgG1 anti-human MCSF
3. rat monoclonal control (TBD)
4. 5A1 rat IgG1 anti-mouse MCSF
5. 5H4 + 5A1 (double treatment)
6. murine + rat isotype controls (double control)
   (no PBS control group)

Dosing. Treatment with antibody began the day following the injection of the tumor cells. For the murine IgG1 isotype control, 5H4 murine IgG1 anti-human MCSF, rat monoclonal control, and 5A1 rat IgG1 anti-mouse MCSF antibodies (Lokeshwar, B. L., Lin, H. S., J Immunol., 15;141(2):483-8 (1988), 10 mg/kg antibody was administered once per week. For the combination treatment group (5H4 + 5A1, murine + rat isotype controls), 10 mg/kg of each individual (i.e., unmixed) antibody was administered once per week at separate injections such that mice in these treatment groups received 2 injections per week.

The dosing solutions were provided at a pre-diluted concentration (=2 mg/ml) such that 100 uL injected IP will deliver the target dose for a 20 gram mouse (=200 µg). For weight adjustment, the volume injected was increased or decreased by 5 µl per gram of weight difference. For example, a 23 gram mouse received 115 µl, while an 18 gram mouse received 90 µl.

Measurements. To assess the severity of osteolysis among the various treatment groups, each mouse received a baseline Faxitron image taken the day following injection of tumor cells. A Faxitron image was taken at the end of the study (8 weeks). Tumor growth was simultaneously measured using the Xenogen system since the tumor cells stably expressed luciferase.

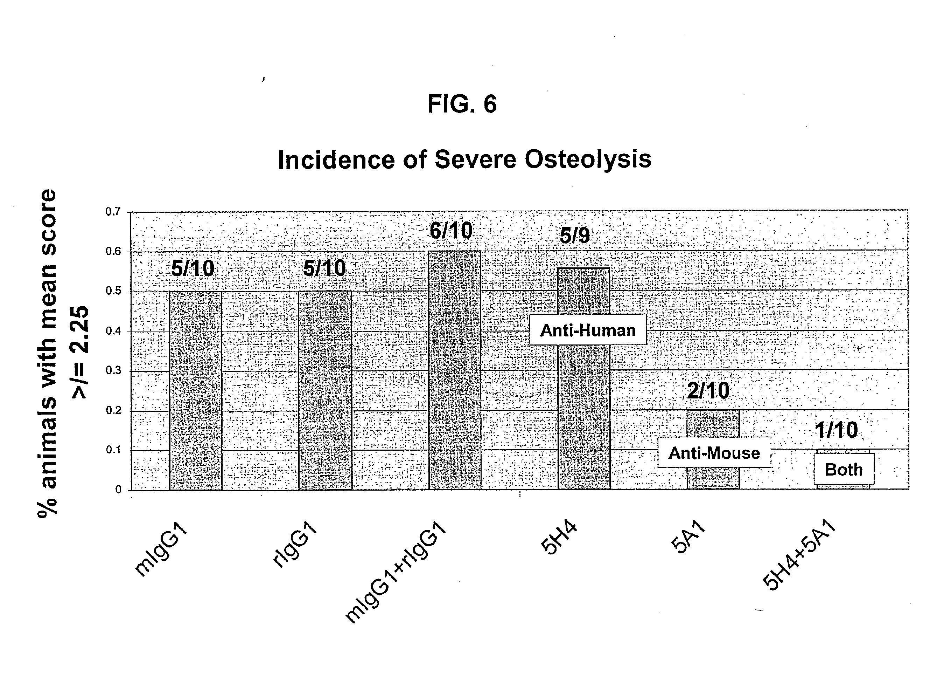

Results. As shown in Figure 6, the number of animals with a mean osteolysis score of ≥ 2.5 was lowest in the group that received the combination of 5A1 + 5H4 antibodies. Osteolytic bone damage was evaluated on a scale from 0-4, with 0 equal to no bone damage; 1-2 equal to some bone damage, such that scores of 2.25 or greater was indicative of severe bone damage. The data indicated that the M-CSF produced by the mouse (i.e. host) has a greater impact on osteolysis than the M-CSF produced by the tumor cells (compare 5H4 to 5A1 individually, and in combination).

### EXAMPLE 6

The study described in Example 5 above was repeated essentially as described with one exception in the experimental design. For this study, antibody treatment did not commence until 2 weeks following tumor inoculation. Radiograms of the hind legs were taken one day after tumor inoculation for getting baseline image and checking for bone fracture caused by injection. Further, on day 14 after tumor inoculation, mice were imaged by Xenogen IVIS system to quantitate the photon emission of fluorescent light from tumor cells.

Sixty mice were chosen with similar amount of tibial photon signal. The mice with chest signals (artificial lung metastases) were excluded from this study. Selected mice were randomly grouped into the 6 treatment groups described and administered antibody treatments as described in Example 5. Photon emission was continuously monitored once per week throughout the study. From the 5^{th} week after tumor inoculation, radiograms were also be taken once per week to assess the tumor growth in the bone.

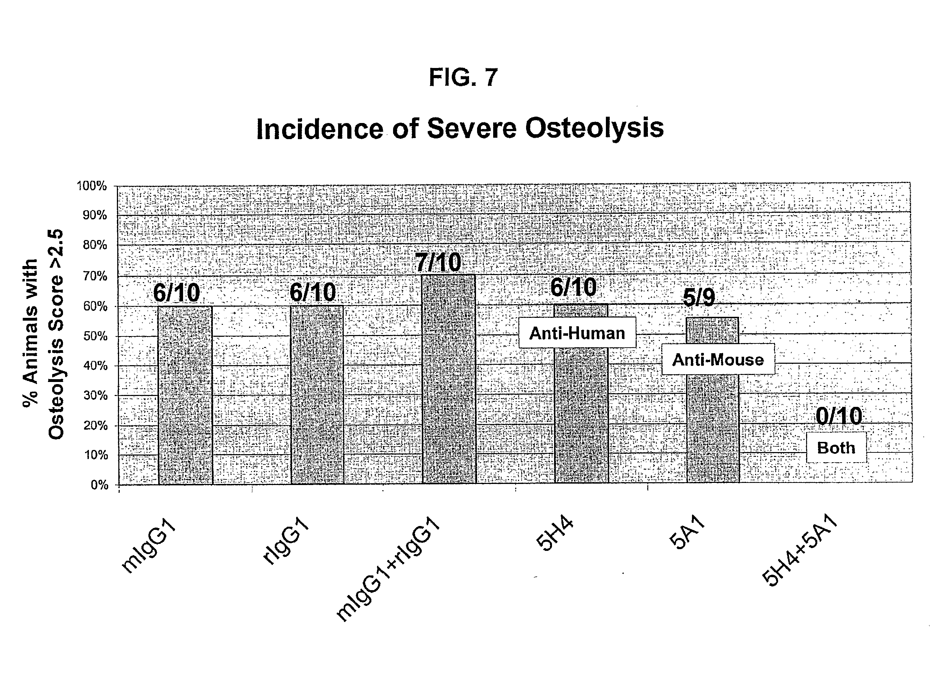

As shown in Figure 7, the number of animals with a mean osteolysis score of ≥ 2.5 was lowest in the group that received the combination of 5A1 + 5H4 antibodies Osteolytic bone damage was evaluated on a scale from 0-4, with 0 equal to no bone damage; 1-2 equal to some bone damage, such that scores of 2.25 or greater was indicative of severe bone damage. The data indicated that the M-CSF produced by the mouse (i.e. host) has a greater impact on osteolysis than the M-CSF produced by the tumor cells (compare 5H4 to 5A1 individually, and in combination).

### EXAMPLE 7

This example sets out a procedure for the evaluation of the anti-cancer activity of anti-M-CSF monoclonal antibody in a subcutaneous SW620 model. Examples 5 and 6 above showed that anti-M-CSF monoclonal antibody treatment significantly inhibited the tumor growth in bone marrow. The purpose of this study is to evaluate whether the antibody can also inhibit the tumor growth in soft tissue.

Female nu/nu mice at the age of 10 weeks old, average weight ∼20g will be used for this study. Mice will undergo an acclimation period of at least 7 days prior to study start. On day 0, the right flank of nude mice will be injected with SW620 human colon cancer cells subcutaneously at 5x10⁶ cells per mouse per 100ul. When tumor volume reaches 100-200 mm³ (usually 1 week after tumor inoculation), mice will be randomized into 5 groups at 10 mice per group as follows:
1) PBS
2) 5H4
3) 5A1
4) mIgG1+rIgG1 isotype Ab control
5) 5A1+5H4

Mice will be treated intraperitoneally with the designated antibodies at 10mpk once a week for 4 weeks. When tumor volume reaches 2000 mm³, the study will be terminated. Alternatively, animals will also be euthanized when any of the following situations are met: tumor surface ulceration is bigger than 30% of total tumor surface area, significant body weight loss (>20%), dehydration, and moribund. Whole blood will be collected from all of the mice and monocyte population will be analyzed as a potential surrogate marker. Tumor growth/size will be measured by 2-D analysis. Measurements of tumor width and length will be used to calculate tumor volume. It is expected that tumor growth in soft tissue will be inhibted as a result of the foregoing experiment.

The following example sets out a procedure for the evaluation of combination therapy for the treatment and prevention severe osteolytic disease associated with cancer metastasis.

Experimental Design. The study described in Example 5 above is repeated essentially as described with the following exceptions. In addition to the antibody or antibody combination set out in the treatment groups below, the animals will receive one of the following additional treatments:
1. Bisphosphonate (e.g., Aredia; Zometa; Clodronate).
2. Surgery
3. Radiation
4. Chemotherapy
5. Hormone therapy (e.g., Tamoxifen; anti-Androgen therapy)
6. Antibody therapy (e.g., RANKL/RANK neutralizing antibodies; PTHrP neutralizing antibody)
7. Therapeutic protein therapy (e.g., soluble RANKL receptor; OPG, and PDGF and MMP inhibitors)
8. Small molecule drug therapy (e.g., Src-kinase inhibitor)
9. Oligonucleotides therapy (e.g., RANKL or RANK or PTHrP Anti-sense)
10. Gene therapy (e.g., RANKL or RANK inhibitors)
11. Peptide therapy (e.g. muteins of RANKL)

The treatment groups are as follows. The above additional treatments are indicated below as "plus therapy X":
1. PBS only
2. treatment with therapy X only
3. rat IgG1 isotype control
4. murine IG1 isotype control
5. 5H4 anti-human MCSF only
6. 5A1 rat IgG1 anti-mouse MCSF only
7. rat IgG1 and murine IgG1 isotype control combination
8. 5H4 an 5A1 combination
9. rat IgG1 isotype control plus therapy X
10. murine IG1 isotype control plus therapy X
11. 5H4 anti-human MCSF plus therapy X
12. 5A1 rat IgG1 anti-mouse MCSF plus therapy X
13. rat IgG1 and murine IgG1 isotype control combination plus therapy X
14. 5H4 an 5A1 combination plus therapy X

Dosing: 0.1-30 mg/kg each antibody is used for administration to each animal. Preferred dosing is 10 mg/kg. The administration route can be IV, IP, SC. The preferred route is IP. Treatment will begin the day following injection of tumor cells, as described in Example 5, above.

Measurements. To assess the severity of osteolysis among the various treatment groups, each mouse receives a baseline Faxitron image taken the day following injection of tumor cells. A Faxitron image is also taken at the end of the study (8 weeks). Tumor growth is simultaneously measured using the Xenogen system since the tumor cells stably express luciferase. It is expected that combination therapy for the treatment and prevention severe osteolytic disease associated with cancer metastasis will be improved with relative to antibody therapy alone.

### EXAMPLE 9

The following example provides a protocol for evaluating the ability of M-CSF-specific antibodies to bind to, for example, breast cancer cells (cell line MDA231) or multiple myeloma cancer cells (cell line ARH77) using a fluorescence-activated cell sorter.

The cells were first washed twice with PBS (no Ca²⁺, Mg²⁺). For each 10-cm plate, 2ml of 3 mM EDTA was added, and the plates were incubated at 37 °C for 2-3 minutes, until the cells were rounded and began to detach from the dish. Next, 10 ml of buffer A (PBS + 5% FBS) was added and mixed. At that time, the cells were pelleted and resuspended at about 5x10⁶ cells/ml in PBS+5% FBS, and the cells were placed into tubules at 100 µl/sample.

At this point, 0.1-10 ug/ml of the primary antibody (used at indicated concentration of M-CSF antibody or control antibody) was added. Dilution, if necessary, was made in 5% FBS/PBS. The mixture was then incubated for 30 min at 4 °C. Following the incubation period, the cells were washed 3 times by centrifugation at 400 g for 5 min., and the cells were resuspended in PBS.

The FITC or PE-labeld anti-IgG antibody (0.25 ug/sample) was diluted in 1% BSA/PBS at the optimal dilution, and the cells were resuspended in this solution and incubated for 30 min at 4 °C. Next, the cells were washed 3 times as described above. Following th cell washes, the cells were resuspended with 0.5 ml/sample PI-PBS (if necessary to distinguish dead cells from live ones). The cells can also be fixed for later analysis (the cells can last about 3 days if they are fixed with 0.1% formaldehyde). The cells were next analyzed in a fluorescence-active FACS using standard procedures.

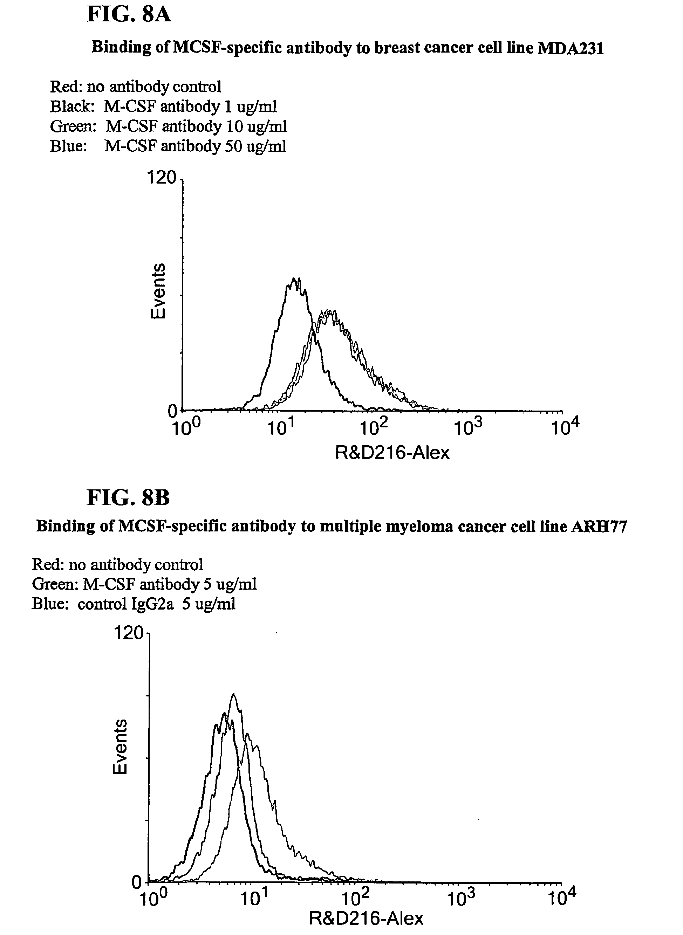

As shown in Figure 8A and 8B, an MCSF-specific antibody bound to breast cancer cell line MDA231 or to multiple myeloma cancer cell line ARH77 at a variety of antibody concentrations as indicated.

### EXAMPLE 10

The following example shows M-CSF is prevalent on a number of cancer cell surfaces. Immunohistochemical staining of M-CSF using a M-CSF-specific antibody was carried out as follows.

At the outset, slides were heated in an oven at 55 - 60°C for 1 hour and allowed to cool for 2-3 minutes. The following de-waxing and re-hydration parameters were used:

| | |
|---|---|
| a. Xylene | 3 x 5 minutes |
| b. 100% Reagent Alcohol | 2 x 5 minutes |
| c. 95% Reagent Alcohol | 2 x 4 minutes |
| d. 75% Reagent Alcohol | 2 x 3 minutes |
| e. 50% Reagent Alcohol | 1 x 3minutes |
| g. dI H2O | 2 - 3 quick rinses |

Prior to the peroxide blocking step, antigen retrieval was prepared using 1 x Biogenex Citra Plus. The solution was initially microwaved at full power to boil. Once the solution boiled, the microwave was quickly set for another 13 min at power-level 2, and allowed to cool before proceeding. The peroxide blocking step was performed as follows. The slides were immersed slides in 3% H2O2 (25ml 30% to 250ml dI H2O) and placed at room temperature for 10 minutes. The slides were next rinsed 2x with dI H2O, and washed with 1 X PBS 2 x 2 minutes.

The avidin/biotin blocking procedure was performed as follows. Slides were placed flat on a metal rack. A Blue PAP pen was used (hydrophobic slide marker) around tissue. Next, 2 drops Zymed Avidin (Reagent A)---enough to cover tissue--was added and the slides were incuabated at room temperature for 10 min. Following the incubation, , the slides were washed as follows:
2 x 3 minute washes in 1 X PBS.
2 drops Zymed Biotin (Reagent B), room temperature for 10 min.
2 x 3 minute washes in 1 X PBS.

The protein blocking procedure was performed as follows. First, 10% serum [to 2% final concentration] of secondary antibody species was added. The BioGenex Power Block was next diluted to 1 X with dI H2O. The rack of slides was immersed in Power Block for 8 min at room temperature, and the slides were rinsed 2x in 1X PBS.

For the addition of the primary antibody, the slides were placed flat on a metal rack. Antibody was added to cover each section (∼350µl), and the antibody was spread with pipet tip (if necessary) without scraping tissue. The slides were then incubated for 1 hour at room temperature. Following the incubation, the slides were washed 3 x with 1 x PBS 3-5 minutes each time. At this point, BioGenex Multi-Link was applied to sections & incubated for 10-11 minutes at room temperature. The sections were then washed 3 minutes each time.

Labelling was performed by applying BioGenex HRP Label to sections, which were then incubated at room temperature for 10-11 min and washed with 1 x PBS 3 x 3 minutes. Next, BioGenex H₂O₂ substrate was added (1 drop AEC for every 2.5 ml H2O2) to the sections and incubated at room temperature for 10 min. The sections were then rinsed several times with dI H₂O. The counterstaining step was performed as follows. The sections were stained with hematoxylin for 1 minute at room temperature. Next, the sections were rinse with H₂O twice, and then incubated in 1 X PBS for 1 minute. Sections were then rinsed well with H₂O to remove PBS. Sections were mounted by applying a drop of BioGenex Super Mount to the section section and then air drying over night at room temperature.

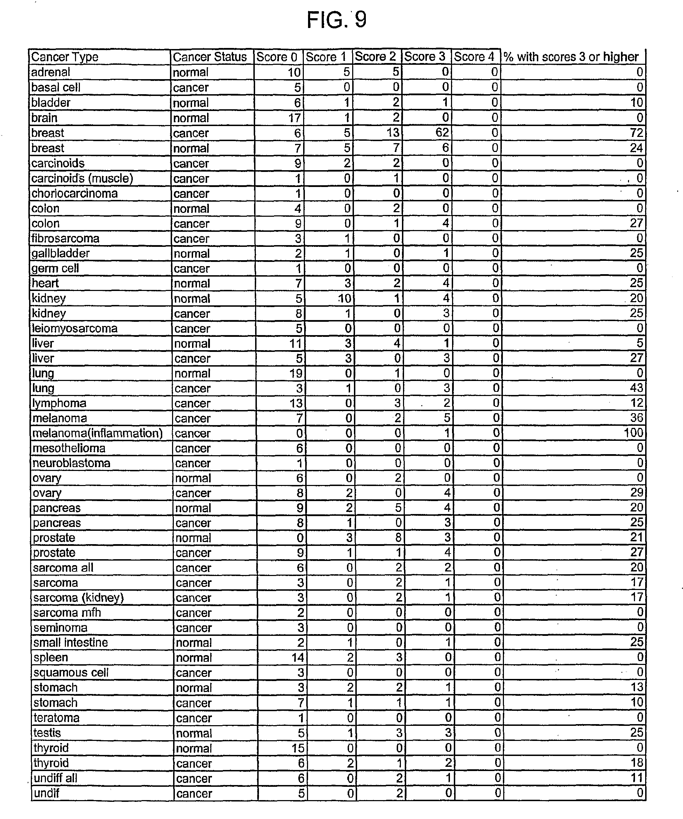

As shown in Figure 9, M-CSF is prevalent on a number of cancer cell surfaces. Sections for the indicated cancer cell types were scored as follows:
0 No staining
1 Staining was similar to background
2 Positive, but weak staining
3 Positive and significant staining
4 Positive and strong staining

## Claims

1. An M-CSF antagonist, for use in:
a) preventing bone metastases in a subject afflicted with metastatic cancer;
b) preventing, in a subject afflicted with metastatic cancer, bone loss associated with the cancer;
c) treating a subject afflicted with a metastatic cancer to bone,
d) reducing, in a subject afflicted with a metastatic cancer to bone, the severity of bone loss associated with the cancer,
e) treating a subject suffering from osteolytic bone metastases, or
f) treating a subject suffering from bone loss associated with cancer metastasis,
wherein said antagonist inhibits the interaction between M-CSF and its receptor (M-CSFR) and is selected from the group consisting of:
i) a polypeptide comprising an anti-M-CSF antibody; and
ii) a polypeptide comprising an anti-M-CSFR antibody.

2. The M-CSF antagonist for use according to claim 1 wherein said M-CSF antagonist is an anti-M-CSF antibody.

3. The M-CSF antagonist for use according to claim 1 wherein said M-CSF antagonist is a polypeptide comprising an anti-M-CSFR antibody.

4. The M-CSF antagonist for use according to any preceding claim, wherein said antagonist inhibits osteoclast proliferation and/or differentiation induced by tumor cells.

5. The M-CSF antagonist for use according to any preceding claim, wherein said antibody is selected from the group consisting of: a) a polyclonal antibody; b) a monoclonal antibody; c) a humanized antibody; d) a human antibody; e) a chimeric antibody; and f) Fab, F(ab') 2 or Fv antibody fragment.

6. The monoclonal antibody for use according to claim 5, wherein said antibody is **characterised by** a dissociation equilibrium constant (Kd) of 10⁻⁸M to 10⁻¹²M.

7. The M-CSF antagonist for use according to claim 2, wherein said antagonist is an antibody that binds to the same epitope as monoclonal antibody 5H4 having ATCC Accession No.HB 10027.

8. The M-CSF antagonist for use according to any of claims 1-7, wherein the metastatic cancer is breast, lung, renal, multiple myeloma, thyroid, prostate, adenocarcinoma, blood cell malignancies, including leukemia and lymphoma; head and neck cancers; gastrointestinal cancers, including stomach cancer, colon cancer, colorectal cancer, pancreatic cancer, liver cancer; malignancies of the female genital tract, including ovarian carcinoma, uterine endometrial cancers and cervical cancer; bladder cancer; brain cancer, including neuroblastoma; sarcoma, osteosarcoma; and skin cancer, including malignant melanoma or squamous cell cancer.

9. The M-CSF antagonist for use according to any preceding claim, wherein the M-CSF antagonist is administered at a dose between 0.01 mg/kg and 100mg/kg.

10. The M-CSF antagonist for use according to any one of claims 1-4, wherein said antagonist is a fully human antibody.

11. The M-CSF antagonist for use according to any one of claims 1-4, wherein said antagonist is a humanized antibody.

12. The M-CSF antagonist for use according to any preceding claim, wherein said antibody is conjugated to a radionuclide or other toxin.

13. The M-CSF antagonist for use according to any preceding claim wherein said subject is a mammal.

14. The M-CSF antagonist for use according to claim 14, wherein said subject is human.

15. The M-CSF antagonist for use according to any preceding claim, wherein the antagonist is to be used in combination with another therapeutic agent.

## Patentansprüche

1. M-CSF-Antagonist, zur Verwendung beim:
a) Vorbeugen von Knochenmetastasen bei einem an einer metastasierenden Krebserkrankung laborierenden Patienten;
b) Vorbeugen, bei einem an einer metastasierenden Krebserkrankung laborierenden Patienten, von mit der Krebserkrankung zusammenhängendem Knochenschwund;
c) Behandeln eines an einer in den Knochen metastasierenden Krebserkrankung laborierenden Patienten,
d) Verringern, bei einem an einer in den Knochen metastasierenden Krebserkrankung laborierenden Patienten, der Schwere von mit der Krebserkrankung zusammenhängendem Knochenschwund,
e) Behandeln eines an osteolytischen Knochenmetastasen leidenden Patienten oder
f) Behandeln eines an mit Krebsmetastasierung zusammenhängendem Knochenschwund leidenden Patienten,
wobei der Antagonist die Wechselwirkung zwischen M-CSF und seinem Rezeptor (M-CSFR) hemmt und aus der aus:
i) einem einen Anti-M-CSF-Antikörper umfassenden Polypeptid; und
ii) einem einen Anti-M-CSFR-Antikörper umfassenden Polypeptid
bestehenden Gruppe ausgewählt ist.

2. M-CSF-Antagonist zur Verwendung nach Anspruch 1, wobei es sich bei dem M-CSF-Antagonisten um einen Anti-M-CSF-Antikörper handelt.

3. M-CSF-Antagonist zur Verwendung nach Anspruch 1, wobei es sich bei dem M-CSF-Antagonisten um ein einen Anti-M-CSFR-Antikörper umfassendes Polypeptid handelt.

4. M-CSF-Antagonist zur Verwendung nach einem vorhergehenden Anspruch, wobei der Antagonist durch Tumorzellen induzierte Osteoklasten-Proliferation und/oder -Differenzierung hemmt.

5. M-CSF-Antagonist zur Verwendung nach einem vorhergehenden Anspruch, wobei der Antikörper aus der aus: a) einem polyklonalen Antikörper; b) einem monoklonalen Antikörper; c) einem humanisierten Antikörper; d) einem menschlichen Antikörper; e) einem chimären Antikörper; und f) dem Fab-, F(ab')2- oder Fv-Antikörperfragment bestehenden Gruppe ausgewählt ist.

6. Monoklonaler Antikörper zur Verwendung nach Anspruch 5, wobei der Antikörper durch eine Dissoziationsgleichgewichtskonstante (Kd) von 10⁻⁸ M bis 10⁻¹² M gekennzeichnet ist.

7. M-CSF-Antagonist zur Verwendung nach Anspruch 2, wobei es sich bei dem Antagonisten um einen Antikörper handelt, der an das gleiche Epitop wie monoklonaler Antikörper 5H4 mit ATCC Accession No.HB 10027 bindet.

8. M-CSF-Antagonist zur Verwendung nach einem der Ansprüche 1-7, wobei es sich bei der metastasierenden Krebserkrankung um Brustkrebs, Lungenkrebs, Nierenkrebs, multiples Myelom, Schilddrüsenkrebs, Prostatakrebs, Adenokarzinom, Blutzellenmalignitäten, einschließlich Leukämie und Lymphom; Krebserkrankungen des Kopf- und Halsbereichs; Krebserkrankungen des Magen-Darm-Trakts, einschließlich Magenkrebs, Kolonkarzinom, Kolorektalkarzinom, Bauchspeicheldrüsenkrebs, Leberkrebs; Malignitäten des weiblichen Genitaltrakts, einschließlich Ovarialkarzinom, Gebärmutterendometriumkarzinome und Gebärmutterhalskrebs; Blasenkrebs; Hirntumore, einschließlich Neuroblastom; Sarkom, Osteosarkom; und Hautkrebs, einschließlich malignem Melanom oder Plattenepithelkarzinom, handelt.

9. M-CSF-Antagonist zur Verwendung nach einem vorhergehenden Anspruch, wobei der M-CSF-Antagonist in einer Dosis zwischen 0,01 mg/kg und 100 mg/kg verabreicht wird.

10. M-CSF-Antagonist zur Verwendung nach einem der Ansprüche 1-4, wobei es sich bei dem Antagonisten um einen komplett menschlichen Antikörper handelt.

11. M-CSF-Antagonist zur Verwendung nach einem der Ansprüche 1-4, wobei es sich bei dem Antagonisten um einen humanisierten Antikörper handelt.

12. M-CSF-Antagonist zur Verwendung nach einem vorhergehenden Anspruch, wobei der Antikörper an ein Radionuklid oder anderes Toxin konjugiert ist.

13. M-CSF-Antagonist zur Verwendung nach einem vorhergehenden Anspruch, wobei es sich bei dem Patienten um einen Säuger handelt.

14. M-CSF-Antagonist zur Verwendung nach Anspruch 13, wobei es sich um einen menschlichen Patienten handelt.

15. M-CSF-Antagonist zur Verwendung nach einem vorhergehenden Anspruch, wobei der Antagonist in Kombination mit einem weiteren Therapeutikum zu verwenden ist.

## Revendications

1. Antagoniste de M-CSF, pour utilisation dans :
a) la prévention des métastases osseuses chez un sujet souffrant d'un cancer métastatique ;
b) la prévention des pertes osseuses associées au cancer chez un sujet souffrant d'un cancer métastatique ;
c) le traitement d'un sujet souffrant d'un cancer métastatique de l'os,
d) la diminution, chez un sujet souffrant d'un cancer métastatique de l'os, de la sévérité des pertes osseuses associées au cancer,
e) le traitement d'un sujet souffrant de métastases osseuses ostéolytiques, ou
f) le traitement d'un sujet souffrant de pertes osseuses associées à une métastase cancéreuse, où ledit antagoniste inhibe l'interaction entre M-CSF et son récepteur (M-CSFR) et est choisi dans le groupe constitué par les suivants :
i) un polypeptide comprenant un anticorps anti-M-CSF ; et
ii) un polypeptide comprenant un anticorps anti-M-CSFR.

2. Antagoniste de M-CSF pour utilisation selon la revendication 1, où ledit antagoniste de M-CSF est un anticorps anti-M-CSF.

3. Antagoniste de M-CSF pour utilisation selon la revendication 1, où ledit antagoniste de M-CSF est un polypeptide comprenant un anticorps anti-M-CSFR.

4. Antagoniste de M-CSF pour utilisation selon l'une quelconque des revendications précédentes, où ledit antagoniste inhibe la prolifération et/ou la différenciation des ostéoclastes induites par les cellules tumorales.

5. Antagoniste de M-CSF pour utilisation selon l'une quelconque des revendications précédentes, où ledit anticorps est choisi dans le groupe constitué par les suivants : a) un anticorps polyclonal ; b) un anticorps monoclonal ; c) un anticorps humanisé ; d) un anticorps humain ; e) un anticorps chimérique ; et f) un fragment d'anticorps Fab, F(ab') 2 ou Fv.

6. Anticorps monoclonal pour utilisation selon la revendication 5, où ledit anticorps est **caractérisé par** une constante d'équilibre de dissociation (Kd) comprise entre 10⁻⁸M et 10⁻¹²M.

7. Antagoniste de M-CSF pour utilisation selon la revendication 2, où ledit antagoniste est un anticorps qui se lie au même épitope que l'anticorps monoclonal 5H4 présentant le n° d'accès ATCC HB 10027.

8. Antagoniste de M-CSF pour utilisation selon l'une quelconque des revendications 1 à 7, où le cancer métastatique est un cancer du sein, du poumon, du rein, un myélome multiple, un cancer de la thyroïde, de la prostate, un adénocarcinome, une tumeur maligne des cellules sanguines, y compris la leucémie et les lymphomes ; un cancer de la tête et du cou ; un cancer gastro-intestinal, y compris un cancer de l'estomac, un cancer du côlon, un cancer colorectal, un cancer du pancréas, un cancer du foie ; une tumeur maligne de l'appareil génital féminin, y compris un carcinome ovarien, un cancer de l'endométrium utérin et un cancer du col de l'utérus ; un cancer de la vessie ; un cancer du cerveau, y compris un neuroblastome ; un sarcome, un ostéosarcome ; et un cancer de la peau, y compris un mélanome malin ou un cancer à cellules squameuses.

9. Antagoniste de M-CSF pour utilisation selon l'une quelconque des revendications précédentes, où l'antagoniste de M-CSF est administré à une dose comprise entre 0,01 mg/kg et 100 mg/kg.

10. Antagoniste de M-CSF pour utilisation selon l'une quelconque des revendications 1 à 4, où ledit antagoniste est un anticorps entièrement humain.

11. Antagoniste de M-CSF pour utilisation selon l'une quelconque des revendications 1 à 4, où ledit antagoniste est un anticorps humanisé.

12. Antagoniste de M-CSF pour utilisation selon l'une quelconque des revendications précédentes, où ledit anticorps est conjugué à un radionucléide ou à une autre toxine.

13. Antagoniste de M-CSF pour utilisation selon l'une quelconque des revendications précédentes, où ledit sujet est un mammifère.

14. Antagoniste de M-CSF pour utilisation selon la revendication 13, où ledit sujet est un humain.

15. Antagoniste de M-CSF pour utilisation selon une quelconque des revendications précédentes, où l'antagoniste doit être utilisé en combinaison avec un autre agent thérapeutique.


### SEQUENCE LISTING

<110> Novartis Vaccines and Diagnostics, Inc.
<120> METHODS FOR PREVENTING AND TREATING CANCER METASTASIS AND BONE LOSS
   ASSOCIATED WITH CANCER METASTASIS
<130> P055579EP
<150> US 60/426,781
   <151> 2002-11-15
<160> 8
<170> PatentIn version 3.2
<210> 1
   <211> 1855
   <212> DNA
   <213> Homo sapiens
<220>
   <221> CDS
   <222> (190)..(960)
<220>
   <221> sig_peptide
   <222> (190)..(285)
<220>
   <221> mat_peptide
   <222> (286)..(957)
<400> 1
   gagggctggc cagtgaggct cggcccgggg aaagtgaaag tttgcctggg tcctctcggc      60
   gccagagccg ctctccgcat cccaggacag cggtgcggcc ctcggccggg gcgcccactc     120
   cgcagcagcc agcgagcgag cgagcgagcg agggcggccg acgcgcccgg ccgggaccca     180
   gctgcccgt atg acc gcg ccg ggc gcc gcc ggg cgc tgc cct ccc acg aca     231
             Met Thr Ala Pro Gly Ala Ala Gly Arg Cys Pro Pro Thr Thr
             1               5                   10
   tgg ctg ggc tcc ctg ctg ttg ttg gtc tgt ctc ctg gcg agc agg agt       279
   Trp Leu Gly Ser Leu Leu Leu Leu Val Cys Leu Leu Ala Ser Arg Ser
   15                  20                  25                  30
   atc acc gag gag gtg tcg gag tac tgt agc cac atg att ggg agt gga       327
   Ile Thr Glu Glu Val Ser Glu Tyr Cys Ser His Met Ile Gly Ser Gly
                   35                  40                  45
   cac ctg cag tct ctg cag cgg ctg att gac agt cag atg gag acc tcg       375
   His Leu Gln Ser Leu Gln Arg Leu Ile Asp Ser Gln Met Glu Thr Ser
               50                  55                  60
   tgc caa att aca ttt gag ttt gta gac cag gaa cag ttg aaa gat cca       423
   Cys Gln Ile Thr Phe Glu Phe Val Asp Gln Glu Gln Leu Lys Asp Pro
           65                  70                  75
   gtg tgc tac ctt aag aag gca ttt ctc ctg gta caa gac ata atg gag       471
   Val Cys Tyr Leu Lys Lys Ala Phe Leu Leu Val Gln Asp Ile Met Glu
       80                  85                  90
   gac acc atg cgc ttc aga gat aac acc ccc aat gcc atc gcc att gtg      519
   Asp Thr Met Arg Phe Arg Asp Asn Thr Pro Asn Ala Ile Ala Ile Val
   95                  100                 105                 110
   cag ctg cag gaa ctc tct ttg agg ctg aag agc tgc ttc acc aag gat      567
   Gln Leu Gln Glu Leu Ser Leu Arg Leu Lys Ser Cys Phe Thr Lys Asp
                   115                 120                 125
   tat gaa gag cat gac aag gcc tgc gtc cga act ttc tat gag aca cct      615
   Tyr Glu Glu His Asp Lys Ala Cys Val Arg Thr Phe Tyr Glu Thr Pro
               130                 135                 140
   ctc cag ttg ctg gag aag gtc aag aat gtc ttt aat gaa aca aag aat      663
   Leu Gln Leu Leu Glu Lys Val Lys Asn Val Phe Asn Glu Thr Lys Asn
           145                 150                 155
   ctc ctt gac aag gac tgg aat att ttc agc aag aac tgc aac aac agc      711
   Leu Leu Asp Lys Asp Trp Asn Ile Phe Ser Lys Asn Cys Asn Asn Ser
       160                 165                 170
   ttt gct gaa tgc tcc agc caa ggc cat gag agg cag tcc gag gga tcc      759
   Phe Ala Glu Cys Ser Ser Gln Gly His Glu Arg Gln Ser Glu Gly Ser
   175                 180                 185                 190
   tcc agc ccg cag ctc cag gag tct gtc ttc cac ctg ctg gtg ccc agt      807
   Ser Ser Pro Gln Leu Gln Glu Ser Val Phe His Leu Leu Val Pro Ser
                   195                 200                 205
   gtc atc ctg gtc ttg ctg gcc gtc gga ggc ctc ttg ttc tac agg tgg      855
   Val Ile Leu Val Leu Leu Ala Val Gly Gly Leu Leu Phe Tyr Arg Trp
               210                 215                 220
   agg cgg cgg agc cat caa gag cct cag aga gcg gat tct ccc ttg gag      903
   Arg Arg Arg Ser His Gln Glu Pro Gln Arg Ala Asp Ser Pro Leu Glu
           225                 230                 235
   caa cca gag ggc agc ccc ctg act cag gat gac aga cag gtg gaa ctg      951
   Gln Pro Glu Gly Ser Pro Leu Thr Gln Asp Asp Arg Gln Val Glu Leu
       240                 245                 250
   cca gtg tag agggaattct aagctggacg cacagaacag tctctccgtg             1000
   Pro Val
   255
   ggaggagaca ttatggggcg tccaccacca cccctccctg gccatcctcc tggaatgtgg   1060
   tctgccctcc accagagctc ctgcctgcca ggactggacc agagcagcca ggctggggcc   1120
   cctctgtctc aacccgcaga cccttgactg aatgagagag gccagaggat gctccccatg   1180
   ctgccactat ttattgtgag ccctggaggc tcccatgtgc ttgaggaagg ctggtgagcc   1240
   cggctcagga ccctcttccc tcaggggctg caccctcctc tcactccctt ccatgccgga   1300
   acccaggcca gggacccacc ggcctgtggt ttgtgggaaa gcagggtgga cgctgaggag   1360
   tgaaagaacc ctgcacccag agggcctgcc tggtgccaag gtatcccagc ctggacaggc   1420
   atggacctgt ctccagagag aggagcctga agttcgtggg gcgggacagc gtcggcctga   1480
   tttcccgtaa aggtgtgcag cctgagagac gggaagagga ggcctctgga cctgctggtc   1540
   tgcactgaca gcctgaaggg tctacaccct cggctcacct aagtgccctg tgctggttgc   1600
   caggcgcaga ggggaggcca gccctgccct caggacctgc ctgacctgcc agtgatgcca   1660
   agagggggat caagcactgg cctctgcccc tcctccttcc agcacctgcc agagcttctc   1720
   caggaggcca agcagaggct cccctcatga aggaagccat tgcactgtga acactgtacc   1780
   tgcctgctga acagcctgcc cccgtccatc catgagccag catccgtccg tcctccactc   1840
   tccagcctct cccca                                                      1855
<210> 2
   <211> 256
   <212> PRT
   <213> Homo sapiens
<400> 2
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Gly His Glu Arg Gln Ser Glu Gly Ser Ser Ser
               180                 185                 190
   Pro Gln Leu Gln Glu Ser Val Phe His Leu Leu Val Pro Ser Val Ile
           195                 200                 205
   Leu Val Leu Leu Ala Val Gly Gly Leu Leu Phe Tyr Arg Trp Arg Arg
       210                 215                 220
   Arg Ser His Gln Glu Pro Gln Arg Ala Asp Ser Pro Leu Glu Gln Pro
   225                 230                 235                 240
   Glu Gly Ser Pro Leu Thr Gln Asp Asp Arg Gln Val Glu Leu Pro Val
                   245                 250                 255
<210> 3
   <211> 2749
   <212> DNA
   <213> Homo sapiens
<220>
   <221> CDS
   <222> (190)..(1854)
<220>
   <221> sig_peptide
   <222> (190)..(285)
<220>
   <221> mat_peptide
   <222> (286)..(1851)
<400> 3
   gagggctggc cagtgaggct cggcccgggg aaagtgaaag tttgcctggg tcctctcggc      60
   gccagagccg ctctccgcat cccaggacag cggtgcggcc ctcggccggg gcgcccactc     120
   cgcagcagcc agcgagcgag cgagcgagcg agggcggccg acgcgcccgg ccgggaccca     180
   gctgcccgt atg acc gcg ccg ggc gcc gcc ggg cgc tgc cct ccc acg aca     231
             Met Thr Ala Pro Gly Ala Ala Gly Arg Cys Pro Pro Thr Thr
             1               5                   10
   tgg ctg ggc tcc ctg ctg ttg ttg gtc tgt ctc ctg gcg agc agg agt       279
   Trp Leu Gly Ser Leu Leu Leu Leu Val Cys Leu Leu Ala Ser Arg Ser
   15                  20                  25                  30
   atc acc gag gag gtg tcg gag tac tgt agc cac atg att ggg agt gga       327
   Ile Thr Glu Glu Val Ser Glu Tyr Cys Ser His Met Ile Gly Ser Gly
                   35                  40                  45
   cac ctg cag tct ctg cag cgg ctg att gac agt cag atg gag acc tcg      375
   His Leu Gln Ser Leu Gln Arg Leu Ile Asp Ser Gln Met Glu Thr Ser
               50                  55                  60
   tgc caa att aca ttt gag ttt gta gac cag gaa cag ttg aaa gat cca      423
   Cys Gln Ile Thr Phe Glu Phe Val Asp Gln Glu Gln Leu Lys Asp Pro
           65                  70                  75
   gtg tgc tac ctt aag aag gca ttt ctc ctg gta caa gac ata atg gag      471
   Val Cys Tyr Leu Lys Lys Ala Phe Leu Leu Val Gln Asp Ile Met Glu
       80                  85                  90
   gac acc atg cgc ttc aga gat aac acc ccc aat gcc atc gcc att gtg      519
   Asp Thr Met Arg Phe Arg Asp Asn Thr Pro Asn Ala Ile Ala Ile Val
   95                  100                 105                 110
   cag ctg cag gaa ctc tct ttg agg ctg aag agc tgc ttc acc aag gat      567
   Gln Leu Gln Glu Leu Ser Leu Arg Leu Lys Ser Cys Phe Thr Lys Asp
                   115                 120                 125
   tat gaa gag cat gac aag gcc tgc gtc cga act ttc tat gag aca cct      615
   Tyr Glu Glu His Asp Lys Ala Cys Val Arg Thr Phe Tyr Glu Thr Pro
               130                 135                 140
   ctc cag ttg ctg gag aag gtc aag aat gtc ttt aat gaa aca aag aat      663
   Leu Gln Leu Leu Glu Lys Val Lys Asn Val Phe Asn Glu Thr Lys Asn
           145                 150                 155
   ctc ctt gac aag gac tgg aat att ttc agc aag aac tgc aac aac agc      711
   Leu Leu Asp Lys Asp Trp Asn Ile Phe Ser Lys Asn Cys Asn Asn Ser
       160                 165                 170
   ttt gct gaa tgc tcc agc caa gat gtg gtg acc aag cct gat tgc aac      759
   Phe Ala Glu Cys Ser Ser Gln Asp Val Val Thr Lys Pro Asp Cys Asn
   175                 180                 185                 190
   tgc ctg tac ccc aaa gcc atc cct agc agt gac ccg gcc tct gtc tcc      807
   Cys Leu Tyr Pro Lys Ala Ile Pro Ser Ser Asp Pro Ala Ser Val Ser
                   195                 200                 205
   cct cat cag ccc ctc gcc ccc tcc atg gcc cct gtg gct ggc ttg acc      855
   Pro His Gln Pro Leu Ala Pro Ser Met Ala Pro Val Ala Gly Leu Thr
               210                 215                 220
   tgg gag gac tct gag gga act gag ggc agc tcc ctc ttg cct ggt gag      903
   Trp Glu Asp Ser Glu Gly Thr Glu Gly Ser Ser Leu Leu Pro Gly Glu
           225                 230                 235
   cag ccc ctg cac aca gtg gat cca ggc agt gcc aag cag cgg cca ccc      951
   Gln Pro Leu His Thr Val Asp Pro Gly Ser Ala Lys Gln Arg Pro Pro
       240                 245                 250
   agg agc acc tgc cag agc ttt gag ccg cca gag acc cca gtt gtc aag      999
   Arg Ser Thr Cys Gln Ser Phe Glu Pro Pro Glu Thr Pro Val Val Lys
   255                 260                 265                 270
   gac agc acc atc ggt ggc tca cca cag cct cgc ccc tct gtc ggg gcc     1047
   Asp Ser Thr Ile Gly Gly Ser Pro Gln Pro Arg Pro Ser Val Gly Ala
                   275                 280                 285
   ttc aac ccc ggg atg gag gat att ctt gac tct gca atg ggc act aat      1095
   Phe Asn Pro Gly Met Glu Asp Ile Leu Asp Ser Ala Met Gly Thr Asn
               290                 295                 300
   tgg gtc cca gaa gaa gcc tct gga gag gcc agt gag att ccc gta ccc      1143
   Trp Val Pro Glu Glu Ala Ser Gly Glu Ala Ser Glu Ile Pro Val Pro
           305                 310                 315
   caa ggg aca gag ctt tcc ccc tcc agg cca gga ggg ggc agc atg cag      1191
   Gln Gly Thr Glu Leu Ser Pro Ser Arg Pro Gly Gly Gly Ser Met Gln
       320                 325                 330
   aca gag ccc gcc aga ccc agc aac ttc ctc tca gca tct tct cca ctc      1239
   Thr Glu Pro Ala Arg Pro Ser Asn Phe Leu Ser Ala Ser Ser Pro Leu
   335                 340                 345                 350
   cct gca tca gca aag ggc caa cag ccg gca gat gta act ggt aca gcc      1287
   Pro Ala Ser Ala Lys Gly Gln Gln Pro Ala Asp Val Thr Gly Thr Ala
                   355                 360                 365
   ttg ccc agg gtg ggc ccc gtg agg ccc act ggc cag gac tgg aat cac      1335
   Leu Pro Arg Val Gly Pro Val Arg Pro Thr Gly Gln Asp Trp Asn His
               370                 375                 380
   acc ccc cag aag aca gac cat cca tct gcc ctg ctc aga gac ccc ccg      1383
   Thr Pro Gln Lys Thr Asp His Pro Ser Ala Leu Leu Arg Asp Pro Pro
           385                 390                 395
   gag cca ggc tct ccc agg atc tca tca ctg cgc ccc cag ggc ctc agc      1431
   Glu Pro Gly Ser Pro Arg Ile Ser Ser Leu Arg Pro Gln Gly Leu Ser
       400                 405                 410
   aac ccc tcc acc ctc tct gct cag cca cag ctt tcc aga agc cac tcc      1479
   Asn Pro Ser Thr Leu Ser Ala Gln Pro Gln Leu Ser Arg Ser His Ser
   415                 420                 425                 430
   tcg ggc agc gtg ctg ccc ctt ggg gag ctg gag ggc agg agg agc acc      1527
   Ser Gly Ser Val Leu Pro Leu Gly Glu Leu Glu Gly Arg Arg Ser Thr
                   435                 440                 445
   agg gat cgg agg agc ccc gca gag cca gaa gga gga cca gca agt gaa      1575
   Arg Asp Arg Arg Ser Pro Ala Glu Pro Glu Gly Gly Pro Ala Ser Glu
               450                 455                 460
   ggg gca gcc agg ccc ctg ccc cgt ttt aac tcc gtt cct ttg act gac      1623
   Gly Ala Ala Arg Pro Leu Pro Arg Phe Asn Ser Val Pro Leu Thr Asp
           465                 470                 475
   aca ggc cat gag agg cag tcc gag gga tcc tcc agc ccg cag ctc cag      1671
   Thr Gly His Glu Arg Gln Ser Glu Gly Ser Ser Ser Pro Gln Leu Gln
       480                 485                 490
   gag tct gtc ttc cac ctg ctg gtg ccc agt gtc atc ctg gtc ttg ctg      1719
   Glu Ser Val Phe His Leu Leu Val Pro Ser Val Ile Leu Val Leu Leu
   495                 500                 505                 510
   gcc gtc gga ggc ctc ttg ttc tac agg tgg agg cgg cgg agc cat caa      1767
   Ala Val Gly Gly Leu Leu Phe Tyr Arg Trp Arg Arg Arg Ser His Gln
                   515                 520                 525
   gag cct cag aga gcg gat tct ccc ttg gag caa cca gag ggc agc ccc      1815
   Glu Pro Gln Arg Ala Asp Ser Pro Leu Glu Gln Pro Glu Gly Ser Pro
               530                 535                 540
   ctg act cag gat gac aga cag gtg gaa ctg cca gtg tag agggaattct       1864
   Leu Thr Gln Asp Asp Arg Gln Val Glu Leu Pro Val
           545                 550
   aagctggacg cacagaacag tctctccgtg ggaggagaca ttatggggcg tccaccacca    1924
   cccctccctg gccatcctcc tggaatgtgg tctgccctcc accagagctc ctgcctgcca    1984
   ggactggacc agagcagcca ggctggggcc cctctgtctc aacccgcaga cccttgactg    2044
   aatgagagag gccagaggat gctccccatg ctgccactat ttattgtgag ccctggaggc    2104
   tcccatgtgc ttgaggaagg ctggtgagcc cggctcagga ccctcttccc tcaggggctg    2164
   caccctcctc tcactccctt ccatgccgga acccaggcca gggacccacc ggcctgtggt    2224
   ttgtgggaaa gcagggtgga cgctgaggag tgaaagaacc ctgcacccag agggcctgcc    2284
   tggtgccaag gtatcccagc ctggacaggc atggacctgt ctccagagag aggagcctga    2344
   agttcgtggg gcgggacagc gtcggcctga tttcccgtaa aggtgtgcag cctgagagac    2404
   gggaagagga ggcctctgga cctgctggtc tgcactgaca gcctgaaggg tctacaccct    2464
   cggctcacct aagtgccctg tgctggttgc caggcgcaga ggggaggcca gccctgccct    2524
   caggacctgc ctgacctgcc agtgatgcca agagggggat caagcactgg cctctgcccc     2584
   tcctccttcc agcacctgcc agagcttctc caggaggcca agcagaggct cccctcatga    2644
   aggaagccat tgcactgtga acactgtacc tgcctgctga acagcctgcc cccgtccatc    2704
   catgagccag catccgtccg tcctccactc tccagcctct cccca                    2749
<210> 4
   <211> 554
   <212> PRT
   <213> Homo sapiens
<400> 4
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
           275                 280                 285
   Pro Gly Met Glu Asp Ile Leu Asp Ser Ala Met Gly Thr Asn Trp Val
       290                 295                 300
   Pro Glu Glu Ala Ser Gly Glu Ala Ser Glu Ile Pro Val Pro Gln Gly
   305                 310                 315                 320
   Thr Glu Leu Ser Pro Ser Arg Pro Gly Gly Gly Ser Met Gln Thr Glu
                   325                 330                 335
   Pro Ala Arg Pro Ser Asn Phe Leu Ser Ala Ser Ser Pro Leu Pro Ala
               340                 345                 350
   Ser Ala Lys Gly Gln Gln Pro Ala Asp Val Thr Gly Thr Ala Leu Pro
           355                 360                 365
   Arg Val Gly Pro Val Arg Pro Thr Gly Gln Asp Trp Asn His Thr Pro
       370                 375                 380
   Gln Lys Thr Asp His Pro Ser Ala Leu Leu Arg Asp Pro Pro Glu Pro
   385                 390                 395                 400
   Gly Ser Pro Arg Ile Ser Ser Leu Arg Pro Gln Gly Leu Ser Asn Pro
                   405                 410                 415
   Ser Thr Leu Ser Ala Gln Pro Gln Leu Ser Arg Ser His Ser Ser Gly
               420                 425                 430
   Ser Val Leu Pro Leu Gly Glu Leu Glu Gly Arg Arg Ser Thr Arg Asp
           435                 440                 445
   Arg Arg Ser Pro Ala Glu Pro Glu Gly Gly Pro Ala Ser Glu Gly Ala
       450                 455                 460
   Ala Arg Pro Leu Pro Arg Phe Asn Ser Val Pro Leu Thr Asp Thr Gly
   465                 470                 475                 480
   His Glu Arg Gln Ser Glu Gly Ser Ser Ser Pro Gln Leu Gln Glu Ser
                   485                 490                 495
   Val Phe His Leu Leu Val Pro Ser Val Ile Leu Val Leu Leu Ala Val
               500                 505                 510
   Gly Gly Leu Leu Phe Tyr Arg Trp Arg Arg Arg Ser His Gln Glu Pro
           515                 520                 525
   Gln Arg Ala Asp Ser Pro Leu Glu Gln Pro Glu Gly Ser Pro Leu Thr
       530                 535                 540
   Gln Asp Asp Arg Gln Val Glu Leu Pro Val
   545                 550
<210> 5
   <211> 1519
   <212> DNA
   <213> Homo sapiens
<220>
   <221> CDS
   <222> (190)..(1506)
<220>
   <221> sig_peptide
   <222> (190)..(285)
<220>
   <221> mat_peptide
   <222> (286)..(1503)
<400> 5
   gagggctggc cagtgaggct cggcccgggg aaagtgaaag tttgcctggg tcctctcggc      60
   gccagagccg ctctccgcat cccaggacag cggtgcggcc ctcggccggg gcgcccactc     120
   cgcagcagcc agcgagcgag cgagcgagcg agggcggccg acgcgcccgg ccgggaccca     180
   gctgcccgt atg acc gcg ccg ggc gcc gcc ggg cgc tgc cct ccc acg aca     231
             Met Thr Ala Pro Gly Ala Ala Gly Arg Cys Pro Pro Thr Thr
             1               5                   10
   tgg ctg ggc tcc ctg ctg ttg ttg gtc tgt ctc ctg gcg agc agg agt       279
   Trp Leu Gly Ser Leu Leu Leu Leu Val Cys Leu Leu Ala Ser Arg Ser
   15                  20                  25                  30
   atc acc gag gag gtg tcg gag tac tgt agc cac atg att ggg agt gga       327
   Ile Thr Glu Glu Val Ser Glu Tyr Cys Ser His Met Ile Gly Ser Gly
                   35                  40                  45
   cac ctg cag tct ctg cag cgg ctg att gac agt cag atg gag acc tcg       375
   His Leu Gln Ser Leu Gln Arg Leu Ile Asp Ser Gln Met Glu Thr Ser
               50                  55                  60
   tgc caa att aca ttt gag ttt gta gac cag gaa cag ttg aaa gat cca       423
   Cys Gln Ile Thr Phe Glu Phe Val Asp Gln Glu Gln Leu Lys Asp Pro
           65                  70                  75
   gtg tgc tac ctt aag aag gca ttt ctc ctg gta caa gac ata atg gag       471
   Val Cys Tyr Leu Lys Lys Ala Phe Leu Leu Val Gln Asp Ile Met Glu
       80                  85                  90
   gac acc atg cgc ttc aga gat aac acc ccc aat gcc atc gcc att gtg       519
   Asp Thr Met Arg Phe Arg Asp Asn Thr Pro Asn Ala Ile Ala Ile Val
   95                  100                 105                 110
   cag ctg cag gaa ctc tct ttg agg ctg aag agc tgc ttc acc aag gat       567
   Gln Leu Gln Glu Leu Ser Leu Arg Leu Lys Ser Cys Phe Thr Lys Asp
                   115                 120                 125
   tat gaa gag cat gac aag gcc tgc gtc cga act ttc tat gag aca cct      615
   Tyr Glu Glu His Asp Lys Ala Cys Val Arg Thr Phe Tyr Glu Thr Pro
               130                 135                 140
   ctc cag ttg ctg gag aag gtc aag aat gtc ttt aat gaa aca aag aat      663
   Leu Gln Leu Leu Glu Lys Val Lys Asn Val Phe Asn Glu Thr Lys Asn
           145                 150                 155
   ctc ctt gac aag gac tgg aat att ttc agc aag aac tgc aac aac agc      711
   Leu Leu Asp Lys Asp Trp Asn Ile Phe Ser Lys Asn Cys Asn Asn Ser
       160                 165                 170
   ttt gct gaa tgc tcc agc caa gat gtg gtg acc aag cct gat tgc aac      759
   Phe Ala Glu Cys Ser Ser Gln Asp Val Val Thr Lys Pro Asp Cys Asn
   175                 180                 185                 190
   tgc ctg tac ccc aaa gcc atc cct agc agt gac ccg gcc tct gtc tcc      807
   Cys Leu Tyr Pro Lys Ala Ile Pro Ser Ser Asp Pro Ala Ser Val Ser
                   195                 200                 205
   cct cat cag ccc ctc gcc ccc tcc atg gcc cct gtg gct ggc ttg acc      855
   Pro His Gln Pro Leu Ala Pro Ser Met Ala Pro Val Ala Gly Leu Thr
               210                 215                 220
   tgg gag gac tct gag gga act gag ggc agc tcc ctc ttg cct ggt gag      903
   Trp Glu Asp Ser Glu Gly Thr Glu Gly Ser Ser Leu Leu Pro Gly Glu
           225                 230                 235
   cag ccc ctg cac aca gtg gat cca ggc agt gcc aag cag cgg cca ccc      951
   Gln Pro Leu His Thr Val Asp Pro Gly Ser Ala Lys Gln Arg Pro Pro
       240                 245                 250
   agg agc acc tgc cag agc ttt gag ccg cca gag acc cca gtt gtc aag      999
   Arg Ser Thr Cys Gln Ser Phe Glu Pro Pro Glu Thr Pro Val Val Lys
   255                 260                 265                 270
   gac agc acc atc ggt ggc tca cca cag cct cgc ccc tct gtc ggg gcc     1047
   Asp Ser Thr Ile Gly Gly Ser Pro Gln Pro Arg Pro Ser Val Gly Ala
                   275                 280                 285
   ttc aac ccc ggg atg gag gat att ctt gac tct gca atg ggc act aat     1095
   Phe Asn Pro Gly Met Glu Asp Ile Leu Asp Ser Ala Met Gly Thr Asn
               290                 295                 300
   tgg gtc cca gaa gaa gcc tct gga gag gcc agt gag att ccc gta ccc     1143
   Trp Val Pro Glu Glu Ala Ser Gly Glu Ala Ser Glu Ile Pro Val Pro
           305                 310                 315
   caa ggg aca gag ctt tcc ccc tcc agg cca gga ggg ggc agc atg cag     1191
   Gln Gly Thr Glu Leu Ser Pro Ser Arg Pro Gly Gly Gly Ser Met Gln
       320                 325                 330
   aca gag ccc gcc aga ccc agc aac ttc ctc tca gca tct tct cca ctc     1239
   Thr Glu Pro Ala Arg Pro Ser Asn Phe Leu Ser Ala Ser Ser Pro Leu
   335                 340                 345                 350
   cct gca tca gca aag ggc caa cag ccg gca gat gta act ggc cat gag     1287
   Pro Ala Ser Ala Lys Gly Gln Gln Pro Ala Asp Val Thr Gly His Glu
                   355                 360                 365
   agg cag tcc gag gga tcc tcc agc ccg cag ctc cag gag tct gtc ttc     1335
   Arg Gln Ser Glu Gly Ser Ser Ser Pro Gln Leu Gln Glu Ser Val Phe
               370                 375                 380
   cac ctg ctg gtg ccc agt gtc atc ctg gtc ttg ctg gcc gtc gga ggc      1383
   His Leu Leu Val Pro Ser Val Ile Leu Val Leu Leu Ala Val Gly Gly
           385                 390                 395
   ctc ttg ttc tac agg tgg agg cgg cgg agc cat caa gag cct cag aga      1431
   Leu Leu Phe Tyr Arg Trp Arg Arg Arg Ser His Gln Glu Pro Gln Arg
       400                 405                 410
   gcg gat tct ccc ttg gag caa cca gag ggc agc ccc ctg act cag gat      1479
   Ala Asp Ser Pro Leu Glu Gln Pro Glu Gly Ser Pro Leu Thr Gln Asp
   415                 420                 425                 430
   gac aga cag gtg gaa ctg cca gtg tag agggaattct aag                   1519
   Asp Arg Gln Val Glu Leu Pro Val
                   435
<210> 6
   <211> 438
   <212> PRT
   <213> Homo sapiens
<400> 6
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
           275                 280                 285
   Pro Gly Met Glu Asp Ile Leu Asp Ser Ala Met Gly Thr Asn Trp Val
       290                 295                 300
   Pro Glu Glu Ala Ser Gly Glu Ala Ser Glu Ile Pro Val Pro Gln Gly
   305                 310                 315                 320
   Thr Glu Leu Ser Pro Ser Arg Pro Gly Gly Gly Ser Met Gln Thr Glu
                   325                 330                 335
   Pro Ala Arg Pro Ser Asn Phe Leu Ser Ala Ser Ser Pro Leu Pro Ala
               340                 345                 350
   Ser Ala Lys Gly Gln Gln Pro Ala Asp Val Thr Gly His Glu Arg Gln
           355                 360                 365
   Ser Glu Gly Ser Ser Ser Pro Gln Leu Gln Glu Ser Val Phe His Leu
       370                 375                 380
   Leu Val Pro Ser Val Ile Leu Val Leu Leu Ala Val Gly Gly Leu Leu
   385                 390                 395                 400
   Phe Tyr Arg Trp Arg Arg Arg Ser His Gln Glu Pro Gln Arg Ala Asp
                   405                 410                 415
   Ser Pro Leu Glu Gln Pro Glu Gly Ser Pro Leu Thr Gln Asp Asp Arg
               420                 425                 430
   Gln Val Glu Leu Pro Val
           435
<210> 7
   <211> 3985
   <212> DNA
   <213> Homo sapiens
<220>
   <221> CDS
   <222> (293)..(3211)
<220>
   <221> sig_peptide
   <222> (293) .. (349)
<220>
   <221> mat_peptide
   <222> (350)..(3208)
<220>
   <221> misc_feature
   <222> (374)..(598)
   <223> Immunoglobulin
<220>
   <221> misc_feature
   <222> (917)..(1177)
   <223> Immunoglobulin
<220>
   <221> misc_feature
   <222> (2516)..(3022)
   <223> Tyrosine Kinase, Catalytic Domain
<400> 7
gaagggcaga cagagtgtcc aaaagcgtga gagcacgaag tgaggagaag gtggagaaga     60
   gagaagagga agaggaagag gaagagagga agcggaggga actgcggcca ggctaaaagg    120
   ggaagaagag gatcagccca aggaggagga agaggaaaac aagacaaaca gccagtgcag    180
   aggagaggaa cgtgtgtcca gtgtcccgat ccctgcggag ctagtagctg agagctctgt    240
   gccctgggca ccttgcagcc ctgcacctgc ctgccacttc cccaccgagg cc atg ggc    298
   cca gga gtt ctg ctg ctc ctg ctg gtg gcc aca gct tgg cat ggt cag      346
   gga atc cca gtg ata gag ccc agt gtc cct gag ctg gtc gtg aag cca      394
   gga gca acg gtg acc ttg cga tgt gtg ggc aat ggc agc gtg gaa tgg      442
   gat ggc ccc cca tca cct cac tgg acc ctg tac tct gat ggc tcc agc      490
   agc atc ctc agc acc aac aac gct acc ttc caa aac acg ggg acc tat      538
   cgc tgc act gag cct gga gac ccc ctg gga ggc agc gcc gcc atc cac      586
   ctc tat gtc aaa gac cct gcc cgg ccc tgg aac gtg cta gca cag gag      634
   gtg gtc gtg ttc gag gac cag gac gca cta ctg ccc tgt ctg ctc aca      682
   gac ccg gtg ctg gaa gca ggc gtc tcg ctg gtg cgt gtg cgt ggc cgg      730
   ccc ctc atg cgc cac acc aac tac tcc ttc tcg ccc tgg cat ggc ttc      778
   acc atc cac agg gcc aag ttc att cag agc cag gac tat caa tgc agt      826
   gcc ctg atg ggt ggc agg aag gtg atg tcc atc agc atc cgg ctg aaa      874
   gtg cag aaa gtc atc cca ggg ccc cca gcc ttg aca ctg gtg cct gca      922
   gag ctg gtg cgg att cga ggg gag gct gcc cag atc gtg tgc tca gcc      970
   agc agc gtt gat gtt aac ttt gat gtc ttc ctc caa cac aac aac acc     1018
   aag ctc gca atc cct caa caa tct gac ttt cat aat aac cgt tac caa      1066
   aaa gtc ctg acc ctc aac ctc gat caa gta gat ttc caa cat gcc ggc      1114
   aac tac tcc tgc gtg gcc agc aac gtg cag ggc aag cac tcc acc tcc      1162
   atg ttc ttc cgg gtg gta gag agt gcc tac ttg aac ttg agc tct gag      1210
   cag aac ctc atc cag gag gtg acc gtg ggg gag ggg ctc aac ctc aaa      1258
   gtc atg gtg gag gcc tac cca ggc ctg caa ggt ttt aac tgg acc tac      1306
   ctg gga ccc ttt tct gac cac cag cct gag ccc aag ctt gct aat gct      1354
   acc acc aag gac aca tac agg cac acc ttc acc ctc tct ctg ccc cgc      1402
   ctg aag ccc tct gag gct ggc cgc tac tcc ttc ctg gcc aga aac cca      1450
   gga ggc tgg aga gct ctg acg ttt gag ctc acc ctt cga tac ccc cca      1498
   gag gta agc gtc ata tgg aca ttc atc aac ggc tct ggc acc ctt ttg      1546
   tgt gct gcc tct ggg tac ccc cag ccc aac gtg aca tgg ctg cag tgc      1594
   agt ggc cac act gat agg tgt gat gag gcc caa gtg ctg cag gtc tgg      1642
   gat gac cca tac cct gag gtc ctg agc cag gag ccc ttc cac aag gtg      1690
   acg gtg cag agc ctg ctg act gtt gag acc tta gag cac aac caa acc      1738
   tac gag tgc agg gcc cac aac agc gtg ggg agt ggc tcc tgg gcc ttc      1786
   ata ccc atc tct gca gga gcc cac acg cat ccc ccg gat gag ttc ctc      1834
   ttc aca cca gtg gtg gtc gcc tgc atg tcc atc atg gcc ttg ctg ctg      1882
   ctg ctg ctc ctg ctg cta ttg tac aag tat aag cag aag ccc aag tac      1930
   cag gtc cgc tgg aag atc atc gag agc tat gag ggc aac agt tat act      1978
   ttc atc gac ccc acg cag ctg cct tac aac gag aag tgg gag ttc ccc      2026
   cgg aac aac ctg cag ttt ggt aag acc ctc gga gct gga gcc ttt ggg      2074
   aag gtg gtg gag gcc acg gcc ttt ggt ctg ggc aag gag gat gct gtc      2122
   ctg aag gtg gct gtg aag atg ctg aag tcc acg gcc cat gct gat gag      2170
   aag gag gcc ctc atg tcc gag ctg aag atc atg agc cac ctg ggc cag      2218
   cac gag aac atc gtc aac ctt ctg gga gcc tgt acc cat gga ggc cct      2266
   gta ctg gtc atc acg gag tac tgt tgc tat ggc gac ctg ctc aac ttt      2314
   ctg cga agg aag gct gag gcc atg ctg gga ccc agc ctg agc ccc ggc      2362
   cag gac ccc gag gga ggc gtc gac tat aag aac atc cac ctc gag aag      2410
   aaa tat gtc cgc agg gac agt ggc ttc tcc agc cag ggt gtg gac acc      2458
   tat gtg gag atg agg cct gtc tcc act tct tca aat gac tcc ttc tct      2506
   gag caa gac ctg gac aag gag gat gga cgg ccc ctg gag ctc cgg gac      2554
   ctg ctt cac ttc tcc agc caa gta gcc cag ggc atg gcc ttc ctc gct      2602
   tcc aag aat tgc atc cac cgg gac gtg gca gcg cgt aac gtg ctg ttg      2650
   acc aat ggt cat gtg gcc aag att ggg gac ttc ggg ctg gct agg gac      2698
   atc atg aat gac tcc aac tac att gtc aag ggc aat gcc cgc ctg cct      2746
   gtg aag tgg atg gcc cca gag agc atc ttt gac tgt gtc tac acg gtt      2794
   cag agc gac gtc tgg tcc tat ggc atc ctc ctc tgg gag atc ttc tca      2842
   ctt ggg ctg aat ccc tac cct ggc atc ctg gtg aac agc aag ttc tat      2890
   aaa ctg gtg aag gat gga tac caa atg gcc cag cct gca ttt gcc cca      2938
   aag aat ata tac agc atc atg cag gcc tgc tgg gcc ttg gag ccc acc      2986
   cac aga ccc acc ttc cag cag atc tgc tcc ttc ctt cag gag cag gcc      3034
   caa gag gac agg aga gag cgg gac tat acc aat ctg ccg agc agc agc      3082
   aga agc ggt ggc agc ggc agc agc agc agt gag ctg gag gag gag agc      3130
   tct agt gag cac ctg acc tgc tgc gag caa ggg gat atc gcc cag ccc      3178
   ttg ctg cag ccc aac aac tat cag ttc tgc tga ggagttgacg acagggagta    3231
   ccactctccc ctcctccaaa cttcaactcc tccatggatg gggcgacacg gggagaacat 3291
   acaaactctg ccttcggtca tttcactcaa cagctcggcc cagctctgaa acttgggaag 3351
   gtgagggatt caggggaggt cagaggatcc cacttcctga gcatgggcca tcactgccag 3411
   tcaggggctg ggggctgagc cctcaccccc ccctccccta ctgttctcat ggtgttggcc 3471
   tcgtgtttgc tatgccaact agtagaacct tctttcctaa tccccttatc ttcatggaaa 3531
   tggactgact ttatgcctat gaagtcccca ggagctacac tgatactgag aaaaccaggc 3591
   tctttggggc tagacagact ggcagagagt gagatctccc tctctgagag gagcagcaga 3651
   tgctcacaga ccacactcag ctcaggcccc ttggagcagg atggctcctc taagaatctc 3711
   acaggacctc ttagtctctg ccctatacgc cgccttcact ccacagcctc acccctccca 3771
   cccccatact ggtactgctg taatgagcca agtggcagct aaaagttggg ggtgttctgc 3831
   ccagtcccgt cattctgggc tagaaggcag gggaccttgg catgtggctg gccacaccaa 3891
   gcaggaagca caaactcccc caagctgact catcctaact aacagtcacg ccgtgggatg 3951
   tctctgtcca cattaaacta acagcattaa tgca 3985
<210> 8
   <211> 972
   <212> PRT
   <213> Homo sapiens
<400> 8